# EUROPEAN PATENT SPECIFICATION

(11) **EP 2 187 730 B1**
(45) Date of publication and mention of the grant of the patent: **06.04.2016**
(21) Application number: 08850946.8
(22) Date of filing: 14.11.2008
(51) Int. Cl.: A01H 5/02, A01H 4/00, C12N 5/10, C12N 9/00, C12N 15/53, C12N 15/82

(54) **GENETICALLY MODIFIED CHRYSANTHEMUMS**
GENETISCH MODIFIZIERTE CHRYSANTHEMEN
CHRYSANTHÈMES GÉNÉTIQUEMENT MODIFIÉS

(30) Priority: 15.11.2007 US 988331 P
(43) Date of publication of application: 26.05.2010
(73) Proprietor: Suntory Holdings Limited, Kita-ku, Osaka-shi, Osaka 530-8203 (JP)
(72) Inventor: BRUGLIERA, Filippa, Preston Victoria 3072 (AU)
(74) Representative: Hoffmann Eitle
(86) International application number: PCT/AU2008/001694
(87) International publication number: WO 2009/062253

(56) References cited:
- EP-A1- 1 652 916
- EP-A2- 0 522 880
- WO-A1-96/36716
- WO-A1-2004/020637
- US-A- 5 948 955
- US-A- 6 114 601
- SEITZ ET AL: "Redirection of anthocyanin synthesis in Osteospermum hybrida by a two-enzyme manipulation strategy", PHYTOCHEMISTRY, PERGAMON PRESS, GB, vol. 68, no. 6, 3 March 2007 (2007-03-03), pages 824-833, XP005912647, ISSN: 0031-9422, DOI: DOI:10.1016/J.PHYTOCHEM.2006.12.012

### SEQUENCE LISTING

<110> INTERNATIONAL FLOWER DEVELOPMENTS PTY LTD
   BRUGLIERA, Filippa (US ONLY)
<120> GENETICALLY MODIFIED PLANTS
<130> 30683376/EJH
<150> US 60/988331
   <151> 2007-11-15
<160> 45
<170> PatentIn version 3.4
<210> 1
   <211> 1648
   <212> DNA
   <213> artificial
<220>
   <223> BPF3'5'H#18.nt
<400> 1
   agccaatatg gcaattccag tcactgacct tgctgtcgcg gttatccttt tcttgatcac      60
   tcgcttccta gttcgttctc ttttcaagaa accaaccgga ccgctcccgc cgggtccttc     120
   aggctggccc ttggtgggcg cgctccctct cctaggcgcc atgcctcacg tcacactagc     180
   caacctcgct aaaaaatacg gtccgatcat gtacctaaaa atgggcacgt gcgacatggt     240
   ggtcgcgtcc actcccgact cggctcgagc cttcctcaaa accctagacc tcaacttctc     300
   cgaccgcccg cccaacgccg gcgccaccca tttggcgtac ggcgcgcagg acttggtctt     360
   cgcgaagtac ggtccaaggt ggaagaccct aagaaaattg agcaacctcc acatgctagg     420
   cgggaaggcg ctggacgatt gggctcacgt gagggctaac gagctaggcc acatgcttaa     480
   cgccatgtgc gaggcgagcc ggtgcggaga gcccgtggtg ctggccgaga tgctcacgta 540
   cgccatggcc aacatgatcg gtcaagtgat actgagtcgg cgcgtgttcg tcaccaaagg 600
   gacagagtcg aacgagttca aagatatggt ggtcgagttg atgacttccg cggggtattt 660
   caacattggt gacttcatac cgtcgattgc ttggatggat ttgcaaggga tcgagcgagg 720
   gatgaagaaa ttgcacacga aattcgatgt tttgttgacg aagatgatga aggagcacag 780
   agcgacgagt catgagcgcg aagggaaatc ggatttcctc gacgtcctct tggaagaatg 840
   cgagaataca aatggcgaga agcttaatgt taccaacgtc aaagctgtcc tcttgaactt 900
   attcacggcg ggtacggaca catcttcaag cataatcgaa tgggcgttaa ccgaaatgat 960
   gaagaatccg acgatcttaa aaaagaccca agaagagatg gatcgagtca tcggtcgcga 1020
   tcggagattg ctcgaatccg acgtttcgaa actcccgtat ttacaagcca tagcgaaaga 1080
   aacatatcgt aaacacccat cgacacctct aaacctgccg aggattgcga tccaagcatg 1140
   tgaagttgat ggctactaca tccccaaaga cacgaggctt agcgtcaaca tttgggcgat 1200
   cggtcgggac ccaagtgttt gggagaatcc atcggagttc tcgcctgaaa gattcttgtc 1260
   tgaggagaat gggaagatca gtccaggcgg gaatgatttt gagctgattc cgtttggagc 1320
   agggaggaga atttgtgctg ggacaaggat gggaatggtc cttgtaagtt atattttggg 1380
   cactttggtc cattcttttg attggaaatt accaaatggg gtcagtgaga ttaacatgga 1440
   tgagagtttt gggcttgcgt tgcaaaaggc cgtgcctctc tcggctacgg tcagtccacg 1500
   attggcccca agcgcgtacg ttatatgagc tgatgggctg ggcctgagcc caaacatatt 1560
   gggtgtgttt tatctgtaat ttttaatatt ataaagttcg taattttgta tttatggtta 1620
   attatgagtt aaaaaaaaaa aaaaaaaa 1648
<210> 2
   <211> 506
   <212> PRT
   <213> artificial
<220>
   <223> BPF3'5'H#18.aa
<400> 2
   Met Ala Ile Pro Val Thr Asp Leu Ala Val Ala Val Ile Leu Phe Leu
   1               5                   10                  15
   Ile Thr Arg Phe Leu Val Arg Ser Leu Phe Lys Lys Pro Thr Gly Pro
               20                  25                  30
   Leu Pro Pro Gly Pro Ser Gly Trp Pro Leu Val Gly Ala Leu Pro Leu
           35                  40                  45
   Leu Gly Ala Met Pro His Val Thr Leu Ala Asn Leu Ala Lys Lys Tyr
       50                  55                  60
   Gly Pro Ile Met Tyr Leu Lys Met Gly Thr Cys Asp Met Val Val Ala
   65                  70                  75                  80
   Ser Thr Pro Asp Ser Ala Arg Ala Phe Leu Lys Thr Leu Asp Leu Asn
                   85                  90                  95
   Phe Ser Asp Arg Pro Pro Asn Ala Gly Ala Thr His Leu Ala Tyr Gly
               100                 105                 110
   Ala Gln Asp Leu Val Phe Ala Lys Tyr Gly Pro Arg Trp Lys Thr Leu
           115                 120                 125
   Arg Lys Leu Ser Asn Leu His Met Leu Gly Gly Lys Ala Leu Asp Asp
       130                 135                 140
   Trp Ala His Val Arg Ala Asn Glu Leu Gly His Met Leu Asn Ala Met
   145                 150                 155                 160
   Cys Glu Ala Ser Arg Cys Gly Glu Pro Val Val Leu Ala Glu Met Leu
                   165                 170                 175
   Thr Tyr Ala Met Ala Asn Met Ile Gly Gln Val Ile Leu Ser Arg Arg
               180                 185                 190
   Val Phe Val Thr Lys Gly Thr Glu Ser Asn Glu Phe Lys Asp Met Val
           195                 200                 205
   Val Glu Leu Met Thr Ser Ala Gly Tyr Phe Asn Ile Gly Asp Phe Ile
       210                 215                 220
   Pro Ser Ile Ala Trp Met Asp Leu Gln Gly Ile Glu Arg Gly Met Lys
   225                 230                 235                 240
   Lys Leu His Thr Lys Phe Asp Val Leu Leu Thr Lys Met Met Lys Glu
                   245                 250                 255
   His Arg Ala Thr Ser His Glu Arg Glu Gly Lys Ser Asp Phe Leu Asp
               260                 265                 270
   Val Leu Leu Glu Glu Cys Glu Asn Thr Asn Gly Glu Lys Leu Asn Val
   275 280 285
   Thr Asn Val Lys Ala Val Leu Leu Asn Leu Phe Thr Ala Gly Thr Asp
   290 295 300
   Thr Ser Ser Ser Ile Ile Glu Trp Ala Leu Thr Glu Met Met Lys Asn
   305 310 315 320
   Pro Thr Ile Leu Lys Lys Thr Gln Glu Glu Met Asp Arg Val Ile Gly
   325 330 335
   Arg Asp Arg Arg Leu Leu Glu Ser Asp Val Ser Lys Leu Pro Tyr Leu
   340 345 350
   Gln Ala Ile Ala Lys Glu Thr Tyr Arg Lys His Pro Ser Thr Pro Leu
   355 360 365
   Asn Leu Pro Arg Ile Ala Ile Gln Ala Cys Glu Val Asp Gly Tyr Tyr
   370 375 380
   Ile Pro Lys Asp Thr Arg Leu Ser Val Asn Ile Trp Ala Ile Gly Arg
   385 390 395 400
   Asp Pro Ser Val Trp Glu Asn Pro Ser Glu Phe Ser Pro Glu Arg Phe
   405 410 415
   Leu Ser Glu Glu Asn Gly Lys Ile Ser Pro Gly Gly Asn Asp Phe Glu
   420 425 430
   Leu Ile Pro Phe Gly Ala Gly Arg Arg Ile Cys Ala Gly Thr Arg Met
   435 440 445
   Gly Met Val Leu Val Ser Tyr Ile Leu Gly Thr Leu Val His Ser Phe
   450 455 460
   Asp Trp Lys Leu Pro Asn Gly Val Ser Glu Ile Asn Met Asp Glu Ser
   465 470 475 480
   Phe Gly Leu Ala Leu Gln Lys Ala Val Pro Leu Ser Ala Thr Val Ser
   485 490 495
   Pro Arg Leu Ala Pro Ser Ala Tyr Val Ile
   500 505
<210> 3
   <211> 1789
   <212> DNA
   <213> artificial
<220>
   <223> BPF3'5'H#40.nt
<400> 3
   ggcacgagga caacatggca attctagtca ccgacttcgt tgtcgcggct ataattttct 60
   tgatcactcg gttcttagtt cgttctcttt tcaagaaacc aacccgaccg ctccccccgg 120
   gtcctctcgg ttggcccttg gtgggcgccc tccctctcct aggcgccatg cctcacgtcg 180
   cactagccaa actcgctaag aagtatggtc cgatcatgca cctaaaaatg ggcacgtgcg 240
   acatggtggt cgcgtccacc cccgagtcgg ctcgagcctt cctcaaaacg ctagacctca 300
   acttctccaa ccgcccaccc aacgcgggcg catcccacct agcgtacggc gcgcaggact 360
   tagtcttcgc caagtacggt ccgaggtgga agactttaag aaaattgagc aacctccaca 420
   tgctaggcgg gaaggcgttg gatgattggg caaatgtgag ggtcaccgag ctaggccaca 480
   tgcttaaagc catgtgcgag gcgagccggt gcggggagcc cgtggtgctg gccgagatgc 540
   tcacgtacgc catggcgaac atgatcggtc aagtgatact cagccggcgc gtgttcgtga 600
   ccaaagggac cgagtctaac gagttcaaag acatggtggt cgagttgatg acgtccgccg 660
   ggtacttcaa catcggtgac ttcataccct cgatcgcttg gatggatttg caagggatcg 720
   agcgagggat gaagaagctg cacacgaagt ttgatgtgtt attgacgaag atggtgaagg 780
   agcatagagc gacgagtcat gagcgcaaag ggaaggcaga tttcctcgac gttctcttgg 840
   aagaatgcga caatacaaat ggggagaagc ttagtattac caatatcaaa gctgtccttt 900
   tgaatctatt cacggcgggc acggacacat cttcgagcat aatcgaatgg gcgttaacgg 960
   agatgatcaa gaatccgacg atcttaaaaa aggcgcaaga ggagatggat cgagtcatcg 1020
   gtcgtgatcg gaggctgctc gaatcggaca tatcgagcct cccgtaccta caagccattg 1080
   ctaaagaaac gtatcgcaaa cacccgtcga cgcctctcaa cttgccgagg attgcgatcc 1140
   aagcatgtga agttgatggc tactacatcc ctaaggacgc gaggcttagc gtgaacattt 1200
   gggcgatcgg tcgggacccg aatgtttggg agaatccgtt ggagttcttg ccggaaagat 1260
   tcttgtctga agagaatggg aagatcaatc ccggtgggaa tgattttgag ctgattccgt 1320
   ttggagccgg gaggagaatt tgtgcgggga caaggatggg aatggtcctt gtaagttata 1380
   ttttgggcac tttggtccat tcttttgatt ggaaattacc aaatggtgtc gctgagctta 1440
   atatggatga aagttttggg cttgcattgc aaaaggccgt gccgctctcg gccttggtca 1500
   gcccacggtt ggcctcaaac gcgtacgcaa cctgagctaa tgggctgggc ctagttttgt 1560
   gggccttaat ttagagactt ttgtgtttta aggtgtgtac tttattaatt gggtgcttaa   1620
   atgtgtgttt taatttgtat ttatggttaa ttatgacttt attgtataat tatttatttt   1680
   tcccttctgg gtattttatc catttaattt ttcttcagaa ttatgatcat agttatcaga   1740
   ataaaattga aaataatgaa tcggaaaaaa aaaaaaaaaa aaaaaaaaa               1789
<210> 4
   <211> 506
   <212> PRT
   <213> artificial
<220>
   <223> BPF3'5'H#40.aa
<400> 4
   Met Ala Ile Leu Val Thr Asp Phe Val Val Ala Ala Ile Ile Phe Leu
   1               5                   10                  15
   Ile Thr Arg Phe Leu Val Arg Ser Leu Phe Lys Lys Pro Thr Arg Pro
               20                  25                  30
   Leu Pro Pro Gly Pro Leu Gly Trp Pro Leu Val Gly Ala Leu Pro Leu
           35                  40                  45
   Leu Gly Ala Met Pro His Val Ala Leu Ala Lys Leu Ala Lys Lys Tyr
       50                  55                  60
   Gly Pro Ile Met His Leu Lys Met Gly Thr Cys Asp Met Val Val Ala
   65                  70                  75                  80
   Ser Thr Pro Glu Ser Ala Arg Ala Phe Leu Lys Thr Leu Asp Leu Asn
   85 90 95
   Phe Ser Asn Arg Pro Pro Asn Ala Gly Ala Ser His Leu Ala Tyr Gly
   100 105 110
   Ala Gln Asp Leu Val Phe Ala Lys Tyr Gly Pro Arg Trp Lys Thr Leu
   115 120 125
   Arg Lys Leu Ser Asn Leu His Met Leu Gly Gly Lys Ala Leu Asp Asp
   130 135 140
   Trp Ala Asn Val Arg Val Thr Glu Leu Gly His Met Leu Lys Ala Met
   145 150 155 160
   Cys Glu Ala Ser Arg Cys Gly Glu Pro Val Val Leu Ala Glu Met Leu
   165 170 175
   Thr Tyr Ala Met Ala Asn Met Ile Gly Gln Val Ile Leu Ser Arg Arg
   180 185 190
   Val Phe Val Thr Lys Gly Thr Glu Ser Asn Glu Phe Lys Asp Met Val
   195 200 205
   Val Glu Leu Met Thr Ser Ala Gly Tyr Phe Asn Ile Gly Asp Phe Ile
   210 215 220
   Pro Ser Ile Ala Trp Met Asp Leu Gln Gly Ile Glu Arg Gly Met Lys
   225 230 235 240
   Lys Leu His Thr Lys Phe Asp Val Leu Leu Thr Lys Met Val Lys Glu
   245 250 255
   His Arg Ala Thr Ser His Glu Arg Lys Gly Lys Ala Asp Phe Leu Asp
   260 265 270
   Val Leu Leu Glu Glu Cys Asp Asn Thr Asn Gly Glu Lys Leu Ser Ile
   275 280 285
   Thr Asn Ile Lys Ala Val Leu Leu Asn Leu Phe Thr Ala Gly Thr Asp
   290 295 300
   Thr Ser Ser Ser Ile Ile Glu Trp Ala Leu Thr Glu Met Ile Lys Asn
   305 310 315 320
   Pro Thr Ile Leu Lys Lys Ala Gln Glu Glu Met Asp Arg Val Ile Gly
   325 330 335
   Arg Asp Arg Arg Leu Leu Glu Ser Asp Ile Ser Ser Leu Pro Tyr Leu
   340 345 350
   Gln Ala Ile Ala Lys Glu Thr Tyr Arg Lys His Pro Ser Thr Pro Leu
   355 360 365
   Asn Leu Pro Arg Ile Ala Ile Gln Ala Cys Glu Val Asp Gly Tyr Tyr
   370 375 380
   Ile Pro Lys Asp Ala Arg Leu Ser Val Asn Ile Trp Ala Ile Gly Arg
   385 390 395 400
   Asp Pro Asn Val Trp Glu Asn Pro Leu Glu Phe Leu Pro Glu Arg Phe
                   405                 410                 415
   Leu Ser Glu Glu Asn Gly Lys Ile Asn Pro Gly Gly Asn Asp Phe Glu
               420                 425                 430
   Leu Ile Pro Phe Gly Ala Gly Arg Arg Ile Cys Ala Gly Thr Arg Met
           435                 440                 445
   Gly Met Val Leu Val Ser Tyr Ile Leu Gly Thr Leu Val His Ser Phe
       450                 455                 460
   Asp Trp Lys Leu Pro Asn Gly Val Ala Glu Leu Asn Met Asp Glu Ser
   465                 470                 475                 480
   Phe Gly Leu Ala Leu Gln Lys Ala Val Pro Leu Ser Ala Leu Val Ser
                   485                 490                 495
   Pro Arg Leu Ala Ser Asn Ala Tyr Ala Thr
               500                 505
<210> 5
   <211> 1617
   <212> DNA
   <213> artificial
<220>
   <223> SalF3'5'H#47.nt
<400> 5
   agatagtaag catggaagcc caagaaaata tgttgttgat tgctagggca cttgttgtag      60
   catccttact ctacattttg atccgtatgt ttatctcaaa attgagcacc cccggccacc     120
   ctctgccccc ggggccgagg ggctttccag tggtgggctc ccttcccttg ctgggcgaca     180
   tgccacatgt tgccctagca aaaatggcca aaacttatgg cccgatcatg tacttgaaaa     240
   tgggcacagt cggcatggtc gtggcgtcca cgccagacgc ggcgcgggcg ttcctaaaaa     300
   cccaggacgc taatttctct aaccggccgg tcaacgcggg tgccaccatc ctggcataca     360
   atgcccagga catggtgttt gccccgtacg gccccaagtg gagattgctg aggaagctga     420
   gcagtctcca catgctgggg agcaaggccc tggaggagtg ggccgacgtc cggacctcgg     480
   aggtggggca catgctggcg gcgatgcacg aggccagccg cctggacgag gccgtggggt     540
   tgccggagat gctggtgtac gcgacggcga acatgatcgg gaaggtgata ttgagccgga     600
   gagttttcgt gacgaaaggg aaggagatga atgagttcaa ggaaatggtg gtggagctca     660
   tgaccacagc tggctatttc aacattggtg atttcattcc atggcttgct tggatggatt     720
   tgcaggggat tgagagaggg atgaagaaac tgcacaagaa gtgggaccgc ttgatcggta     780
   agatgctgga tgatcgattg aaatcaacct acaaacgcaa cgacaagcca gatcttcttg     840
   attctctctt ggcaaatcat gatgatgaga gtaaggatga tgatgaggat tgcaagctca     900
   ccaccaccaa tattaaagcc cttttactga atttatttac tgcagggaca gacacatcgt     960
   cgagcataat agaatgggca ctagcggaga tgatcaagaa tccaagcatc caaaaaaggg    1020
   ctcaccaaga gatggacaga gtcatcggga gagagcggcg tttgctcgaa tccgacatcc    1080
   caaatctgcc atacctcaaa gccatatgca aagaggcata ccgaaaacac ccttccacgc    1140
   cactaaacct gcctcggatc tccacggatg catgcgtcgt cgatggctac cacatcccca    1200
   agaacacgag gttgagcgtc aacatctggg ccataggccg agatcccgac gtttgggaga    1260
   atccccttga cttcaaccct gacaggttta tgtcagggtt gcaggggatt gagcccggag    1320
   ggaatcactt cgagctcatt ccctttgggg cggggcgcag gatctgcgcc ggcagcagaa 1380
   tggggattgt aatagtggag tatttgctgg cgacactcgt gcactctttc gaatgggatt 1440
   tgccagccgg ctcagcggag atggacatgg aggaggtgtt cgggctggcc ttgcagaaag 1500
   ctgtaccact tgctgctagg ctcactccta ggttgccttc acattgctat gcacctcctt 1560
   ctatttaatt tgcatattta tatatgttgt gttacattga aaaaaaaaaa aaaaaaa 1617
<210> 6
   <211> 518
   <212> PRT
   <213> artificial
<220>
   <223> SalF3'5'H47.aa
<400> 6
   Met Glu Ala Gln Glu Asn Met Leu Leu Ile Ala Arg Ala Leu Val Val
   1 5 10 15
   Ala Ser Leu Leu Tyr Ile Leu Ile Arg Met Phe Ile Ser Lys Leu Ser
   20 25 30
   Thr Pro Gly His Pro Leu Pro Pro Gly Pro Arg Gly Phe Pro Val Val
   35 40 45
   Gly Ser Leu Pro Leu Leu Gly Asp Met Pro His Val Ala Leu Ala Lys
   50 55 60
   Met Ala Lys Thr Tyr Gly Pro Ile Met Tyr Leu Lys Met Gly Thr Val
   65 70 75 80
   Gly Met Val Val Ala Ser Thr Pro Asp Ala Ala Arg Ala Phe Leu Lys
   85 90 95
   Thr Gln Asp Ala Asn Phe Ser Asn Arg Pro Val Asn Ala Gly Ala Thr
   100 105 110
   Ile Leu Ala Tyr Asn Ala Gln Asp Met Val Phe Ala Pro Tyr Gly Pro
   115 120 125
   Lys Trp Arg Leu Leu Arg Lys Leu Ser Ser Leu His Met Leu Gly Ser
   130 135 140
   Lys Ala Leu Glu Glu Trp Ala Asp Val Arg Thr Ser Glu Val Gly His
   145 150 155 160
   Met Leu Ala Ala Met His Glu Ala Ser Arg Leu Asp Glu Ala Val Gly
   165 170 175
   Leu Pro Glu Met Leu Val Tyr Ala Thr Ala Asn Met Ile Gly Lys Val
   180 185 190
   Ile Leu Ser Arg Arg Val Phe Val Thr Lys Gly Lys Glu Met Asn Glu
   195 200 205
   Phe Lys Glu Met Val Val Glu Leu Met Thr Thr Ala Gly Tyr Phe Asn
   210 215 220
   Ile Gly Asp Phe Ile Pro Trp Leu Ala Trp Met Asp Leu Gln Gly Ile
   225 230 235 240
   Glu Arg Gly Met Lys Lys Leu His Lys Lys Trp Asp Arg Leu Ile Gly
                   245                 250                 255
   Lys Met Leu Asp Asp Arg Leu Lys Ser Thr Tyr Lys Arg Asn Asp Lys
               260                 265                 270
   Pro Asp Leu Leu Asp Ser Leu Leu Ala Asn His Asp Asp Glu Ser Lys
           275                 280                 285
   Asp Asp Asp Glu Asp Cys Lys Leu Thr Thr Thr Asn Ile Lys Ala Leu
       290                 295                 300
   Leu Leu Asn Leu Phe Thr Ala Gly Thr Asp Thr Ser Ser Ser Ile Ile
   305                 310                 315                 320
   Glu Trp Ala Leu Ala Glu Met Ile Lys Asn Pro Ser Ile Gln Lys Arg
                   325                 330                 335
   Ala His Gln Glu Met Asp Arg Val Ile Gly Arg Glu Arg Arg Leu Leu
               340                 345                 350
   Glu Ser Asp Ile Pro Asn Leu Pro Tyr Leu Lys Ala Ile Cys Lys Glu
           355                 360                 365
   Ala Tyr Arg Lys His Pro Ser Thr Pro Leu Asn Leu Pro Arg Ile Ser
       370                 375                 380
   Thr Asp Ala Cys Val Val Asp Gly Tyr His Ile Pro Lys Asn Thr Arg
   385                 390                 395                 400
   Leu Ser Val Asn Ile Trp Ala Ile Gly Arg Asp Pro Asp Val Trp Glu
   405 410 415
   Asn Pro Leu Asp Phe Asn Pro Asp Arg Phe Met Ser Gly Leu Gln Gly
   420 425 430
   Ile Glu Pro Gly Gly Asn His Phe Glu Leu Ile Pro Phe Gly Ala Gly
   435 440 445
   Arg Arg Ile Cys Ala Gly Ser Arg Met Gly Ile Val Ile Val Glu Tyr
   450 455 460
   Leu Leu Ala Thr Leu Val His Ser Phe Glu Trp Asp Leu Pro Ala Gly
   465 470 475 480
   Ser Ala Glu Met Asp Met Glu Glu Val Phe Gly Leu Ala Leu Gln Lys
   485 490 495
   Ala Val Pro Leu Ala Ala Arg Leu Thr Pro Arg Leu Pro Ser His Cys
   500 505 510
   Tyr Ala Pro Pro Ser Ile
   515
<210> 7
   <211> 1812
   <212> DNA
   <213> artificial
<220>
   <223> PetHf1.nt
<400> 7
   ctttctacta gctacttcgt tatatatatg taaaattgtg actttgaaaa tcatttaaat 60
   tatcataagg ttcattttat cttgatcaaa atatttactt cggccatata cgttttcctt 120
   tagtcatgat gctacttact gagcttggtg cagcaacttc aatctttcta atagcacaca 180
   taatcatttc aactcttatt tcaaaaacta ccggccggca tctaccgccg gggccaagag 240
   ggtggccggt gatcggagca cttccacttt taggagccat gccacatgtt tccttagcta 300
   aaatggcaaa aaaatatgga gcaatcatgt atctcaaagt tggaacatgt ggcatggcag 360
   ttgcttctac ccctgatgct gctaaagcat tcttgaaaac acttgatatc aacttctcca 420
   atcgtccacc taatgcaggt gccactcact tagcttataa tgctcaagac atggtttttg 480
   cacattatgg accacgatgg aagttgctaa ggaaattaag caacttgcat atgctagggg 540
   gaaaagcctt agagaattgg gcaaatgttc gtgccaatga gctagggcac atgctaaaat 600
   caatgtccga tatgagtcga gagggccaga gggttgtggt ggcggagatg ttgacatttg 660
   ccatggccaa tatgatcgga caagtgatgc taagcaaaag agtatttgta gataaaggtg 720
   ttgaggtaaa tgaatttaag gacatggttg tagagttaat gacaatagca gggtatttca 780
   acattggtga ttttattcct tgtttagctt ggatggattt acaagggata gaaaaacgaa 840
   tgaaacgttt acataagaag tttgatgctt tattgacaaa gatgtttgat gaacacaaag 900
   caactaccta tgaacgtaag gggaaaccag attttcttga tgttgttatg gaaaatgggg 960
   acaattctga aggagaaaga ctcagtacaa ccaacatcaa agcacttttg ctgaatttgt 1020
   tcacagctgg tacggacact tcttctagtg caatagaatg ggcacttgca gaaatgatga 1080
   agaaccctgc cattttgaaa aaagcacaag cagaaatgga tcaagtcatt ggaagaaata 1140
   ggcgtttact cgaatccgat atcccaaatc tcccttacct ccgagcaatt tgcaaagaaa 1200
   catttcgaaa acacccttct acaccattaa atcttcctag gatctcgaac gaaccatgca 1260
   tagtcgatgg ttattacata ccaaaaaaca ctaggcttag tgttaacata tgggcaattg 1320
   gaagagatcc ccaagtttgg gaaaatccac tagagtttaa tcccgaaaga ttcttgagtg 1380
   gaagaaactc caagattgat cctcgaggga acgattttga attgatacca tttggtgctg 1440
   gacgaagaat ttgtgcagga acaagaatgg gaattgtaat ggtggaatat atattaggaa 1500
   ctttggttca ttcatttgat tggaaattac caagtgaagt tattgagttg aatatggaag 1560
   aagcttttgg cttagctttg cagaaagctg tccctcttga agctatggtt actccaaggt 1620
   tacaattgga tgtttatgta ccatagctat agatgtgtat tgtgctataa ttgcgcatgt 1680
   tgttggttgt agcatgagat attaaaagga gtacatgaag cgcattgcat gagtttaact 1740
   tgtagctcct taatatttta ggtatttttc aattaataag ttcttgttgg ttgggtaaaa 1800
   aaaaaaaaaa aa 1812
<210> 8
   <211> 506
   <212> PRT
   <213> artificial
<220>
   <223> PetHf1.aa
<400> 8
   Met Met Leu Leu Thr Glu Leu Gly Ala Ala Thr Ser Ile Phe Leu Ile
   1               5                   10                  15
   Ala His Ile Ile Ile Ser Thr Leu Ile Ser Lys Thr Thr Gly Arg His
               20                  25                  30
   Leu Pro Pro Gly Pro Arg Gly Trp Pro Val Ile Gly Ala Leu Pro Leu
           35                  40                  45
   Leu Gly Ala Met Pro His Val Ser Leu Ala Lys Met Ala Lys Lys Tyr
       50                  55                  60
   Gly Ala Ile Met Tyr Leu Lys Val Gly Thr Cys Gly Met Ala Val Ala
   65                  70                  75                  80
   Ser Thr Pro Asp Ala Ala Lys Ala Phe Leu Lys Thr Leu Asp Ile Asn
                   85                  90                  95
   Phe Ser Asn Arg Pro Pro Asn Ala Gly Ala Thr His Leu Ala Tyr Asn
               100                 105                 110
   Ala Gln Asp Met Val Phe Ala His Tyr Gly Pro Arg Trp Lys Leu Leu
           115                 120                 125
   Arg Lys Leu Ser Asn Leu His Met Leu Gly Gly Lys Ala Leu Glu Asn
   130 135 140
   Trp Ala Asn Val Arg Ala Asn Glu Leu Gly His Met Leu Lys Ser Met
   145 150 155 160
   Ser Asp Met Ser Arg Glu Gly Gln Arg Val Val Val Ala Glu Met Leu
   165 170 175
   Thr Phe Ala Met Ala Asn Met Ile Gly Gln Val Met Leu Ser Lys Arg
   180 185 190
   Val Phe Val Asp Lys Gly Val Glu Val Asn Glu Phe Lys Asp Met Val
   195 200 205
   Val Glu Leu Met Thr Ile Ala Gly Tyr Phe Asn Ile Gly Asp Phe Ile
   210 215 220
   Pro Cys Leu Ala Trp Met Asp Leu Gln Gly Ile Glu Lys Arg Met Lys
   225 230 235 240
   Arg Leu His Lys Lys Phe Asp Ala Leu Leu Thr Lys Met Phe Asp Glu
   245 250 255
   His Lys Ala Thr Thr Tyr Glu Arg Lys Gly Lys Pro Asp Phe Leu Asp
   260 265 270
   Val Val Met Glu Asn Gly Asp Asn Ser Glu Gly Glu Arg Leu Ser Thr
   275 280 285
   Thr Asn Ile Lys Ala Leu Leu Leu Asn Leu Phe Thr Ala Gly Thr Asp
       290                 295                 300
   Thr Ser Ser Ser Ala Ile Glu Trp Ala Leu Ala Glu Met Met Lys Asn
   305                 310                 315                 320
   Pro Ala Ile Leu Lys Lys Ala Gln Ala Glu Met Asp Gln Val Ile Gly
                   325                 330                 335
   Arg Asn Arg Arg Leu Leu Glu Ser Asp Ile Pro Asn Leu Pro Tyr Leu
               340                 345                 350
   Arg Ala Ile Cys Lys Glu Thr Phe Arg Lys His Pro Ser Thr Pro Leu
           355                 360                 365
   Asn Leu Pro Arg Ile Ser Asn Glu Pro Cys Ile Val Asp Gly Tyr Tyr
       370                 375                 380
   Ile Pro Lys Asn Thr Arg Leu Ser Val Asn Ile Trp Ala Ile Gly Arg
   385                 390                 395                 400
   Asp Pro Gln Val Trp Glu Asn Pro Leu Glu Phe Asn Pro Glu Arg Phe
                   405                 410                 415
   Leu Ser Gly Arg Asn Ser Lys Ile Asp Pro Arg Gly Asn Asp Phe Glu
               420                 425                 430
   Leu Ile Pro Phe Gly Ala Gly Arg Arg Ile Cys Ala Gly Thr Arg Met
           435                 440                 445
   Gly Ile Val Met Val Glu Tyr Ile Leu Gly Thr Leu Val His Ser Phe
   450 455 460
   Asp Trp Lys Leu Pro Ser Glu Val Ile Glu Leu Asn Met Glu Glu Ala
   465 470 475 480
   Phe Gly Leu Ala Leu Gln Lys Ala Val Pro Leu Glu Ala Met Val Thr
   485 490 495
   Pro Arg Leu Gln Leu Asp Val Tyr Val Pro
   500 505
<210> 9
   <211> 2934
   <212> DNA
   <213> artificial
<220>
   <223> RoseCHS 5'
<400> 9
   aagcttcagc aagagttgaa gaaataggga cagagccatc catgtgcttt gatgaatctg 60
   atgggataca aaatgtgaaa gattcacttg ctgatttatc cagaatttct tcatatagtg 120
   aggagaatgt tgaaagatct aatgatgagc actctgttaa actagacgga attcatgtgc 180
   agcacgagtg tcatgagggc agtgaagaag acaaacctga tggtaagagc ggtgagaatg 240
   cagttgatct ggctaatcat ggcatggctc gaactgattt ttgtcagata acagaagaga 300
   ttgagaatgg agtagtcatc actgagatga gcaacattgc caaccctgat aaaactgata 360
   ttccaaacgg ggtgcctcaa aatgagactg atgatggatt taataacact caggatgatg 420
   ctaatacaaa ggaagtgaca gaagagaatt ctgacagacg tgcgaaggaa gtgacagaag     480
   agaattctga caaagatgtt ttgaagaata tccttgaatt ctcacgtgct tcttctgtgg     540
   tggattttga aattccagtg ttggatgtga aatttacttc tcttgaaagt tgcagtgcca     600
   cttgttctct tgcagccctt ttgtctgaat cgccggaatc aatgactgaa gcaccttgtg     660
   tgaggcaaat tgatgatgtg cccccggttg gtgaggagtc tagcttgatt ttggtggaag     720
   atcgggagcc ggttggtcct actcctgatg gtaatttttc tgtggatatg gattactata     780
   gtgtagcaga acctttgagc acatgggatg cgaatctgca gtgtgaaaca tcaaatagcc     840
   atgagacttt tgctgcaagt ctcatttgat agcttctgtg ttaataactt tgttagtctg     900
   tacataaatt tgtctagaca agaattggtc gtgtactatc gtgtgttttt gccgtgcttt     960
   agtactcatg aaccaattca gagaaaactg gctgcatatt ttgaggagtc tctgaattct    1020
   tcaatgctca actggtatgc atgtaggtgg catatcactt cagggattct tctattcttt    1080
   aactttacgc atcttgacat tttgtatata acaaaatcag gtctattggg tgaaagtaat    1140
   tggctagaat ggaaagctct acggttttac cgcaggtcaa ttttcatagc tccacaagtg    1200
   aattgaaaat gctcataggc tttatgtttg tcctccacct ctggcgacga tgtttgttgg    1260
   ggagttaact caaacctacc accaaactcg aacccatctt ccataattta taatacaaat    1320
   ttgcgatcat ttgttcatcc aattattgtg acactcggct accacccaaa atatcggtca    1380
   cagacccaaa cgtattgtca caacaaatcg tgtctctcgc attaaacaca gctagaaaga    1440
   agagttgaac ccacaattcg agcacccact acctatgtac gaagtcatga gttcgagtca    1500
   ccataggggt agaagtgaaa tcatttgatc atctttaaag aaataaaagg aagagttgaa    1560
   cccacaattg gctcttgtcc caaaaagaac taatagttca gtgcaccgac gtgtatttgc    1620
   accgacataa atggattgtt agattatatt aaatacactc ttaggttatt aataaaaata 1680
   ttaattataa atatcaaaag ttgagatcat cttataaatg ttgggtcagt tacaccgtcg 1740
   gtgcatagaa taatttccaa actatataat agccttcatt ttctgattta gctcatggga 1800
   catgattgct ataaataatt gtactcgtag aggcatactt gtgtcttttt atacagttgt 1860
   actgaagctc agaaaagttt atgaaggtga gaactgagaa gggcaaggca tttggtagtt 1920
   gaggtatatg agagcatgaa ccccatgcat tgcagctacc acctctcttt tttccttctt 1980
   cccatacaaa taaaaccaac tcttctcacc taagtctatc atctttattt atggcagctc 2040
   ttgcttaatt agctcatcta tattatatta tttatctata atatgtgtca ctctgtctac 2100
   ctaccagccc aaaataaaac tgataatagt caatttgatg atattttttg ttttttgttt 2160
   tgttttgtct tttttgtatt gattttttta aaattaaaat gacttcattt tttgtttttg 2220
   tttttttttc tatttttttt tatagaaaaa ttggcaaact ttcattatct gttattgatg 2280
   acaattaagc cattaaaacc tataattaat tatctttcaa ttcgagtaaa tttaaaacgg 2340
   tgtaaaatta aaatatgatc gtattcttaa atgaataaaa ctcacttaat aatagtaata 2400
   cttgaatcac atctacgaac atagattctt ttcatccagt ctaaccatgt ttgaatatat 2460
   agagtttgat tatggttatg tctttgtcca cattttggtt tgtaaataaa tgtgcaacgg 2520
   aggtatggta ctgttgctct atcaaattca agtttgaatt aaaagaaaaa aaaaaagacg 2580
   atattttgtg cgctttgttt ggtaggtaaa acgagagaac aaacgcattc caaatcatgc 2640
   ggattttgat cggcaacaca caccacaaaa aaccgtacac gatgcacgtg ccatttgccg 2700
   ggggtttcta acaaggtaat tgggcaggca cgtgatcccc cagctaccca cctctcgctt 2760
   cccttctcaa actccttttc catgtatata tacaacccct tttctcagac cattatattc 2820
   taacattttt gctttgctat tgtaacgcaa caaaaactgc tcattccatc cttgttcctc 2880
   cccattttga tcttctctcg acccttctcc gagatgggta ccgagctcga attc 2934
<210> 10
   <211> 2544
   <212> DNA
   <213> artificial
<220>
   <223> carnANS 5'
<400> 10
   actcgaaatg atactgtgag tctgcgatag gctcgttttg aggcggaaat tatgatttta 60
   cgacgtgatc aattgagcat gacttaaatt tgcgtcttct cagtcgtcgt tgcattgcaa 120
   tttttagtat tttcaggtgc tctgaaagtg tttagtacat cgtttttaaa atggatatct 180
   ttttgttctg gtcgacttac tcttcgcttt ttaatgcaga cgtgcccgtt attgctacgt 240
   gtattcacaa aggtatgacc gtgttctgta gcgtctaatg ataatatatg aagtcgaggt 300
   tgcatttgta ctagtccgat aataattagt atcgttttca tactgatact agatcggagg 360
   tcaccatacc cgtgaagatt tttctgtgag aggaaaagaa cccaaggacg aggttcaaat 420
   ctacacatgg aaagacgcca cgcttcgtga gttaactgac cttgtatgtc gccatcgctt 480
   agcgtagcgc tgaacatcgt tttcaccctg ctccatccat caaccattta ttggtcttat 540
   acatgtgtga ttgcgttgtt cttacattta ggtgaaagac gtttctccag ctgctaggag 600
   tcgagatgcg aaattgtcgt ttgcgactgt ataccttgat agaaatggat gcatgcaagt 660
   aaagaaggta tcttctaatt catctttcgt agagacatag cgtgaatttg gacggggtct 720
   ttggtttgag aaagataaca gctttacgta tttttgtaga tgggtgaaac cttttcaaat 780
   ccgtataagc gtaaagacga caactgggct ttaggggaca cattctttca ggtataattg    840
   atgcgactaa caatagtctc cactgatcat attctactct tctacgttcg atactgactg    900
   tttctggtta tttggtagac aggagattat ttggacgtag caattcagta gcgtagagat    960
   gtttccacac gtgttatcgt aaaagaagca agataagcct aatgcctagg gtggtggtat   1020
   gacttccgtt gcttatcgat cgtgcttgta agtaatttcc gtcttatctt ttcctgttat   1080
   ataaagttaa tcttctctag gactttcatg aaccttgttt gtgtatttat ttctcgatca   1140
   acatgataga gctagttttt aagcaacgta tactagtagt ctattggaag ttaagacacg   1200
   gttcttaaaa aggtacgatc caagtgaagc atgttagata tgacactttc ttctagggac   1260
   gactctcgta tgccacccga ctttttcaat tttttttgtg aatgttagat gtgtgtatat   1320
   aatgcatccg aaagatgtct caacgaacaa atgagccacc tacttcgatc actcgctatc   1380
   aatgttatta atgccttgtt gattttaata gttgatcaat aatagtaaaa tctattcaag   1440
   ggtatagtct cccgttcaca ctcatcgggg ttacactagc gagctccatt aatcggtgcc   1500
   ttaatcgaga cgctaagaac tataccatga cctagtcagc gccatgggac tgatgtaggc   1560
   cacacaatct cgatgatccg aaaacgctag agttcaagac ctagttcgag accatggtca   1620
   cggtttcaac cgcgatatct caacaatgca atttttttcg agactagaca gacgaataag   1680
   tcttgtgtac gatgggtagc tagtgaatta aaggtaatca ctttactcgt gttcacaaga   1740
   agaccattca tatgaacatt tcgtgttctc agacgagctc ctttcgctag tttggtaaac   1800
   ttgtttggtg ttacctggct tatcatagcc cactcaattt ggcgaaaaca taacaattgt   1860
   ttcacatgcg aaggtctaca cacccatact cgtgataaaa acggttgcat ttattattat   1920
   tgcctttcga gcaatttacg ttgtttgaag tttgtgtaaa aacaaaatcg atctataatt   1980
   actgactgga acggtaatat caaaactgtt aggacctgtt cttttcggct tataagagct   2040
   gaacttataa aagctgtact tataggagct gagctgaact gaacttataa gagttgaact   2100
   gaacttataa gggctgaaat taagctgtaa agaacaggtt cttatttcgc ctgatttgaa   2160
   tccgactaaa gttggatgaa gatagaggcg agaaacgctc ccctttccac agtttgactc    2220
   tactcgaatc cgaccaaaat tggatgtaaa ttagaagtga gcattcctca tgccaaccac   2280
   cgttcttggt cgtgaaaaca tgatttgtta gtctgctgta tacttcccaa acccgtgata   2340
   atctgccaca cttccaacac ttaattgatt tttatcaaaa ttcctacagt tttttggttt   2400
   tactcctaaa ctatgctgtc tttttacaag ttgttacacc tttgtcaaca actttatgct   2460
   ttattatatt tcttatataa agaccatata acttccctac actaatgcca caacacttaa   2520
   aagcatacaa cacaaacctc ataa                                          2544
<210> 11
   <211> 822
   <212> DNA
   <213> artificial
<220>
   <223> carnANS 3'
<400> 11
   actcactacg tgtttacgat tgtgtgtttg tcgtgtttgc ttaaatcgac catgtcctca     60
   actttgagaa atcaatagtt gtacttttga gttattgtta tctcaataac gatattattg    120
   cgttatacgg agtactgttt agaagacgat atgtaataaa tgaaatcgta gttgtctata    180
   gcttcatgat tatcgttaca ctattattct tatacttcat ctttatttta gttaatctta    240
   tcaaatactt cgtatattgg aaaatttcaa aaagttactt aaaaaataat aaaatacacc    300
   gaatttcaga aaactcagga atattcaggc gtaataacat ccgttaatac cgaaaatatt     360
   atgaaacgtg gcaaatgttg gacggtgtgc aatatgagac acaaaaaaac aattgtgaaa     420
   aatctttggt cgtaacgaat tgcgtcctaa atcatcataa taatacaaat aaaaaaatgt     480
   cattttcatt aaatttcttc tcttctgtaa tcatagaatt ttattccaac ttcatcatct     540
   aacttcaggt acatttctcc ttctctaact tattatcatt actttttatt gttttaaatt     600
   atatttttta tgtctattaa acgatttaaa atgatcacag tttactctgt acttgtcgta     660
   aataatgtgc tgtatgcata caacagtctt attggtttac gtgaactgtt agctgtggtt     720
   gcatacaatt tagattgaac cgacagatat cgcctttact gtcggaggga gctgttacag     780
   acttacgata ctgttcgtaa atggcaagct tgatatcgaa tt                        822
<210> 12
   <211> 4573
   <212> DNA
   <213> artificial
<220>
   <223> Cineraria gF3'5'H
<400> 12
   cagctgcaag tattagtaaa agacgttcgg tttgactttt gaggtcaaca catagaaaaa      60
   ttctactcca attttactcg aagtaatgtg attttcagga aagattacaa agaaactcgt     120
   aacatattaa atatgggaca atattagtat taagaactta cccagattca aatcagtttg     180
   aaaatttgaa agttatatat aaagataaaa tttgacctct caaggtcaaa cagagaaatc     240
   caactccgtt tatacacaac cttaacgaaa ttttaagaaa atatcaacga ttaccaaaac     300
   agttctaaca tgttaacacg tggaaacgat tcgtctcttg agactaagta aattatattt     360
   acattaatgt gtgattctga aaaaaggtcg tcaaaatatc tattaaatct aatgtacctg 420
   tagattatgg gcgtgagctc gggttttgag attgggaggc gtttgaatgt agttatcttt 480
   cacaggaaca acagtgtctt tccatggacc aataccgaca acatattctt tgatatattt 540
   gaagtaacga tctgaagtga tttctgcata cgtctgcaaa tgaaaaagaa atcagattat 600
   aaacatccat tgcaaactat ccttgcatcg tgtttggatg ttcgttttaa gcgagtattt 660
   tatggaatag ggagaatcag acaattagtt gtaataaaac atgatcttta attgtgctac 720
   tagtttaagt tataatgata atagaaaaca tttagtcttc ggaaaattat ataaattacc 780
   aaaaatgggt ttaactgttt caaaccaaaa gtggcaagat gtcaggtcgg atggattggg 840
   taacgggtca aaatgggttg gactgaaaca tgttcaaaca tagcgcgtag gccgtagaga 900
   ttacaaaaat tctccgttcc aaataaggtt aacagatatg actatgctga ctttttaagt 960
   gtcaaatgcg attctctttt ccggtatgca taaaaaactg acgacggaca ttacactata 1020
   taaaaattta gaaggttata ataaaccaag aaaatataat tgtattaaat tgtgtgagtt 1080
   atatgaatta catagaacct tttatatatg gttgaattac cttgctgaac aagaaaccta 1140
   aacctattag aaatgtctca aaaatcctaa gcttcaggaa taccttcccg gccttagcga 1200
   cgaggaagat atgctagagt gtatgtgtga ctcgttaaaa tcatgaacta gaacaaaggg 1260
   aaaggaacaa tgttacaatc tcaatgatta gataggatat aactcgataa caaacctaac 1320
   cagcagagtt agatcaagtg gtaagtcttt gcctttgaag acataggtcg agggttcgat 1380
   cctcactcca tgtggtcgga ggtttattgg tgaatgcatg cttagctacc gttcaaagta 1440
   actttattgg tgaatgcatg cttagctacc gttcaaaatc ttcaaaaagg gtaattatgt 1500
   ctaatatgcc atctaagttc taaccaaccc ttcaaatgtt cattcctata attactaacc 1560
   aattcttacg ttgtcaagta aataaaatga gcattctaac cctaatctgc accttcatca 1620
   ctggtttgat gttctatggg ttggttaatt tgcttagccg tcgcgctagc cgtcttcctc 1680
   caggtccaac cccatggcca atcatcggca acctaatgca ccttggtaaa cttccacatc 1740
   actcgctggc ggacttggcg aaaaagtatg gtccgttgat acatgtccga ctagggtccg 1800
   ttgatgttgt ggtggcctcg tctgcgtccg ttgctgggca gtttttaaag gtgcatgatg 1860
   cgaattttgc caacaggcca ccaaattctg gagctaaaca tatggcgtat aattatcatg 1920
   atatggtgtt tgcgccgtat ggtccaaggt ggcgaatgct tcgaaagatg tgctccatgc 1980
   atctgttttc tgccaaagca ctcactgatt ttcgtcaagt tcgacaggtt ttgtactttc 2040
   actttcgtca tatatatagg gagattagta cgagaacgaa cacttttaaa atcacttttt 2100
   aataatcaaa atatcttttt ttttttaaac aaaatcatgg aatcttattc aaataacttt 2160
   tctaaccttc taaatttttt ttaatttttt aatttttttt ttacttacag tgattaagat 2220
   aatcacataa aatatataga taatcacatg aaattttttg tgattattta gttcaaatac 2280
   attattatcg atatattttt tgtgattatc ttaaccaccg taaaaaaaat tcaaaaataa 2340
   aataaaatct gagaaggtta aaaaagttat ataaataaga ttttccgatt ttgttttcaa 2400
   caataaaata aaatttcaga acgtaataaa aattgatttt ttgttaacga gagtttgtaa 2460
   caatagacgg tcaacggaaa atgtgtatta tctggtggta tcaccatcgg attatgccaa 2520
   gcatgcataa aaaaacaaaa tcgtaactac aggaggaggt aacgatactc acgcgcgttt 2580
   tggccaggac tggacaatcg gcagtgaaac tagatcaaca acttaacgtg tgcttcgcaa 2640
   acacattatc ccgaatgatg ttagacagga gagtatttgg agacggtgat ccaaaggcgg 2700
   acgactacaa ggatatggtg gttgagttga tgactttggc cggacaattc aacatcggtg 2760
   actacattcc ttggcttgac ttgcttgacc tacaaggcat tgtcaaaagg atgaagaaag 2820
   ttcattctca attcgattcg ttccttgaca ccatcattga tgaacatact attggcacgg 2880
   gccgtcatgt tgacatgtta agcacaatga tttcactcaa agataatgcc gatggagagg 2940
   gagggaagct ttcgttcatc gagatcaaag ctcttctact ggtgcgcgta atacatagta 3000
   gtcaactttt ttttttttct ggtaatgact ctttgagcag gtaaaatgtc cccaacagga 3060
   atcaaacttg gtacctatca tttttgggaa aaattttaaa agtactagct ttttcaaaaa 3120
   gattatgaaa agtatctgtt tttctggacg attgttaaat ctaccccaaa cgcatgtctt 3180
   atatgcgttc ccttaatcaa acgttgaggg tgcgcatatg gtacatgcat accctccaaa 3240
   ggagttccca tgcacgttga gggtgcacat atacacatgc gcaccctctt cgtggtttgc 3300
   caccaaggca aatcctggag gacagtcaac ctttttgata taagttcaga tctaactcta 3360
   ggctaatact gttgatgttt cagaacttgt tctcagcggg aacggacacg tcatctagta 3420
   ccgtggaatg gggaatagcg gaactcattc gccacccaca gctaatgaaa caagcgcaag 3480
   aagaaatgga cattgtagtt ggaaaaaacc ggcttgtaac agaaatggac ataagccaac 3540
   taacattcct tcaagccatt gtgaaagaaa cgtttaggct acaccccgcg acgccacttt 3600
   ccctgccaag gattgcatca gaaagctgtg aggtcaaggg gtatcatgtt cctaagggat 3660
   cgatactctt tgttaacgtg tgggccattg ctcgacaatc agaattgtgg accgacccac 3720
   ttgaatttcg gcctggtcgt ttcctaatcc caggagaaaa acctaatgtt gaagtgaagc 3780
   caaatgattt cgaaattgta ccattcgggg gaggacgaag gatttgtgca ggtatgagcc 3840
   tcggattgag aatggtcaat ttgcttattg caacattggt tcaagccttt gattgggaat 3900
   tggctaatgg gttagagcca gaaaagctta acatggaaga agtgtttggg attagccttc 3960
   aaagggttca acccttgttg gtgcacccga ggccaaggtt agcccgtcac gtatacggaa 4020
   cgggttaagg aaataaactg tctgtttgta agatgaatct gtttgaattt atgtattaaa 4080
   tagttatgct aagaactatt tttacaaata aaagtatatt ggtttgattg ttctcgctta 4140
   gcctttgcta aatcttagat agatgagttg tataacacat catcattaac tcacatcacg 4200
   tggtaacgat ttgtttttga gttaaaattt ttaaagaaag gaaagaaaga gaaagtaaat 4260
   ataaaaaaat ttgtgttccc gagaagtttt ttacgaagga agaggggaga aagagagaga 4320
   attttagaga aattttgagt attttacaac aaaaatcatc ctctcatttt tgggatgatt 4380
   tggaggatct tttttctttc ttttccttcg tccacttcac ctccctttct ttccaaaaaa 4440
   atctcggaaa catagcgtaa tgataaacaa aaaccaataa aaatgagcag gagcaaaccc 4500
   tagaaggacg aaatcttgaa aatttattct aagattttta aaaaaaactt ggcagttgga 4560
   aagggcggcg gat 4573
<210> 13
   <211> 504
   <212> PRT
   <213> artificial
<220>
   <223> Cineraria gF3'5'H.aa
<400> 13
   Met Ser Ile Leu Thr Leu Ile Cys Thr Phe Ile Thr Gly Leu Met Phe
   1 5 10 15
   Tyr Gly Leu Val Asn Leu Leu Ser Arg Arg Ala Ser Arg Leu Pro Pro
   20 25 30
   Gly Pro Thr Pro Trp Pro Ile Ile Gly Asn Leu Met His Leu Gly Lys
   35 40 45
   Leu Pro His His Ser Leu Ala Asp Leu Ala Lys Lys Tyr Gly Pro Leu
   50 55 60
   Ile His Val Arg Leu Gly Ser Val Asp Val Val Val Ala Ser Ser Ala
   65 70 75 80
   Ser Val Ala Gly Gln Phe Leu Lys Val His Asp Ala Asn Phe Ala Asn
   85 90 95
   Arg Pro Pro Asn Ser Gly Ala Lys His Met Ala Tyr Asn Tyr His Asp
   100 105 110
   Met Val Phe Ala Pro Tyr Gly Pro Arg Trp Arg Met Leu Arg Lys Met
   115 120 125
   Cys Ser Met His Leu Phe Ser Ala Lys Ala Leu Thr Asp Phe Arg Gln
   130 135 140
   Val Arg Gln Glu Glu Val Thr Ile Leu Thr Arg Val Leu Ala Arg Thr
   145 150 155 160
   Gly Gln Ser Ala Val Lys Leu Asp Gln Gln Leu Asn Val Cys Phe Ala
   165 170 175
   Asn Thr Leu Ser Arg Met Met Leu Asp Arg Arg Val Phe Gly Asp Gly
   180 185 190
   Asp Pro Lys Ala Asp Asp Tyr Lys Asp Met Val Val Glu Leu Met Thr
   195 200 205
   Leu Ala Gly Gln Phe Asn Ile Gly Asp Tyr Ile Pro Trp Leu Asp Leu
   210 215 220
   Leu Asp Leu Gln Gly Ile Val Lys Arg Met Lys Lys Val His Ser Gln
   225 230 235 240
   Phe Asp Ser Phe Leu Asp Thr Ile Ile Asp Glu His Thr Ile Gly Thr
   245 250 255
   Gly Arg His Val Asp Met Leu Ser Thr Met Ile Ser Leu Lys Asp Asn
   260 265 270
   Ala Asp Gly Glu Gly Gly Lys Leu Ser Phe Ile Glu Ile Lys Ala Leu
   275 280 285
   Leu Leu Asn Leu Phe Ser Ala Gly Thr Asp Thr Ser Ser Ser Thr Val
   290 295 300
   Glu Trp Gly Ile Ala Glu Leu Ile Arg His Pro Gln Leu Met Lys Gln
   305 310 315 320
   Ala Gln Glu Glu Met Asp Ile Val Val Gly Lys Asn Arg Leu Val Thr
   325 330 335
   Glu Met Asp Ile Ser Gln Leu Thr Phe Leu Gln Ala Ile Val Lys Glu
   340 345 350
   Thr Phe Arg Leu His Pro Ala Thr Pro Leu Ser Leu Pro Arg Ile Ala
   355 360 365
   Ser Glu Ser Cys Glu Val Lys Gly Tyr His Val Pro Lys Gly Ser Ile
   370 375 380
   Leu Phe Val Asn Val Trp Ala Ile Ala Arg Gln Ser Glu Leu Trp Thr
   385 390 395 400
   Asp Pro Leu Glu Phe Arg Pro Gly Arg Phe Leu Ile Pro Gly Glu Lys
   405 410 415
   Pro Asn Val Glu Val Lys Pro Asn Asp Phe Glu Ile Val Pro Phe Gly
   420 425 430
   Gly Gly Arg Arg Ile Cys Ala Gly Met Ser Leu Gly Leu Arg Met Val
   435 440 445
   Asn Leu Leu Ile Ala Thr Leu Val Gln Ala Phe Asp Trp Glu Leu Ala
   450 455 460
   Asn Gly Leu Glu Pro Glu Lys Leu Asn Met Glu Glu Val Phe Gly Ile
   465 470 475 480
   Ser Leu Gln Arg Val Gln Pro Leu Leu Val His Pro Arg Pro Arg Leu
   485 490 495
   Ala Arg His Val Tyr Gly Thr Gly
   500
<210> 14
   <211> 1586
   <212> DNA
   <213> artificial
<220>
   <223> Cineraria F3'5'H 5'
<400> 14
   cagctgcaag tattagtaaa agacgttcgg tttgactttt gaggtcaaca catagaaaaa     60
   ttctactcca attttactcg aagtaatgtg attttcagga aagattacaa agaaactcgt    120
   aacatattaa atatgggaca atattagtat taagaactta cccagattca aatcagtttg    180
   aaaatttgaa agttatatat aaagataaaa tttgacctct caaggtcaaa cagagaaatc    240
   caactccgtt tatacacaac cttaacgaaa ttttaagaaa atatcaacga ttaccaaaac    300
   agttctaaca tgttaacacg tggaaacgat tcgtctcttg agactaagta aattatattt    360
   acattaatgt gtgattctga aaaaaggtcg tcaaaatatc tattaaatct aatgtacctg    420
   tagattatgg gcgtgagctc gggttttgag attgggaggc gtttgaatgt agttatcttt    480
   cacaggaaca acagtgtctt tccatggacc aataccgaca acatattctt tgatatattt    540
   gaagtaacga tctgaagtga tttctgcata cgtctgcaaa tgaaaaagaa atcagattat    600
   aaacatccat tgcaaactat ccttgcatcg tgtttggatg ttcgttttaa gcgagtattt    660
   tatggaatag ggagaatcag acaattagtt gtaataaaac atgatcttta attgtgctac    720
   tagtttaagt tataatgata atagaaaaca tttagtcttc ggaaaattat ataaattacc    780
   aaaaatgggt ttaactgttt caaaccaaaa gtggcaagat gtcaggtcgg atggattggg    840
   taacgggtca aaatgggttg gactgaaaca tgttcaaaca tagcgcgtag gccgtagaga    900
   ttacaaaaat tctccgttcc aaataaggtt aacagatatg actatgctga ctttttaagt     960
   gtcaaatgcg attctctttt ccggtatgca taaaaaactg acgacggaca ttacactata    1020
   taaaatttta gaaggttata ataaaccaag aaaatataat tgtattaaat tgtgtgagtt    1080
   atatgaatta catagaacct tttatatatg gttgaattac cttgctgaac aagaaaccta    1140
   aacctattag aaatgtctca aaaatcctaa gcttcaggaa taccttcccg gccttagcga    1200
   cgaggaagat atgctagagt gtatgtgtga ctcgttaaaa tcatgaacta gaacaaaggg    1260
   aaaggaacaa tgttacaatc tcaatgatta gataggatat aactcgataa caaacctaac    1320
   cagcagagtt agatcaagtg gtaagtcttt gcctttgaag acataggtcg agggttcgat    1380
   cctcactcca tgtggtcgga ggtttattgg tgaatgcatg cttagctacc gttcaaagta    1440
   actttattgg tgaatgcatg cttagctacc gttcaaaatc ttcaaaaagg gtaattatgt    1500
   ctaatatgcc atctaagttc taaccaaccc ttcaaatgtt cattcctata attactaacc    1560
   aattcttacg ttgtcaagta aataaa                                         1586
<210> 15
   <211> 545
   <212> DNA
   <213> artificial
<220>
   <223> Cineraria F3'5'H 3'
<400> 15
   ggaaataaac tgtctgtttg taagatgaat ctgtttgaat ttatgtatta aatagttatg      60
   ctaagaacta tttttacaaa taaaagtata ttggtttgat tgttctcgct tagcctttgc     120
   taaatcttag atagatgagt tgtataacac atcatcatta actcacatca cgtggtaacg     180
   atttgttttt gagttaaaat ttttaaagaa aggaaagaaa gagaaagtaa atataaaaaa     240
   atttgtgttc ccgagaagtt ttttacgaag gaagagggga gaaagagaga gaattttaga     300
   gaaattttga gtattttaca acaaaaatca tcctctcatt tttgggatga tttggaggat 360
   cttttttctt tcttttcctt cgtccacttc acctcccttt ctttccaaaa aaatctcgga 420
   aacatagcgt aatgataaac aaaaaccaat aaaaatgagc aggagcaaac cctagaagga 480
   cgaaatcttg aaaatttatt ctaagatttt taaaaaaaac ttggcagttg gaaagggcgg 540
   cggat 545
<210> 16
   <211> 1000
   <212> DNA
   <213> artificial
<220>
   <223> R. rugosa DFR 5'
<400> 16
   aagcttacag tgtaattatc ttctggatct acacacaaat ccaacacttg catttcaaat 60
   aataggcata ggcaaatccc atcatttgct tctggaagtt tgtgaccaaa aagcccaact 120
   ctaatgaata tgtttgcaac tttgcaatag aactttccct ttagggaata gtattatgta 180
   gtcatcattc tttctcccta aaactggttg ttgagatgac taaaattctt gacttttaag 240
   tggtaggtgg tattgcatgg attaggtaat ggattaatgg gttgcagctc acaaaattag 300
   agagaagaaa gaaatcgaaa cctaatacga agtcaatggt ggtcttgaca cgctagcata 360
   agtctgttgt ttaaaaaaaa aaaaaaaaaa aaactgaaac tggtcctaaa tcctaagcgt 420
   gttaggaaca atattttggc taatgatatt atcacagtct ctcaaaaact gtctcatagg 480
   tgagtagctc tgctgccgaa atcgaaatat ctgattaagt tggtaaatca atttttacgt 540
   tataaaagtg gtatcgcaaa acaatcaaag taattctata taaatctgga atcacacagt 600
   atgattccac ataactagtt gtgtggatgt tatgaaggtc acatacctat atcactgctt 660
   taaagctaac cgattgttac aaaggtttca acttgtaagt acgatgagat taatttgggg 720
   tggctatcaa gtatcaacca ccacgcacgt gcatcaaccg ttaggagacc acgtgatcga 780
   gctcggaagc tgagcctaaa ccaaaacagg agcacgagcg tgttaggcta ggctgctaat 840
   gttagctggg taataataca ctccctctcc attcatcagt ataaaattat aaatatattt 900
   ttagtcacct taagaagatc gcattaaaca ttctcctttt cgaacttccc tctctcccgg 960
   agtctccgat catcacctct atctatccta agatggatcc 1000
<210> 17
   <211> 1238
   <212> DNA
   <213> artificial
<220>
   <223> R. rugosa F3H 5'
<400> 17
   aagcttctag ttagacaaaa agctactcat atttcttaca tacttgttat atgtttgtga 60
   cggtttgtct tccccttacc caatccctgg ctttggtttt tacgtgatct ccaaatactg 120
   ggtacatcgc cataaggagc atttagtata ttaggtgatc gtgacatata tctattcgga 180
   catttattga tatgaacaga gaagagaaga atggtttcta tggtatttca ttcctattca 240
   gataaaatat taacagctct aagagagttg aattaatttc tgttaaaaaa gtgatcaggt 300
   gacgagattt tagtaatgag gcataattag tagggggaaa tgatcattta tcctgaggca 360
   gaattgggac atgttcatcc gggacggctg atctactccc cacctccata atgagtattt 420
   agtatattag gccgtgacat ttatcgagtc ggccctcata aaaagtttgt gactatacca 480
   ttttggctca agtagttatg tacttatgtt gtggtggttt catatcacac gatgcatgtt 540
   tcacaaatta ataaaatttc acatacatga aacaccgtaa tcgtatttat ccgcattaac 600
   tactattttc atttatttaa aaaccatctt tctttcttct ttttcttctt gttaaaaata 660
   gcaaaaagtg aaaagaagag gttttttttt ttttttcaaa cattttcact cttctacata 720
   catcgtttca ataacattct tgaaaacact ggcggaccta tctaagggca agtgagggct 780
   catgcgtcta ctgagttttt gtttgtaatg gagtaaatta atgatttact agcttgtata 840
   caaaaaatta acctaaatat tagccctcac atcatttttt tttttttaat gacaatatat 900
   ctctgtataa atcaaattct gaggaatcgt cactgcttaa aaatgatatc caatctaggg 960
   tccatagaat gccaaatatt caagaagcca aaaaatgggg gtttggtata gcttgaggct 1020
   agtttcagtt tgtctttgtg atgactgaaa gaagagaagc tccctcacga gcgctttgag 1080
   gtagttgtag gcttgtagct gccaacccct ttttatcggc cattaaatcc ccaatatata 1140
   ccacccctca cccctcaccc cacaccaaac atcatcttct tcatcgcatt tttcacttgt 1200
   cttccccaaa acagaacgga aatatcaaga gagaattc 1238
<210> 18
   <211> 1102
   <212> DNA
   <213> artificial
<220>
   <223> BPF3'5'H#40 5'
<400> 18
   aagcttgtga tcgacatctc tctcccggct ccaacttgac ggggggtgtt aaacgtatac 60
   aagattttct aatcttgtat atttgatttt ctaatatctt gtatatttga ttttctatta 120
   tcttgtattt gaacttttgt atttccttag tatcaggaaa gttagttgta gatattattt 180
   tatatttcaa atctgtatct aatacttgcc tatataaagg ccaactaatc aatgaaatga 240
   acacatcaat tttctcaatt tctcattctc tgttttcata tctattctct attttcacat 300
   tttctgaaaa gaaagatgct tgacatgatc agagacagtt ctttcttctt catactttcg 360
   tactaaactt ctcctggtcc gcaactaatc ttccatcatt ttcttgtgat cttcacttga 420
   ggatagtctc tagaaaacgg cacggtcacg ctggataagt gtttagctag cctcgaagtt 480
   gagttgcatg aattttgcgg gtacgcaagt gacttgactc ttatcttgga cgtcttatat 540
   gctcgaccaa atgttggcca agtcgggatg ctcgggttaa gcctctctta ggtcaagttt 600
   atgagcgaac ccctttcttt gagggctctt tatttgccaa ctcgtctgcc attaaagttc 660
   tattagagct ctaatgctgt gtatgtggct accgatcacc ttcattctca gaggaatcct 720
   cttttcgaat ttctggtact ttgaaactag ctgcttcaat ttcagccact cgaattaaac 780
   actaaaacag aacattgaga ggaacgggcc ctcttccaaa tatagaaaga aacagataat 840
   gtcaaaagac acatcaacta ggtcgagata cctgctcaca tgcatcacat ctaaccaact 900
   cgagtcggac gagaaatgag ttcgtaactc gatgataata aggcaaaggt ctaaaaccac 960
   attcggttgg tggttgtgtt catggaccga tcacgtgccc taacctaacc cccgcatcca 1020
   tccaccaaca gctagtcctc gccgagtccc ccaaagttcc tatttatatc actaaagtcc 1080
   ctttttctca acatagggat cc 1102
<210> 19
   <211> 20
   <212> DNA
   <213> artificial
<220>
   <223> Hf1-5
<400> 19
   tcaaaatatt tacttcggcc 20
<210> 20
   <211> 20
   <212> DNA
   <213> artificial
<220>
   <223> Hf1-3
<400> 20
   tttcattcga catcctttgg 20
<210> 21
   <211> 20
   <212> DNA
   <213> artificial
<220>
   <223> CHSA+34
<400> 21
   acgtgacaag tgtaagtatc 20
<210> 22
   <211> 20
   <212> DNA
   <213> artificial
<220>
   <223> CHSA-782
<400> 22
   gttttccaaa tcttgacgtg 20
<210> 23
   <211> 35
   <212> DNA
   <213> artificial
<220>
   <223> C1
<400> 23
   gtacatattg tcgttagaac gcgtaatacg actca 35
<210> 24
   <211> 24
   <212> DNA
   <213> artificial
<220>
   <223> BP40-i5
<400> 24
   aggtgcatga tcggaccata cttc 24
<210> 25
   <211> 35
   <212> DNA
   <213> artificial
<220>
   <223> C2
<400> 25
   cgttagaacg cgtaatacga ctcactatag ggaga 35
<210> 26
   <211> 25
   <212> DNA
   <213> artificial
<220>
   <223> BP-i7
<400> 26
   gaccatactt cttagcgagt ttggc 25
<210> 27
   <211> 25
   <212> DNA
   <213> artificial
<220>
   <223> BP40 pro-F
<400> 27
   actcaaacaa gcatctcgcc atagg 25
<210> 28
   <211> 25
   <212> DNA
   <213> artificial
<220>
   <223> BP40 pro-HindIII-F
<400> 28
   aagcttgtga tcgacatctc tctcc 25
<210> 29
   <211> 21
   <212> DNA
   <213> artificial
<220>
   <223> BP40 pro-Nhel-F
<400> 29
   cgaggctagc taaacactta t 21
<210> 30
   <211> 20
   <212> DNA
   <213> artificial
<220>
   <223> BP40 pro-Nhel-F
<400> 30
   tttagctagc ctcgaagttg 20
<210> 31
   <211> 27
   <212> DNA
   <213> artificial
<220>
   <223> BP40 pro-BamHI-R
<400> 31
   ggatccctat gttgagaaaa agggact 27
<210> 32
   <211> 24
   <212> DNA
   <213> artificial
<220>
   <223> DFRproHindIIIF
<400> 32
   taataagctt acagtgtaat tatc 24
<210> 33
   <211> 23
   <212> DNA
   <213> artificial
<220>
   <223> DFRproHneIR
<400> 33
   ttatgctagc gtgtcaagac cac 23
<210> 34
   <211> 22
   <212> DNA
   <213> artificial
<220>
   <223> DFRproNheIF
<400> 34
   acacgctagc ataagtctgt tg 22
<210> 35
   <211> 20
   <212> DNA
   <213> artificial
<220>
   <223> DFRproBamHI-R
<400> 35
   gcttggggat ccatcttagg 20
<210> 36
   <211> 25
   <212> DNA
   <213> artificial
<220>
   <223> RrF3H-F
<400> 36
   aagcttctag ttagacaaaa agcta 25
<210> 37
   <211> 25
   <212> DNA
   <213> artificial
<220>
   <223> RrF3H
<400> 37
   ggatcctctc ttgatatttc cgttc 25
<210> 38
   <211> 36
   <212> DNA
   <213> artificial
<220>
   <223> DFRint F
<400> 38
   gcatccatgg agatcttcgt gatcctggta tgtttg 36
<210> 39
   <211> 31
   <212> DNA
   <213> artificial
<220>
   <223> DFRint R
<400> 39
   gcattctaga cttcttgttc tctacaaaat c 31
<210> 40
   <211> 20
   <212> DNA
   <213> artificial
<220>
   <223> Chrys Red F
<400> 40
   cctcatcctc gggtcagtac 20
<210> 41
   <211> 21
   <212> DNA
   <213> artificial
<220>
   <223> Chrys Red R
<400> 41
   gcaactggac cattctcaag c 21
<210> 42
   <211> 30
   <212> DNA
   <213> artificial
<220>
   <223> 3369Xba.F
<400> 42
   gcatctctag aacaccatgg cctatagtcg 30
<210> 43
   <211> 30
   <212> DNA
   <213> artificial
<220>
   <223> 3369Xba.R
<400> 43
   gcatctctag attcaagagg atccgcccac. 30
<210> 44
   <211> 1660
   <212> DNA
   <213> artificial
<220>
   <223> chrysF3'H.nt
<400> 44
   aaaatgacca ttttagcttt cgtattttac gccctcatcc tcgggtcagt actctatgta      60
   tttcttaact taagttcacg taaatccgcc agactcccac ccgggccaac accatggcct     120
   atagtcggga acttaccaca ccttggccca atcccacacc acgcactcgc ggccttagcc     180
   aagaagtacg ggccattgat gcacctgcgg ctcgggtgtg tggacgtggt tgtggccgcg     240
   tctgcttccg tagctgcaca gtttttaaaa gttcacgacg caaattttgc tagtaggccg 300
   ccaaattctg gcgcgaaaca tgtggcgtat aattatcagg atcttgtgtt tgcaccttat 360
   ggtccaaggt ggcgtttgtt aaggaagatt tgttcggtcc atttgttttc tgctaaagca 420
   cttgatgatt ttcgtcatgt tcgacaggag gaggtagcag tcctaacccg cgtactactg 480
   agtgctggaa actcaccggt acagcttggc caactactta acgtgtgtgc cacaaacgcc 540
   ttagcacggg taatgttagg taggagagtt ttcggagacg gaattgacag gtcagccaat 600
   gagttcaaag atatggtagt agagttaatg gtattagcag gagaatttaa ccttggtgac 660
   tttattcctg tacttgacct attcgaccta caaggcatta ctaaaaaaat gaagaagctt 720
   catgttcggt tcgattcatt tcttagtaag atcgttgagg agcataaaac ggcacctggt 780
   gggttgggtc atactgattt gctgagcacg ttgatttcac ttaaagatga tgctgatatt 840
   gaaggtggga agcttacaga tactgaaatc aaagctttgc ttctgaattt attcgctgcg 900
   ggaacagaca catcctctag tacagtagaa tgggcaatag ccgaactcat tcgtcatcca 960
   caaatattaa aacaagcccg agaagagata gacgctgtag ttggtcaaga ccggcttgta 1020
   acagaattgg acttgagcca actaacatac ctccaggctc ttgtgaaaga ggtgtttagg 1080
   ctccaccctt caacgccact ctccttacca agaatatcat ccgagagttg tgaggtcgat 1140
   gggtattata tccctaaggg atccacactc ctcgttaacg tgtgggccat tgcgcgagac 1200
   ccaaaaatgt gggcggatcc tcttgaattt aggccttctc ggtttttacc cgggggagaa 1260
   aagcccggtg ctgatgttag gggaaatgat tttgaagtta taccatttgg ggcaggacga 1320
   aggatttgtg cgggtatgag cctaggcttg agaatggtcc agttgctcat tgcaacattg 1380
   gtccaaactt ttgattggga actggctaac gggttagagc cggagatgct caacatggaa 1440
   gaagcgtatg gattgaccct tcaacgggct gcacccttga tggttcaccc gaagccgagg   1500
   ttagctcccc acgtatatga aagtatttaa ggactagttt ctcttttgcc tttttgtttc   1560
   gcaaaggtta atgaataaac gatttcatga ctcagatagt tatgtaaaca attgtgtttg   1620
   ctgtttatat atttatctat ttttctagaa caaaaaaaaa                         1660
<210> 45
   <211> 508
   <212> PRT
   <213> artificial
<220>
   <223> chrysF3'H.aa
<400> 45
   Met Thr Ile Leu Ala Phe Val Phe Tyr Ala Leu Ile Leu Gly Ser Val
   1               5                   10                  15
   Leu Tyr Val Phe Leu Asn Leu Ser Ser Arg Lys Ser Ala Arg Leu Pro
               20                  25                  30
   Pro Gly Pro Thr Pro Trp Pro Ile Val Gly Asn Leu Pro His Leu Gly
           35                  40                  45
   Pro Ile Pro His His Ala Leu Ala Ala Leu Ala Lys Lys Tyr Gly Pro
       50                  55                  60
   Leu Met His Leu Arg Leu Gly Cys Val Asp Val Val Val Ala Ala Ser
   65                  70                  75                  80
   Ala Ser Val Ala Ala Gln Phe Leu Lys Val His Asp Ala Asn Phe Ala
                   85                  90                  95
   Ser Arg Pro Pro Asn Ser Gly Ala Lys His Val Ala Tyr Asn Tyr Gln
               100                 105                 110
   Asp Leu Val Phe Ala Pro Tyr Gly Pro Arg Trp Arg Leu Leu Arg Lys
           115                 120                 125
   Ile Cys Ser Val His Leu Phe Ser Ala Lys Ala Leu Asp Asp Phe Arg
       130                 135                 140
   His Val Arg Gln Glu Glu Val Ala Val Leu Thr Arg Val Leu Leu Ser
   145                 150                 155                 160
   Ala Gly Asn Ser Pro Val Gln Leu Gly Gln Leu Leu Asn Val Cys Ala
                   165                 170                 175
   Thr Asn Ala Leu Ala Arg Val Met Leu Gly Arg Arg Val Phe Gly Asp
               180                 185                 190
   Gly Ile Asp Arg Ser Ala Asn Glu Phe Lys Asp Met Val Val Glu Leu
           195                 200                 205
   Met Val Leu Ala Gly Glu Phe Asn Leu Gly Asp Phe Ile Pro Val Leu
       210                 215                 220
   Asp Leu Phe Asp Leu Gln Gly Ile Thr Lys Lys Met Lys Lys Leu His
   225                 230                 235                 240
   Val Arg Phe Asp Ser Phe Leu Ser Lys Ile Val Glu Glu His Lys Thr
   245 250 255
   Ala Pro Gly Gly Leu Gly His Thr Asp Leu Leu Ser Thr Leu Ile Ser
   260 265 270
   Leu Lys Asp Asp Ala Asp Ile Glu Gly Gly Lys Leu Thr Asp Thr Glu
   275 280 285
   Ile Lys Ala Leu Leu Leu Asn Leu Phe Ala Ala Gly Thr Asp Thr Ser
   290 295 300
   Ser Ser Thr Val Glu Trp Ala Ile Ala Glu Leu Ile Arg His Pro Gln
   305 310 315 320
   Ile Leu Lys Gln Ala Arg Glu Glu Ile Asp Ala Val Val Gly Gln Asp
   325 330 335
   Arg Leu Val Thr Glu Leu Asp Leu Ser Gln Leu Thr Tyr Leu Gln Ala
   340 345 350
   Leu Val Lys Glu Val Phe Arg Leu His Pro Ser Thr Pro Leu Ser Leu
   355 360 365
   Pro Arg Ile Ser Ser Glu Ser Cys Glu Val Asp Gly Tyr Tyr Ile Pro
   370 375 380
   Lys Gly Ser Thr Leu Leu Val Asn Val Trp Ala Ile Ala Arg Asp Pro
   385 390 395 400
   Lys Met Trp Ala Asp Pro Leu Glu Phe Arg Pro Ser Arg Phe Leu Pro
   405 410 415
   Gly Gly Glu Lys Pro Gly Ala Asp Val Arg Gly Asn Asp Phe Glu Val
   420 425 430
   Ile Pro Phe Gly Ala Gly Arg Arg Ile Cys Ala Gly Met Ser Leu Gly
   435 440 445
   Leu Arg Met Val Gln Leu Leu Ile Ala Thr Leu Val Gln Thr Phe Asp
   450 455 460
   Trp Glu Leu Ala Asn Gly Leu Glu Pro Glu Met Leu Asn Met Glu Glu
   465 470 475 480
   Ala Tyr Gly Leu Thr Leu Gln Arg Ala Ala Pro Leu Met Val His Pro
   485 490 495
   Lys Pro Arg Leu Ala Pro His Val Tyr Glu Ser Ile
   500 505

## Description

### FILING DATA

This application is associated with and claims priority from US Provisional Patent Application No. 60/988,331, filed on 15 November 2007.

### FIELD

The present invention relates generally to the field of genetically modified plants. More particularly, the present invention is directed to genetically modified ornamental flowering plants expressing selected color phenotypes in desired parts of the plants. Even more particularly, the present invention provides genetically modified chrysanthemum plants expressing altered or desired inflorescence.

### BACKGROUND

Bibliographic details of references provided in the subject specification are listed at the end of the specification.

Reference to any prior art is not, and should not be taken as an acknowledgment or any form of suggestion that this prior art forms part of the common general knowledge in any country.

The flower or ornamental plant industry strives to develop new and different varieties of flowers and/or plants. An effective way to create such novel varieties is through the manipulation of flower color. Classical breeding techniques have been used with some success to produce a wide range of colors for almost all of the commercial varieties of flowers and/or plants available today. This approach has been limited, however, by the constraints of a particular species' gene pool and for this reason it is rare for a single species to have the full spectrum of colored varieties. For example, the development of novel colored varieties of plants or plant parts such as flowers, foliage and stems would offer a significant opportunity in both the cut flower and ornamental markets. In the flower or ornamental plant industry, the development of novel colored varieties of chrysanthemum is of particular interest. This includes not only different colored flowers but also anthers and styles.

Flower color is predominantly due to three types of pigment: flavonoids, carotenoids and betalains. Of the three, the flavonoids are the most common and contribute to a range of colors from yellow to red to blue. The flavonoid molecules that make the major contribution to flower color are the anthocyanins, which are glycosylated derivatives of cyanidin and its methylated derivative peonidin, delphinidin and its methylated derivatives petunidin and malvidin and pelargonidin. Anthocyanins are localized in the vacuole of the epidermal cells of petals or the vacuole of the sub epidermal cells of leaves.

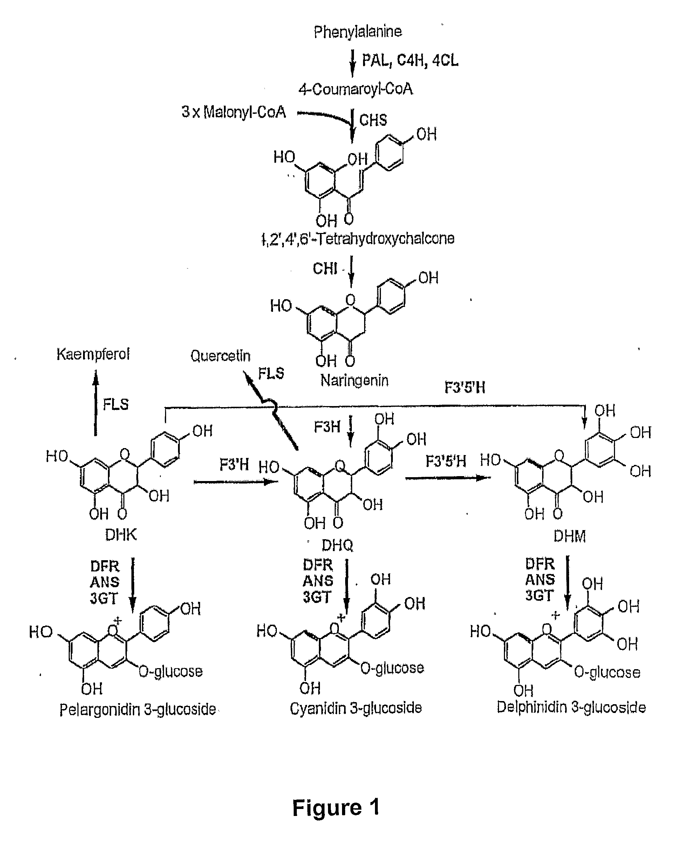

The flavonoid pigments are secondary metabolites of the phenylpropanoid pathway. The biosynthetic pathway for the flavonoid pigments (flavonoid pathway) is well established, (Holton and Cornish, Plant Cell 7:1071-1083, 1995; Mol et al, Trends Plant Sci. 3:212-217, 1998; Winkel-Shirley, Plant Physiol. 126:485-493, 2001a; and Winkel-Shirley, Plant Physiol. 127:1399-1404, 2001b, Tanaka and Mason, In Plant Genetic Engineering, Singh and Jaiwal (eds) SciTech Publishing Llc., USA, 1:361-385, 2003, Tanaka et al, Plant Cell, Tissue and Organ Culture 80:1-24, 2005, Tanaka and Brugliera, In Flowering and Its Manipulation, Annual Plant Reviews Ainsworth (ed), Blackwell Publishing, UK, 20:201-239, 2006) and is shown in Figure 1. Three reactions and enzymes are involved in the conversion of phenylalanine to *p*-coumaroyl-CoA, one of the first key substrates in the flavonoid pathway. The enzymes are phenylalanine ammonia-lyase (PAL), cinnamate 4-hydroxylase (C4H) and 4-coumarate: CoA ligase (4CL). The first committed step in the pathway involves the condensation of three molecules of malonyl-CoA (provided by the action of acetyl CoA carboxylase (ACC) on acetyl CoA and CO₂) with one molecule of *p*-coumaroyl-CoA. This reaction is catalyzed by the enzyme chalcone synthase (CHS). The product of this reaction, 2',4,4',6', tetrahydroxy-chalcone, is normally rapidly isomerized by the enzyme chalcone flavanone isomerase (CHI) to produce naringenin. Naringenin is subsequently hydroxylated at the 3 position of the central ring by flavanone 3-hydroxylase (F3H) to produce dihydrokaempferol (DHK).

The pattern of hydroxylation of the B-ring of dDHK plays a key role in determining petal color. The B-ring can be hydroxylated at either the 3', or both the 3' and 5' positions, to produce dihydroquercetin (DHQ) or dihydromyricetin (DHM), respectively. Two key enzymes involved in this part of the pathway are flavonoid 3'-hydroxylase (F3'H) and flavonoid 3', 5'-hydroxylase (F3'5'H), both members of the cytochrome P450 class of enzymes. F3'H is involved in the generation of cyanidin-based pigments which, in many plant species, contribute to red and pink flower color.

Nucleotide sequences encoding F3'5'Hs have been cloned (see International Patent Application No. PCT/AU92/00334 and Holton et al, Nature, 366:276-279, 1993 and International Patent Application No. PCT/AU03/01111). These sequences were efficient in modulating 3', 5' hydroxylation of flavonoids in petunia (see International Patent Application No. PCT/AU92/00334 incorporated herein by reference and Holton *et al,* 1993, *sura*), tobacco (see International Patent Application No. PCT/AU92/00334 carnations (see International Patent Application No. PCT/AU96/00296 and roses (see International Patent Application No. PCT/AU03/01111.

The next step in the pathway, leading to the production of the colored anthocyanins from the dihydroflavonols (DHK, DHQ, DHM), involves dihydroflavonol-4-reductase (DFR) leading to the production of the leucoanthocyanidins. The leucoanthocyanidins are subsequently converted to the anthocyanidins, pelargonidin, cyanidin and delphinidin. These flavonoid molecules are unstable under normal physiological conditions and glycosylation at the 3-position, through the action of glycosyltransferases, stabilizes the anthocyanidin molecule thus allowing accumulation of the anthocyanins. In general, the glycosyltransferases transfer the sugar moieties from UDP sugars to the flavonoid molecules and show high specificities for the position of glycosylation and relatively low specificities for the acceptor substrates (Seitz and Hinderer, Anthocyanins. In: Cell Culture and Somatic Cell Genetics of Plants, Constabel and Vasil (eds.), Academic Press, New York, USA, 5:49-76, 1988). Anthocyanins can occur as 3-monosides, 3-biosides and 3-triosides as well as 3, 5-diglycosides and 3,7-diglycosides associated with the sugars glucose, galactose, rhamnose, arabinose and xylose (Strack and Wray, In: The Flavonoids - Advances in Research since 1986. Harborne, J.B. (ed), Chapman and Hall, London, UK, 1-22, 1993).

Chrysanthemums are a highly evolved flowering plant. A member of the Asteraceae family, species of *Chrysanthemum* are herbaceous perennial plants growing to 50-150cm tall, with deeply lobed leaves and large flower heads, white, yellow or pink in the wild species. The flowers occur in various forms and can be daisy-like, decorative, pompons or buttons. The National Chrysanthemum Society (USA) divides chrysanthemums into 13 classes based on flower shape. The classes are respectively irregular incurve, reflex, regular incurve, decorative, intermediate incurve, pompon, single and semi-double, anemone, spoon, quill, spider, brush or thistle and unclassified.

The anthocyanins found in chrysanthemums are generally based on cyanidin. Delphinidin-based pigments are not present due to the lack of a F3'5'H activity and pelargonidin-based pigments are rarely found. It has been suggested that the absence of pelargonidin-based pigments in chrysanthemum is due to the presence of a F3'H activity rather than the DFR specificity. For example, when chrysanthemum petals were fed with a cytochrome P450 inhibitor, pelargonidin-based pigments were detected (Schwinn et al, Phytochemistry, 35:145-150, 1993).

Whilst classical breeding practices have produced hybrid chrysanthemums, genetic engineering has failed to produce a violet/purple to blue color in chrysanthemum flowers. There is a need to develop ornamental flowering plants and in particular chrysanthemum plants with altered inflorescence.

### SUMMARY

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

Nucleotide and amino acid sequences are referred to by a sequence identifier number (SEQ ID NO). The SEQ ID NOs correspond numerically to the sequence identifiers <400>1 (SEQ ID NO: 1), <400>2 (SEQ ID NO:2), etc.

A summary of sequence identifiers used throughout the subject specification is provided in Table 1.

The present invention provides genetically modified ornamental flowering plants and in particular genetically modified chrysanthemums exhibiting altered including selected or desired inflorescence as defined in the claims. Even more particularly, the altered inflorescence is a color in the range of red-purple to blue in chrysanthemum tissue or organelles including flowers, petals, anthers and styles. In one embodiment, the color is determined using the Royal Horticultural Society (RHS) color chart where colors are arranged in order of the fully saturated colors with the less saturated and less bright colors alongside. The color groups proceed through the spectrum and the colors referred to in this application are generally in the red-purple (RHSCC 58-74), purple (RHSCC 75-79), purple-violet (RHSCC 81-82), violet (RHSCC 83-88), violet-blue (RHSCC 89-98), blue (RHSCC 99-110) groups contained in Fan 2. Colors are selected from the range including 75C, 75D, 76A, 76B, 76C, 77C, 77D, 78D, N80D, N81B, 84C and colors in between or proximal thereto. In particular, genetically modified chrysanthemums are provided which produce delphinidin and which are purple/violet to blue in color. Non-genetically modified chrysanthemums do not produce delphinidin-based flavonoid molecules due to an absence of F3'5'H activity. Color change is facilitated by the presence of delphinidin. Conveniently, delphinidin can be detected using TLC or HPLC.

**TABLE 1**

| ***Summary of sequence identifiers*** | | | | |
|---|---|---|---|---|
| **SEQ ID NO:** | **NAME** | **SPECIES** | **TYPE OF SEQ** | **DESCRIPTION** |
| 1 | *BPF3'5'H*#*18.nt* | *Viola sp* | nucleotide¹ | *F3'5'H* cDNA |
| 2 | *BPF3'5'H#l18.aa* | *Viola sp* | amino acid | deduced F3'5'H amino acid sequence |
| 3 | *BPF3'5'H#40.nt* | *Viola sp* | nucleotide¹ | *F3'5'H* cDNA |
| 4 | *BPF3'5'H#40.aa* | *Viola sp* | amino acid | deduced F3'5'H amino acid sequence |
| 5 | *SalF3'5'H#47.nt* | *Salvia sp* | nucleotide¹ | *F3'5'H* cDNA |
| 6 | *SalF3'5H#47.aa* | *Salvia sp* | amino acid | deduced F3'5'H amino acid sequence |
| 7 | *PetHf1.nt* | *Petunia hybrida* | nucleotide² | *F3'5'H* cDNA |
| 8 | *PetHf1.aa* | *Petunia hybrida* | amino acid | deduced F3'5'H amino acid sequence |
| 9 | *RoseCHS 5'* | *Rosa hybrida* | nucleotide | promoter sequence |
| 10 | *carnANS 5'* | *Dianthus caryophyllus* | nucleotide | promoter sequence |
| 11 | *carnANS 3'* | *Dianthus caryophyllus* | nucleotide | terminator sequence |
| 12 | *Cineraria gF3'5'H* | *Cineraria sp* | nucleotide | *F3'5'H genomic* clone |
| 13 | *Cineraria gF3'5'H* | *Cineraria sp* | amino acid | deduced F3'5'H amino acid sequence |
| 14 | *Cineraria F3'5'H 5'* | *Cineraria sp* | nucleotide | promoter sequence |
| 15 | *Cineraria F3'5'H 3'* | *Cineraria sp* | nucleotide | terminator sequence |
| 16 | *R. rugosa DFR 5'* | *Rosa rugosa* | nucleotide | promoter sequence |
| 17 | *R. rugosa F3H 5'* | *Rosa rugosa* | nucleotide | promoter sequence |
| 18 | *BPF3'5'H#40 5'* | *Viola sp* | nucleotide | promoter sequence |
| 19 | *Hf1-5* | *P. hybrida* | nucleotide | primer |
| 20 | *Hf1-3* | *P. hybrida* | nucleotide | primer |
| 21 | *CHSA*+*34* | *P. hybrida* | nucleotide | primer |
| 22 | *CHSA-782* | *P. hybrida* | nucleotide | primer |
| 23 | *C1* | | nucleotide | primer |
| 24 | *BP40-i5* | *Viola sp.* | nucleotide | primer |
| 25 | *C2* | | nucleotide | primer |
| 26 | *BP-i7* | *Viola sp.* | nucleotide | primer |
| 27 | *BP40 pro-F* | *Viola sp.* | nucleotide | primer |
| 28 | *BP40 pro-HindIII-F* | *Viola sp.* | nucleotide | primer |
| 29 | *BP40pro-HneI-R* | *Viola sp.* | nucleotide | primer |
| 30 | *BP40 pro-NheI-F* | *Viola sp.* | nucleotide | primer |
| 31 | *BP40 pro-BamHI-R* | *Viola sp.* | nucleotide | primer |
| 32 | *DFRproHindIIIF* | *R. rugosa* | nucleotide | primer |
| 33 | *DFRproHneIR* | *R. rugosa* | nucleotide | primer |
| 34 | *DFRproHheIF* | *R. rugosa* | nucleotide | primer |
| 35 | *DFRproBamHI-R* | *R. rugosa* | nucleotide | primer |
| 36 | *RrF3H-F* | *R. rugosa* | nucleotide | primer |
| 37 | *RrF3H* | *R. rugosa* | nucleotide | primer |
| 38 | *DFRint F* | *Petunia* | Nucleotide | primer |
| 39 | *DFRint R* | *Petunia* | nucleotide | primer |
| 40 | *Chrys Red F* | *Chrysanthemum* | nucleotide | primer |
| 41 | *Chrys Red R* | *Chrysanthemum* | nucleotide | primer |
| 42 | *3369Xba.F* | *Chrysanthemum* | nucleotide | primer |
| 43 | *3369Xba.R* | *Chrysanthemum* | nucleotide | primer |
| 44 | *ChrysF3'H.nt* | *Chrysanthemum* | nucleotide | *F3'H* cDNA clone |
| 45 | *chrysF3'H.aa* | *Chrysanthemum* | amino acid | deduced F3'H amino acid sequence |

| | | | | |
|---|---|---|---|---|
| ¹See PCT/AU03/01111 ²See *Holton et al,* 1993, supra and PCT/AU92/00334 | | | | |

The present invention provides genetically modified chrysanthemums exhibiting altered including selected or desired inflorescence as defined in the claims. Even more particularly, the altered inflorescence is a color in the range of red-purple to blue in chrysanthemum tissue or organelles including flowers, petals, anthers and styles. Color is subjective to determine. In one embodiment, the color is determined using the Royal Horticultural Society (RHS) color chart where colors are arranged in order of the fully saturated colors with the less saturated and less bright colors alongside. The color groups proceed through the spectrum and the colors referred to in this application are generally in the red-purple (RHSCC 58-74), purple (RHSCC 75-79), purple-violet (RHSCC 81-82), violet (RHSCC 83-88), violet-blue (RHSCC 89-98), blue (RHSCC 99-110) groups contained in Fan 2. Colors are selected from the range including 75C, 75D,76A, 76B, 76C, 77C, 77D, 78D, N80D, N81B, 84C and colors in between or proximal thereto. In particular, genetically modified chrysanthemums are provided which now produce delphinidin. This is a novel feature of the genetically modified chrysanthemums and is encompassed by the term "elevated delphinidin" levels. The "elevation" is relative to zero or substantially zero levels in non-genetically modified chrysanthemum plants.

In one embodiment, the modified chrysanthemums comprise genetic sequences encoding a F3'5'H enzyme in combination with selected promoter and terminator sequences. In another embodiment, the chrysanthemum comprise genetic sequences encoding at least two F3'5'H. In yet another embodiment, the genetic sequences further encode a DFR and/or inhibit an indigenous F3'H. In yet another embodiment, the genetic sequences further encode a DFR and/or inhibit an indigenous F3'H and inhibit an indigenous DFR.

The chrysanthemums may be of any genetic background or hybrid background and include the *Chrysanthemum* sp. and *Dendranthema* sp species such as florist's chrysanthemum, *Chrysanthemum x moriflorium, Dendranthema gradiflora*, *Chrysanthemum aphrodite, Chrysanthemum arcticum, Chrysanthemum argyrophyllum, Chrysanthemum arisanense, Chrysanthemum boreale, Chrysanthemum chalchingolicum, Chrysanthemum chanetii, Chrysanthemum cinerariaefolium*, *Chrysanthemum coronarium,* Crown daisy, *Chrysanthemum crassum, Chrysanthemum glabriusculum, Chrysanthemum hypargyrum, Chrysanthemum indicum, Chrysanthemum japonese, Chrysanthemum japonicum, Chrysanthemum lavandulifolium, Chrysanthemum mawii*, *Chrysanthemum maximowiczii, Chrysanthemum mongolicum, Chrysanthemum morifolium, Chrysanthemum morii*, *Chrysanthemum okiense, Chrysanthemum oreastrum, Chrysanthemum ornatum, Chrysanthemum pacificum, Chrysanthemum potentilloides, Chrysanthemum segetum, Chrysanthemum shiwogiku, Chrysanthemum sinuatum, Chrysanthemum vestitum, Chrysanthemum weyrichii, Chrysanthemum yoshinaganthum* and *Chrysanthemum zawadskii.*

Particular varieties of interest include Blue Ridge, Madeline, Moneymaker, Mundial, Reagan and sports such as Improved Reagan and Dark Splendid Reagan, Sei Aida, Sei Figaro, Sei Faust, Sei Florea, Sei Spire, Sei Titan, Sei Titan 406, Sei Norma, Sei Amelie and Mari Fusha.

Hence, one aspect of the present invention is directed to a genetically modified ornamental flowering plant exhibiting altered inflorescence in selected tissue, the ornamental plant comprising expressed genetic material encoding an F3'5'H enzyme in combination with a selected promoter and terminator as defined in the claims.

The term "altered inflorescence" in this context means an inflorescence altered compared to the inflorescence of a plant (e.g. parent plant or a plant of the same species but not genetically modified) prior to genetic manipulation. The term "encoding" includes the expression of genetic material to produce a functional F3'5'H enzyme.

Another aspect of the present invention is directed to a chrysanthemum plant exhibiting altered inflorescence in selected tissue, the chrysanthemum comprising expressed genetic material encoding an F3'5'H enzyme in combination with a selected promoter and terminator, as defined in the claims.

Yet another aspect of the present invention is directed to a genetically modified chrysanthemum line or sport thereof exhibiting tissues of a red/purple to blue color including a violet/purple color, the chrysanthemum comprising an expressed genetic sequence encoding an F3'5'H enzyme in combination with a selected promoter and terminator, as defined in the claims.

Yet another aspect of the present invention provides a genetically modified chrysanthemum line or sport thereof exhibiting tissues of red/purple to blue color including a violet/purple color, the chrysanthemum comprising an expressed genetic sequence encoding an F3'5'H in combination with a selected promoter and terminator and an expressed genetic sequence encoding at least one other entity selected from the list comprising another F3'5'H, a DFR and an inhibitory nucleic acid sequence for an indigenous F3'H and/or DFR genetic sequence. An "indigenous" F3'H is an F3'H normally resident and expressed in the chrysanthemum parent line. An "indigenous" DFR is a DFR normally resident and expressed in the chrysanthemum parent line. This definition is the same for non-chrysanthemum plant species contemplated herein An "inhibitory" nucleic acid sequence includes *inter alia* antisense, sense, hairpin, double stranded RNA, RNA*i* and ribozyme.

Reference to a chrysanthemum comprising a genetic sequence means that the cells of the chrysanthemum plant contain the genetic material.

The present invention provides, therefore, a genetically modified chrysanthemum plant which comprises genetic material selected from the list comprising:
(i) a nucleotide sequence encoding an F3'5'H operably linked to a selected promoter and terminator, as defined in the claims.
(ii) a nucleotide sequence encoding an F3'5'H operably linked to a selected promoter and terminator, as defined in the claims and a heterologous DFR;
(iii) a nucleotide sequence encoding an F3'5'H operably linked to a selected promoter and terminator, as defined in the claims and a heterologous DFR and genetic material to down-regulate an indigenous F3'H;
(iv) a nucleotide sequence encoding an F3'5'H operably linked to a selected promoter and terminator, as defined in the claims and genetic material to down-regulate an indigenous DFR; and
(v) a nucleotide sequence encoding an F3'5'H operably linked to a selected promoter and teminator, as defined in the claims and a heterologous DFR and genetic material to down-regulate an indigenous F3'H and genetic material to down-regulate an indigenous DFR.

Progeny, reproductive material, cut flowers, tissue culturable cells and regenerable cells from the genetically modified chrysanthemums, as defined in the claims also form part of the present invention.

The present invention is also directed to the use of a genetic sequence encoding an F3'5'H enzyme, as defined in the claims in the manufacture of a chrysanthemum or sports thereof exhibiting altered inflorescence including tissue having a red-purple to blue color including a violet/purple to blue color. The genetic sequence may also encode a second F3'5'H, a DFR or a nucleic acid molecule which inhibits expression of a F3'H genetic sequence, or a nucleic acid molecule which inhibits expression of a DFR genetic sequence.

The F3'5'H described herein may be from any source. An F3'5'H enzyme from *Viola* sp; or *Cineraria* sp, is particularly useful. Suitable nucleotide sequences encoding an F3'5'H enzyme from *Viola* sp, or *Cineraria* sp are set forth in Table 1.

The genetically modified chrysanthemum plants of the present invention produce enables production of delphinidin, a feature not present-based flavonoid molecules including delphinidin in the plants prior to genetic modificationcluysanthemums which are genetically modified to express a F3'5'H. This enables production of novel colors and altered inflorescence.

### BRIEF DESCRIPTION OF THE FIGURES

**Figure 1** is a schematic representation of the biosynthesis of anthocyanidin 3-glucosides that occur in most plants that produce anthocyanins. Enzymes involved in the pathway have been indicated as follows: PAL = Phenylalanine ammonia-lyase; C4H = Cinnamate 4-hydroxylase; 4CL = 4-coumarate: CoA ligase; CHS = Chalcone synthase; CHI = Chalcone flavanone isomerase; F3H = Flavanone 3-hydroxylase; DFR = Dihydroflavonol-4-reductase; ANS = Anthocyanidin synthase, 3GT = UDP-glucose: flavonoid 3-*O*-glucosyltransferase. Other abbreviations include: DHK = dihydrokaempferol, DHQ = dihydroquercetin, DHM = dihydromyricetin.

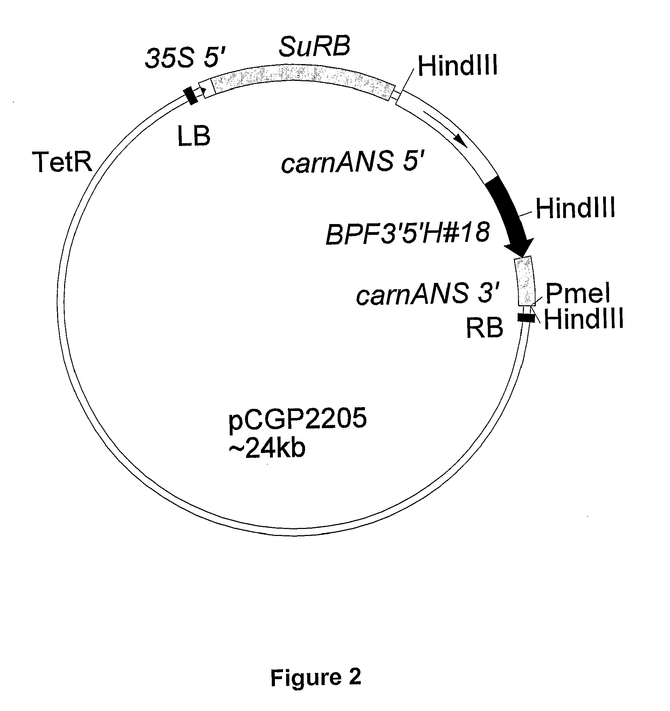

**Figure 2** is a diagrammatic representation of plasmid pCGP2205 comprising *carnANS 5': BPF3'5'H#18: carnANS 3'* and *35S 5': SuRB* expression cassettes. Abbreviations include: RB = right border of the T-DNA, LB = left border of the T-DNA, TetR = tetracycline resistance gene which confers resistance to the antibiotic tetracycline. See Table 3 for gene expression cassette details. Selected restriction endonuclease sites are marked.

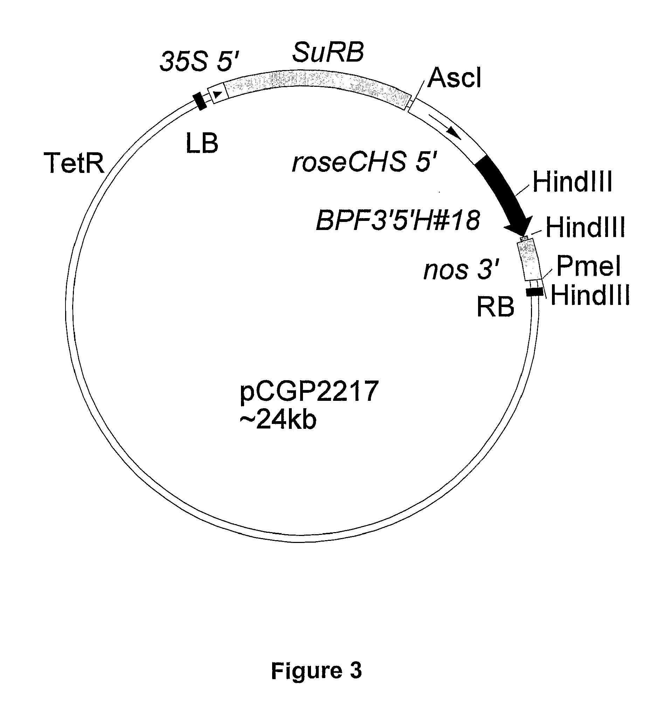

**Figure 3** is a diagrammatic representation of plasmid pCGP2217 comprising *RoseCHS 5': BPF3'5'H#18: nos* 3' and *35S 5': SuRB* expression cassettes. Abbreviations include: RB = right border of the T-DNA, LB = left border of the T-DNA, TetR = tetracycline resistance gene which confers resistance to the antibiotic tetracycline. See Table 3 for gene expression cassette details. Selected restriction endonuclease sites are marked.

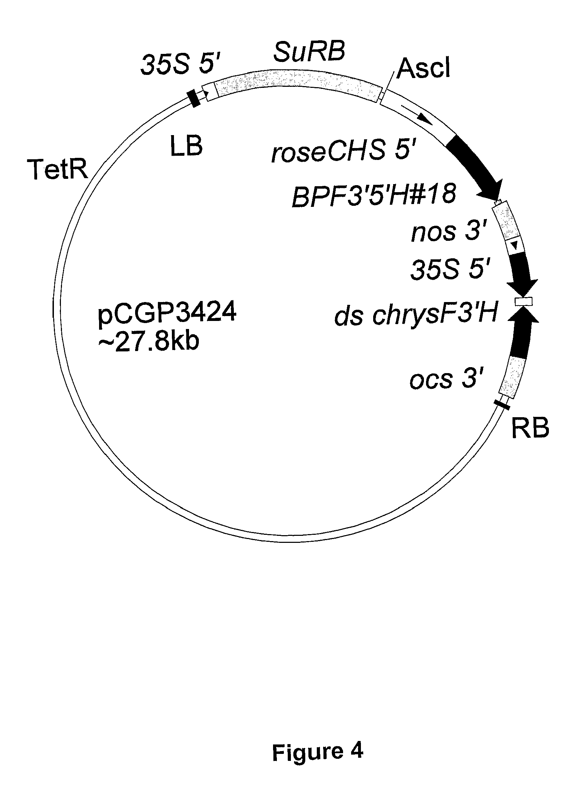

**Figure 4** is a diagrammatic representation of plasmid pCGP3424 comprising *RoseCHS 5': BPF3'5'H#18: nos 3', 35S 5': ds chrysF3'H: ocs* 3' and *35S 5': SuRB* expression cassettes. Abbreviations include: RB = right border of the T-DNA, LB = left border of the T-DNA, TetR = tetracycline resistance gene which confers resistance to the antibiotic tetracycline. See Table 3 for gene expression cassette details.

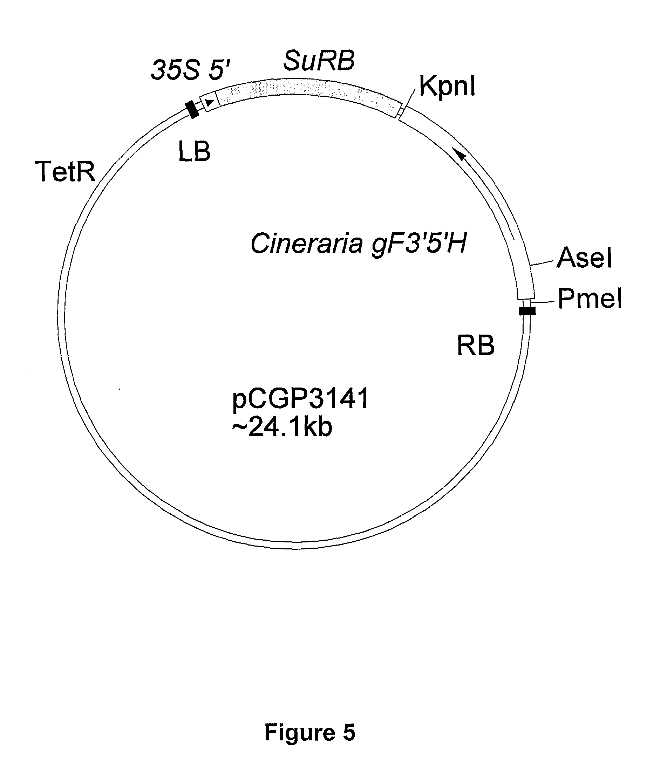

**Figure 5** is a diagrammatic representation of plasmid pCGP3141 comprising *Cinereria gF3'5'H* and *35S 5': SuRB* expression cassettes. Abbreviations include: RB = right border of the T-DNA, LB = left border of the T-DNA, TetR = tetracycline resistance gene which confers resistance to the antibiotic tetracycline. See Table 3 for gene expression cassette details.

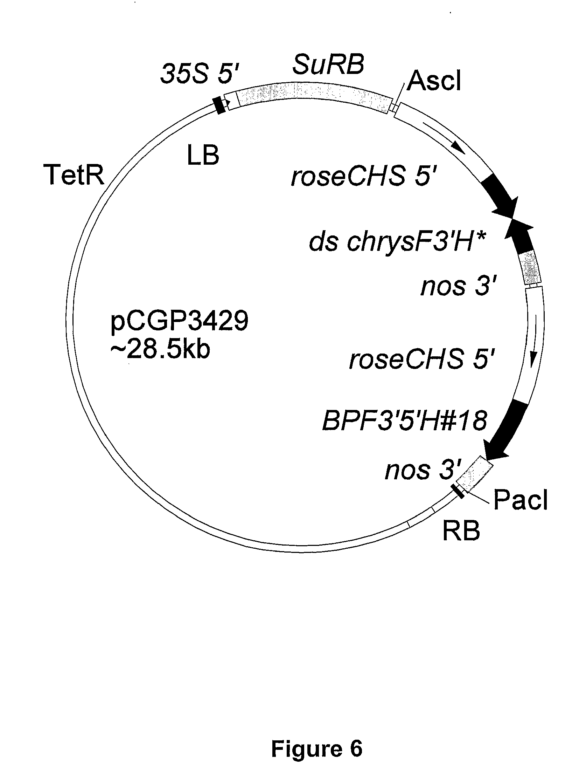

**Figure 6** is a diagrammatic representation of plasmid pCGP3429 comprising *RoseCHS 5': BPF3'5'H#18: nos 3', RoseCHS 5': ds chrysF3'H*: nos 3'* and *35S 5': SuRB* expression cassettes. Abbreviations include: RB = right border of the T-DNA, LB = left border of the T-DNA, TetR = tetracycline resistance gene which confers resistance to the antibiotic tetracycline. See Table 3 for gene expression cassette details.

### DETAILED DESCRIPTION

As used in the subject specification, the singular forms "a", "an" and "the" include plural aspects unless the context clearly dictates otherwise. Thus, for example, reference to "a plant" includes a single plant, as well as two or more plants; reference to "an F3'5'H enzyme" includes a single enzyme as well as two or more enzymes; reference to "the invention" includes a single aspect or multiple aspects of an invention; and so on.

The present invention contemplates genetically modified ornamental flowering plants and in particular chrysanthemums exhibiting altered including selected or desired inflorescence. The altered inflorescence may be in any tissue or organelle including flowers, petals, anthers and styles. Particular inflorescence contemplated herein includes a violet/purple to blue color. The color determination is conveniently measured against the Royal Horticultural Society (RHS) color chart and includes a color selected from or within the range of red-purple (RHSCC 58-74), purple (RHSCC 75-79), purple-violet (RHSCC 81-82), violet (RHSCC 83-88), violet-blue (RHSCC 89-98), blue (RHSCC 99-110) groups contained in Fan 2 of the RHS color chart. Particular colors include 75C, 75D,76A, 76B, 76C, 77C, 78D, N80D, N81B, 84C and colors in between or proximal to either end of the above range. The term "inflorescence" is not to be narrowly construed and relates to any colored cells, tissues organelles or parts thereof.

The present invention provides a genetically modified chrysanthemum exhibiting altered inflorescence compared to a plant of the same species prior to genetic modification, the genetically modified plant expressing a nucleotide sequence encoding an F3'5'H operably linked to a selected promoter and terminator as defined in the claims.

Yet another aspect of the present invention provides a genetically modified chrysanthemum exhibiting altered inflorescence compared to a plant of the same species prior to genetic modification, the genetically modified plant expressing a nucleotide sequence encoding an F3'5'H operably linked to a selected promoter and terminator as defined in the claims and further expressing a heterologous DFR nucleotide sequence.

A further aspect of the present invention provides a genetically modified chrysanthemum exhibiting altered inflorescence compared to a plant of the same species prior to genetic modification, the genetically modified plant expressing a nucleotide sequence encoding an F3'5'H operably linked to a selected promoter and terminator as defined in the claims and further expressing a heterologous DFR nucleotide sequence and genetic material which down-regulates an indigenous F3'H.

Still another aspect of the present invention provides a genetically modified chrysanthemum exhibiting altered inflorescence compared to a plant of the same species prior to genetic modification, the genetically modified plant expressing a nucleotide sequence encoding an F3'5'H operably linked to a selected promoter and terminator as defined in the claims and further expressing a heterologous DFR nucleotide sequence and genetic material which down-regulates an indigenous DFR.

Yet a further aspect of the present invention provides a genetically modified chrysanthemum exhibiting altered inflorescence compared to a plant of the same species prior to genetic modification, the genetically modified plant expressing a nucleotide sequence encoding an F3'5'H operably linked to a selected promoter and terminator as defined in the claims and further expressing a heterologous DFR nucleotide sequence and genetic material which down-regulates an indigenous F3'H and an indigenous DFR.

The genetically modified chrysanthemums now produce delphinidin-based flavonoid molecules such as delphinidin. This enables alteration of inflorescence such as generation of purple/blue colors.

The present invention provides, therefore, a genetically modified chrysanthemum plant which comprises genetic material selected from the list comprising:
(i) a nucleotide sequence encoding an F3'5'H operably linked to a selected promoter and terminator as defined in the claims;
(ii) a nucleotide sequence encoding an F3'5'H operably linked to a selected promoter and terminator as defined in the claims and a heterologous DFR;
(iii) a nucleotide sequence encoding an F3'5'H operably linked to a selected promoter and terminator as defined in the claims and a heterologous DFR and genetic material to down-regulate an indigenous F3'H;
(iv) a nucleotide sequence encoding an F3'5'H operably linked to a selected promoter and terminator as defined in the claims and genetic material to down-regulate an indigenous DFR; and
(v) a nucleotide sequence encoding an F3'5'H operably linked to a selected promoter and terminator as defined in the claims and a heterologous DFR and genetic material to down-regulate an indigenous F3'H and genetic material to down-regulate an indigenous DFR.

The genetically modified chrysanthemum plants of the present invention produce delphinidin which is not produced in non-genetically modified chrysanthemum plants. This is encomopassed by the term "elevated delphinidin" levels.

Promoters disclosed herein include the promoters from flavonoid pathway genes such as *CHS, ANS, F3'H, DFR, F3H, F3'5'H, F3'H, 3RT, MT, 3GT, AT* genes, promoter sequence from the *Rosa hybrida* chalcone synthase (*CHS*) gene (*Rose CHS 5'*), promoter sequence from the *Antirrhinum majus* chalcone synthase (*CHS*) gene (*AmCHS 5'*), cauliflower mosaic virus 35S promoter (*CaMV35S*) or fragment thereof (e.g. *35S 5'*), promoter sequence from the chrysanthemum *CHS* (*chrysCHS 5'*), a hybrid promoter consisting of the promoter from the mannopine synthase (*mas*) gene and CaMV35S enhancer region *(Mac), Petunia hybrida* phospholipid transfer protein gene promoter (*petD8 5'*), promoter sequence from the petunia anthocyanidin-3-glucoside rhamnosyltransferase (3RT) gene (*petRT 5'*), a promoter sequence from the carnation *ANS* gene (*carnANS 5'*), a promoter sequence from the cineraria *F3'5'H* gene (*Cineraria F3'5'H 5'*)*,* a promoter sequence from the petunia *F3'5'H* gene (*Pet F3'5'H 5'*) a promoter sequence from the rose *F3H* gene (*R. rugosa F3H 5'*), promoter sequence from the rose *DFR* gene (*R. rugosa DFR 5'*), promoter sequence from the *Viola F3'5'H* gene (*BPF3'5'H#40 5'*), or promoter sequence from the petunia *DFR-A* gene. Useful terminators include the terminator from *mars* gene of *A. tumefaciens* (*mas 3'*), terminator region from *nos* gene of *A. tumefaciens* (*nos 3'*), terminator fragment from octopine synthase gene (*ocs 3'*), phospholipid transfer protein gene terminator (*petD8 3'*), terminator sequence from the petunia *3RT* gene (*petRT 3'*), tumor morphology long gene terminator from *A. tumefaciens* (*tm1 3'*), a terminator sequence from carnation *ANS* gene (*carnANS 3'*), a terminator sequence from cineraria *F3'5'H* gene (*Cineraria F3'5'H 3'*), a terminator sequence from petunia *F3'5'H* gene (*Pet F3'5'H3'*) or a terminator sequence from petunia *DFR* gene (*petDFR 3'*).

Accordingly, another aspect of the present invention is directed to a genetically modified chrysanthemum line or sports thereof exhibiting tissues of a red/purple to blue color including a violet/purple color, the chrysanthemum comprising genetic sequences as defined in the claims encoding an F3'5'H enzyme operably linked to a promoter and operably linked to a terminator selected from the list comprising the *mas* gene of *A*. *tumefaciens* (*mas 3*'), terminator region from *nos* gene of *A. tumefaciens* (*nos 3'*), terminator fragment from octopine synthase gene (*ocs 3'*), phospholipid transfer protein gene terminator (*petD8 3'*), *3RT* gene terminator from Petunia (*petRT 3*'), tumor morphology long gene terminator from *A. tumefaciens* (*tm1 3'*) or *ANS* gene terminator from carnation (*carnANS 3'*), cineraria *F3'5'H* gene terminator, and a petunia *DFR* gene terminator.

Yet another aspect of the present invention is directed to a genetically modified chrysanthemum line or sports thereof exhibiting tissues of a red/purple to blue color including a violet/purple color, the chrysanthemum comprising genetic sequences as defined in the claims encoding an F3'5'H enzyme operably linked to a promoter and operably linked to a terminator sequence selected from the list comprising petunia *3RT* gene (*petRT 3*'), tumor morphology long gene terminator from *A. tumefaciens* (*tm1 3*'*)*, a terminator sequence from carnation *ANS* gene (*carnANS 3*'), a terminator sequence from cineraria *F3'5'H* gene (*Cineraria F3'5'H 3'*), a terminator sequence from petunia *F3'5'H* gene (*Pet F3'5'H 3'*) and a terminator sequence from petunia *DFR* gene.

The genetic sequence is conveniently a single construct carrying the nucleotide sequences encoding the F3'5'H enzyme or two F3'5'H enzymes operably linked to the promoter and terminator. In addition, the genetic sequence may be integrated into the genome of a plant cell or it may be maintained as an extra-chromosomal artificial chromosome. Still furthermore, the generation of a chrysanthemum expressing an F3'5'H enzyme may be generated by recombinant means alone or by a combination of conventional breeding and recombinant DNA manipulation.

Hence, the present invention contemplates a method for producing a chrysanthemum exhibiting altered inflorescence, the method comprising introducing into regenerable cells of a chrysanthemum plant, genetic material encoding an F3'5'H enzyme operably linked to a selected promoter and a terminator and regenerating a plant therefrom.

Another aspect of the present invention contemplates a method for producing a chrysanthemum exhibiting altered inflorescence, the method comprising introducing into regenerable cells of a chrysanthemum plant, genetic material encoding an F3'5'H enzyme operably linked to a selected promoter and a terminator and genetic material which down-regulates an indigenous F3'H and regenerating a plant therefrom.

Yet another aspect of the present invention contemplates a method for producing a chrysanthemum exhibiting altered inflorescence, the method comprising introducing into regenerable cells of a chrysanthemum plant, genetic material encoding an F3'5'H enzyme operably linked to a selected promoter and a terminator and genetic material expressing a heterologous DFR and regenerating a plant therefrom.

Still another aspect of the present invention contemplates a method for producing a chrysanthemum exhibiting altered inflorescence, the method comprising introducing into regenerable cells of a chrysanthemum plant, genetic material encoding an F3'5'H enzyme operably linked to a selected promoter and a terminator and genetic material expressing a heterologous DFR and genetic material which down-regulates an indigenous DFR and regenerating a plant therefrom.

Even yet another aspect of the present invention contemplates a method for producing a chrysanthemum exhibiting altered inflorescence, the method comprising introducing into regenerable cells of a chrysanthemum plant, genetic material encoding an F3'5'H enzyme operably linked to a selected promoter and a terminator and genetic material expressing a heterologous DFR and genetic material which down-regulates an indigenous F3'H and an indigenous DFR.

The genetic mutant is operably linked to a promoter to facilitate expression. The genetically manipulated plant may also then undergo various generations of growth or cultivation. Hence, reference to a "genetically modified chrysanthemum" includes progeny thereof and sister lines thereof as well as sports thereof. The present invention also extends to reproductive material from the genetically modified chrysanthemum.

Another aspect of the present disclosure provides a method for producing a chrysanthemum line exhibiting altered inflorescence, the method comprising selecting a chrysanthemum comprising genetic material encoding an F3'5'H enzyme and operably linked to a selected promoter and terminator and crossing this plant with another chrysanthemum having a desired genetic background and then selecting F1 or subsequent generation plants. The "desired genetic background" includes flower color, rigor, vacuolar pH and the like.

The present disclosure further contemplates a method for producing a chrysanthemum line exhibiting altered inflorescence, the method comprising selecting a chrysanthemum comprising genetic material encoding an F3'5'H enzyme operably linked to a selected promoter and a terminator and genetic material which down-regulates an indigenous F3'H and regenerating a plant therefrom or genetically modified progeny thereof and crossing this plant with another chrysanthemum having a desired genetic background and then selecting F1 or subsequent generation plants.

Yet another aspect of the present invention contemplates a method for producing a chrysanthemum exhibiting altered inflorescence, the method comprising introducing into regenerable cells of a chrysanthemum plant, genetic material encoding an F3'5'H enzyme operably linked to a selected promoter and a terminator and genetic material expressing a heterologous DFR and and regenerating a plant therefrom.

Still another aspect of the present invention contemplates a method for producing a chrysanthemum exhibiting altered inflorescence, the method comprising introducing into regenerable cells of a chrysanthemum plant, genetic material encoding an F3'5'H enzyme operably linked to a selected promoter and a terminator and genetic material expressing a heterologous DFR and genetic material which down-regulates an indigenous DFR and regenerating a plant therefrom.

Even yet another aspect of the present invention contemplates a method for producing a chrysanthemum exhibiting altered inflorescence, the method comprising introducing into regenerable cells of a chrysanthemum plant, genetic material encoding an F3'5'H enzyme operably linked to a selected promoter and a terminator and genetic material expressing a heterologous DFR and genetic material which down-regulates an indigenous F3'H and an indigenous DFR.

The F3'5'H enzyme may be from a source as defined in the claims.

The presence of F3'5'H enzymes leads to the pressure of production of delphinidin in the plant;. In chrysanthemum plants, this feature is a novel outcome. Delphinidin and delphinidin-based flavonoid molecules is not otherwise observednormally produced in non-geentically modified chrysanthemum plants. This facilitates production of colors new to chrysanthemum plants.

The present invention further contemplates the use of genetic sequences encoding an F3'5'H enzyme and in the manufacture of a chrysanthemum or sports thereof exhibiting altered inflorescence including tissue having a red/purple to blue color including a violet/purple color.

The present invention further contemplates the use of genetic sequences encoding an F3'5'H enzyme and also which down-regulate expression of an indigenous F3'H in the manufacture of a chrysanthemum or sports thereof exhibiting altered inflorescence including tissue having a red/purple to blue color including a violet/purple color.

The present invention also provides the use of genetic sequences which encode an F3'5'H and a heterologous DFR and also which down-regulate expression of an indigenous F3'H and/or a DFR in the manufacture of a chrysanthemum or sports thereof exhibiting altered inflorescence including tissue having a red/purple to blue color including a violet/purple color.

Generally, the genetic sequences comprise a selected promoter and terminator.

Cut flowers, tissue culturable cells, regenerable cells, parts of plants, seeds, reproductive material (including pollen) are all encompassed by the present invention.

Nucleic acid molecules encoding F3'5'Hs are also provided in International Patent Application No. PCT/AU92/00334 and Holton *et al,* 1993 *supra.* These sequences have been used to modulate 3', 5' hydroxylation of flavonoids in petunia (see International Patent Application No. PCT/AU92/00334 and Holton *et al,* 1993, *supra),* tobacco (see International Patent Application No. PCT/AU92/00334) and carnations (see International Patent Application No. PCT/AU96/00296). Nucleotide sequences of F3'5'H from other species such as *Viola, Salvia* and *Sollya* have been cloned (see International Patent Application No. PCT/AU03/01111). Any of these sequences may be used in combination with a promoter and/or terminator. The present invention particularly contemplates a F3'5'H encoded by SEQ ID NOs:1 or 12 (see Table 1) or a nucleotide sequence capable of hybridizing to SEQ ID NOs:1, or 12 or a complementary form thereof under high stringency conditions or which has at least about 70% identity to SEQ ID NO:1 or 12 after optimal alignment. In one particular embodiment, the nucleic acid molecule encoding an F3'5'H is in construct pCGP2205 which comprises *carnANS 5': BPF3'5H#18: carnANS 3'.* In yet another embodiment, the nucleic acid molecule encoding an F3'5'H is in construct pCGP3141 which comprises *Cineraria gF3*'*5*'*H*. Such plants produce delphinidin as detected by TLC or HPLC. This may be referred to as "elevated" levels of delphinidin. It is elevated relative to substantially zero amounts of delphinidin in non-genetically modified plants.

For the purposes of determining the level of stringency to define nucleic acid molecules capable of hybridizing to SEQ ID NO:1 or SEQ ID NO:12 or their complementary forms, low stringency includes and encompasses from at least about 0% to at least about 15% v/v formamide and from at least about 1M to at least about 2 M salt for hybridization, and at least about 1 M to at least about 2M salt for washing conditions. Generally, low stringency is from about 25-30°C to about 42°C. The temperature may be altered and higher temperatures used to replace the inclusion of formamide and/or to give alternative stringency conditions. Alternative stringency conditions may be applied where necessary, such as medium stringency, which includes and encompasses from at least about 16% v/v to at least about 30% v/v formamide and from at least about 0.5 M to at least about 0.9 M salt for hybridization, and at least about 0.5 M to at least about 0.9 M salt for washing conditions, or high stringency, which includes and encompasses from at least about 31% v/v to at least about 50% v/v formamide and from at least about 0.01 M to at least about 0.15 M salt for hybridization, and at least about 0.01 M to at least about 0.15 M salt for washing conditions. In general, washing is carried out Tₘ = 69.3 + 0.41 (G+C)% (Marmur and Doty, J. Mol. Biol. 5:109, 1962). However, the Tₘ of a duplex DNA decreases by 1°C with every increase of 1% in the number of mismatched base pairs (Bonner and Lackey, Eur. J. Biochem. 46:83, 1974). Formamide is optional in these hybridization conditions. Accordingly, particularly preferred levels of stringency are defined as follows: low stringency is 6 x SSC buffer, 1.0% w/v SDS at 25-42°C; a moderate stringency is 2 x SSC buffer, 1.0% w/v SDS at a temperature in the range 20°C to 65°C; high stringency is 0.1 x SSC buffer, 0.1% w/v SDS at a temperature of at least 65°C.

Reference to at least 70% identity includes 70, 71, 72, 73, 74, 75, 76, 77, 78, 79, 80, 81, 82, 83, 84, 85, 86, 87, 88, 89, 90, 91, 92, 93, ,94, 95, 96, 97, 98, 99 and 100% identity. The comparison may also be made at the level of similarity of amino acid sequences of SEQ ID NO:s:2 or 13. Hence, nucleic acid molecules are contemplated herein which encode an F3'5'H enzyme having at least 70% similarity to the amino acid sequence set forth in SEQ ID NOs:2 or 13. Again, at least 70% similarity includes 70, 71, 72, 73, 74, 75, 76, 77, 78, 79, 80, 81, 82, 83, 84, 85, 86, 87, 88, 89, 90, 91, 92, 93, ,94, 95, 96, 97, 98, 99 and 100% similarity or identity.

The nucleic acid molecule encoding the F3'5'H enzyme includes one or more promoters and/or terminators. In one aspect of the disclosure, a promoter is selected which directs expression of a F3'5'H nucleotide sequence in tissue having a higher pH.

In one aspect of the disclosure, the promoter sequence is native to the host chrysanthemum plant to be transformed or may be derived from an alternative source, where the region is functional in the host plant. Other sources include the *Agrobacterium* T-DNA genes, such as the promoters of genes encoding enzymes for biosynthesis of nopaline, octapine, mannopine, or other opines; promoters from plants, such as promoters from genes encoding ubiquitin; tissue specific promoters (see, e.g, US Patent No. 5,459,252 to Conkling et al*;* WO 91/13992 to Advanced Technologies); promoters from plant viruses (including host specific viruses), or partially or wholly synthetic promoters. Numerous promoters that are potentially functional in chrysanthemum plants (see, for example, Greve, J. Mol. Appl. Genet. 1:499-511, 1983; Salomon et al, EMBO, J. 3:141- 146, 1984; Garfinkel et al, Cell 27:143-153, 1981; Barker et al, Plant Mol. Biol. 2:235-350, 1983); including various promoters isolated from plants (such as the Ubi promoter from the maize obi-1 gene, see, e.g, US Patent No. 4,962,028) and plant viruses (such as the cauliflower mosaic virus promoter, CaMV 35S). In other aspects of the disclosure the promoter is *35S* 5', *RoseCHS 5', AmCHS 5', chrysCHS 5', petD8 5', petRT 5', carnANS 5', cineraria F3'5'H 5', BPF3'5'H#40 5', R. rugosa DFR 5*', *R. rugosa F3H 5'* and/or promoter region from the *DFR* gene of petunia with corresponding terminators *pet D8 3'*, *nos 3*', *ocs 3*', *carnANS 3*' and *petDFR 3*' (terminator region from the *DFR* gene from petunia)..

The promoter sequences may include cis-acting sequences which regulate transcription, where the regulation involves, for example, chemical or physical repression or induction (e.g, regulation based on metabolites, light, or other physicochemical factors; see, e.g, WO 93/06710 disclosing a nematode responsive promoter) or regulation based on cell differentiation (such as associated with leaves, roots, seed, or the like in plants; see, e.g. US. Patent Number. 5,459,252 disclosing a root-specific promoter).

Other cis-acting sequences which may be employed include transcriptional and/or translational enhancers. These enhancer regions are well known to persons skilled in the art, and can include the ATG initiation codon and adjacent sequences.

The nucleic acid molecule encoding an F3'5'H enzyme, in combination with suitable promoters and/or terminators is/are used to modulate the level of a flavonoid molecule such as delphinidin in a chrysanthemum. Modulating the level of a delphinidin-based molecule relates to an elevation or reduction in levels of up to 30% or more particularly of 30-50%, or even more particularly 50-75% or still more particularly 75% or greater above or below the normal endogenous or existing levels of activity. Conveniently delphinidin levels are detected by TLC or HPLC although any detection means is contemplated herein.

Hence, the present invention provides a genetically modified chrysanthemum plant comprising genetic material which is expressed to produce an F3'5'H enzyme, which produces delphinidin in tissue which contributes to the tissue being a color in the range red/purple to blue color including a violet/purple color.

The present invention further provides a genetically modified chrysanthemum plant comprising genetic material which is expressed to produce an F3'5'H enzyme and a heterologous DFR, which plant produces delphinidin in tissue which contributes to the tissue being a color in the range red/purple to blue color including a violet/purple color.

Another aspect of the present invention is directed to a genetically modified chrysanthemum plant comprising genetic material which is expressed to produce an F3'5'H enzyme and a down-regulated indigenous F3'H, which plant produces delphinidin in tissue which contributes to the tissue being a color in the range red/purple to blue color including a violet/purple color.

Yet another aspect of the present invention relates to a genetically modified chrysanthemum plant comprising genetic material which is expressed to produce an F3'5'H enzyme and a heterologous DFR and a down-regulated indigenous DFR, which plant produces delphinidin in tissue which contributes to the tissue being a color selected from a red/purple to blue color including aviolet/purple color.

Still another aspect of the present invention provides a genetically modified chrysanthemum plant comprising genetic material which is expressed to produce an F3'5'H enzyme and a heterologous DFR and a down-regulated indigenous DFR and a down-regulated F3'H, which plant produces delphinidin in tissue which contributes to the tissue being a color in the range red/purple to blue color including a violet/purple color.

The term "inflorescence" as used herein refers to the flowering part of a plant or any flowering system of more than one flower which is usually separated from the vegetative parts by an extended internode, and normally comprises individual flowers, bracts and peduncles, and pedicels. As indicated above, reference to a "transgenic plant" may also be read as a "genetically modified plant" and includes a progeny or hybrid line ultimately derived from a first generation transgenic plant.

The present invention also contemplates the use of a genetic sequence encoding an F3'5'H enzyme operably linked to a selected promoter and terminator in the manufacture of a chrysanthemum plant or sports thereof exhibiting altered inflorescence including tissue having a red/purple to blue color including a violet/purple color and which plant comprises elevated levels of delphinidin.

Another aspect of the present invention contemplates the use of a genetic sequence encoding an F3'5'H enzyme operably linked to a selected promoter and a terminator and genetic material expressing a heterologous DFR in the manufacture of a chrysanthemum plant or sports thereof exhibiting altered inflorescence including tissue having a red/purple to blue color including a violet/purple color and which plant comprises elevated levels of delphinidin.

Yet another aspect of the present invention is directed to a use of genetic material encoding an F3'5'H enzyme operably linked to a selected promoter and a terminator and and genetic material which down-regulates an indigenous F3'H in the manufacture of a chrysanthemum plant or sports thereof exhibiting altered inflorescence including tissue having a red/purple to blue color including a violet/purple color and which plant comprises elevated levels of delphinidin.

Still another aspect of the present invention contemplates use of genetic material encoding an F3'5'H enzyme operably linked to a selected promoter and a terminator and genetic material expressing a heterologous DFR and genetic material which down-regulates an indigenous DFR and in the manufacture of a chrysanthemum plant or sports thereof exhibiting altered inflorescence including tissue having a red/purple to blue color including a violet/purple color and which plant comprises elevated levels of delphinidin.

Even yet another aspect of the present invention provides a use of genetic material encoding an F3'5'H enzyme operably linked to a selected promoter and a terminator and genetic material expressing a heterologous DFR and genetic material which down-regulates an indigenous F3'H and an indigenous DFR the manufacture of a chrysanthemum plant or sports thereof exhibiting altered inflorescence including tissue having a red/purple to blue color including a violet/purple color and which plant comprises elevated levels of delphinidin.

The present invention represents a new approach to developing plant varieties and in particular chrysanthemums having altered color characteristics. In addition, the present invention enables the generation of extracts of F3'5'H transformed plants and in particular genetically modified chrysanthemum paints wherein the extract has use, for example, as a flavoring or food additive or health product or beverage or juice or coloring. Beverages may include but are not limited to teas, wines, spirits, coffee, milk and dairy products.

Reference to an "extract" includes a purified or substantially purified fraction or molecule or delphinidin-containing portion.

Another aspect of the present invention contemplates the use of the extracts from genetically modified plants or plant parts of genetically modified plants or progeny of the genetically modified plants containing all or part of the nucleic acid sequences of the present invention and, in particular, the extracts from those genetically modified plants when used as a flavoring or food additive or health product or beverage or juice or coloring. In particular, the genetically modified plant is a chrysanthemum producing delphinidin-based flavonoid compounds or molecules such as delphinidin.

In addition, the development of novel colored varieties of plant parts such as seeds offer significant opportunities in agriculture. For example, novel colored chrysanthemum seeds are useful as proprietary tags for plants.

The present invention is further described by the following non-limiting Example. In this Example, materials and methods as outlined below were employed:

Methods followed were as described in Sambrook et al, Molecular Cloning: A Laboratory Manual, Cold Spring Harbor Laboratories, Cold Spring Harbor, NY, USA, 1989 or Sambrook and Russell, Molecular Cloning: A Laboratory Manual 3rd edition, Cold Spring Harbor Laboratories, Cold Spring Harbor, NY, USA, 2001 or Plant Molecular Biology Manual (2nd edition), Gelvin and Schilperoot (eds), Kluwer Academic Publisher, The Netherlands, 1994 or Plant Molecular Biology Labfax, Croy (ed), Bios scientific Publishers, Oxford, UK, 1993.

The cloning vectors pBluescript and PCR script were obtained from Stratagene, USA. pCR7 2.1 was obtained from Invitrogen, USA.

### E. coli transformation

The *Escherichia coli* strains used were:
DH5α
   supE44, Δ (lacZYA-ArgF)U169, (ø801acZΔM15), hsdR17(rₖ, mₖ⁺),
   recA1, endA1, gyrA96, thi-1, relA1, deoR. (Hanahan, J. Mol. Biol. 166:557, 1983)
XL1-Blue
   supE44, hsdR17(rₖ⁻, mₖ⁺), recA1, endA1, gyrA96, thi-1, relAl,
   lac⁻,[F'proAB, lacI^{q}, lacZΔM15, Tn10(tet^{R})] (Bullock et al, Biotechniques 5:376, 1987).
BL21-CodonPlus-RIL strain
   *ompT hsdS*(Rb- mB-) *dcm*+ Tet^{r} *gal endA* Hte [*argU ileY leuW* Cam^{r}]
   M15 *E. coli* is derived from *E. coli* K12 and has the phenotype Nal^{s}, Str^{s}, Rif, Thi⁻, Ara⁺, Gal⁺, Mtl⁻, F⁻, RecA⁺, Uvr⁺, Lon⁺. Transformation of the *E. coli* strains was performed according to the method *of* Inoue et al, (Gene 96:23-28, 1990).

### Agrobacterium tumefaciens strains and transformations

The disarmed *Agrobacterium tumefaciens* strain used was AGL0 (Lazo et al, Bio/technology 9:963-967, 1991).

Plasmid DNA was introduced into the *Agrobacterium tumefaciens* strain AGL0 by adding 5 µg of plasmid DNA to 100 µL of competent AGL0 cells prepared by inoculating a 50 mL LB culture (Sambrook *et al,* 1989, *supra*) and incubation for 16 hours with shaking at 28°C. The cells were then pelleted and resuspended in 0.5 mL of 85% (v/v) 100 mM CaCl₂/15% (v/v) glycerol. The DNA*-Agrobacterium* mixture was frozen by incubation in liquid N₂ for 2 minutes and then allowed to thaw by incubation at 37°C for 5 minutes. The DNA/bacterial mix was then placed on ice for a further 10 minutes. The cells were then mixed with 1mL of LB (Sambrook *et al,* 1989 *supra)* media and incubated with shaking for 16 hours at 28°C. Cells of *A. tumefaciens* carrying the plasmid were selected on LB agar plates containing appropriate antibiotics such as 50 µg/mL tetracycline or 100 µg/g/mL gentamycin. The confirmation of the plasmid in *A. tumefaciens* was by restriction endonuclease mapping of DNA isolated from the antibiotic-resistant transformants.

### DNA ligations

DNA ligations were carried out using the Amersham Ligation Kit or Promega Ligation Kit according to procedures recommended by the manufacturer.

### Isolation and purification of DNA fragments

Fragments were generally isolated on a 1% (w/v) agarose gel and purified using the QIAEX II Gel Extraction kit (Qiagen) or Bresaclean Kit (Bresatec, Australia) following procedures recommended by the manufacturer.

### Repair of overhanging ends after restriction endonuclease digestion

Overhanging 5' ends were repaired using DNA polymerase I Klenow fragment according to standard protocols (Sambrook *et al,* 1989 *supra).* Overhanging 3' ends were repaired using Bacteriophage T4 DNA polymerase according to standard protocols (Sambrook *et al,* 1989 *supra*).

### Removal of phosphoryl groups from nucleic acids

Shrimp alkaline phosphatase (SAP) [USB] was typically used to remove phosphoryl groups from cloning vectors to prevent re-circularization according to the manufacturer's recommendations.

### Polymerase Chain Redaction (PCR)

Unless otherwise specified, PCR conditions using plasmid DNA as template included using 2 ng of plasmid DNA, 100 ng of each primer, 2 µL 10 mM dNTP mix, 5 µL 10 x Taq DNA polymerase buffer, 0.5 µL Taq DNA Polymerase in a total volume of 50 µL. Cycling conditions comprised an initial denaturation step of 5 minutes at 94°C, followed by 35 cycles of 94°C for 20 sec, 50°C for 30 sec and 72°C for 1 minute with a final treatment at 72°C for 10 minutes before storage at 4°C.

PCRs were performed in a Perkin Elmer GeneAmp PCR System 9600.

### ³²P-Labeling of DNA Probes

DNA fragments were radioactively labeled with 50 µCi of [α-³²P]-dCTP using a Gigaprime kit (Geneworks, AUSTRALIA) or a Decaprime kit (AMBION, USA), following methods recommended by the manufacturer. Unincorporated [α-³²P]-dCTP was removed by chromatography on Sephadex G-50 (Fine) columns or Microbiospin P-30 Tris chromatography columns (BioRad).

### Plasmid Isolation

Single colonies were analyzed for inserts by inoculating LB broth (Sambrook *et al,* 1989, *supra)* with appropriate antibiotic selection (e.g. 100 µg/mL ampicillin or 10 to 50 µg/mL tetracycline etc.) and incubating the liquid culture at 37°C (for *E. coli*) or 29°C (for *A. tumefaciens*) for ~16 hours with shaking. Plasmid DNA was purified using the alkali-lysis procedure (Sambrook *et al,* 1989, *supra)* or using The WizardPlus SV minipreps DNA purification system (Promega) or Qiagen Plasmid Mini Kit (Qiagen). Once the presence of an insert had been determined, larger amounts of plasmid DNA were prepared from 50 mL overnight cultures using the alkali-lysis procedure (Sambrook *et al,* 1989, *supra)* or QIAfilter Plasmid Midi kit (Qiagen) and following conditions recommended by the manufacturer.

### DNA Sequence Analysis

DNA sequencing was performed using the PRISM (trademark) Ready Reaction Dye Primer Cycle Sequencing Kits from Applied Biosystems. The protocols supplied by the manufacturer were followed. The cycle sequencing reactions were performed using a Perkin Elmer PCR machine (GeneAmp PCR System 9600). Sequencing runs were generally performed by the Australian Genome Research Facility at the University of Queensland, St Lucia, Brisbane, Australia and at The Walter and Eliza Hall Institute of Medical Research (Melbourne, Australia)..

Sequences were analysed using a MacVector (Trade mark) application (version 10.0.2 and earlier) [MacVector Inc, Cary, North Carolina, USA].

Homology searches against Genbank, SWISS-PROT and EMBL databases were performed using the FASTA and TFASTA programs (Pearson and Lipman, Proc. Natl. Acad. Sci. USA 85(8):2444-2448, 1988) or BLAST programs (Altschul et al, J. Mol. Biol. 215(3):403-410, 1990). Percentage sequence similarities were obtained using LALIGN program (Huang and Miller, Adv. Appl. Math. 12:373-381, 1991) or ClustalW program (Thompson et al, Nucleic Acids Research 22:4673-4680, 1994) within the MacVector (Trade mark) application (MacVector Inc, USA) using default settings.

Multiple sequence alignments were produced using ClustalW (Thompson *et al,* 1994, *supra)* using default settings.

### Plant transformation

Plant transformations are as described in International Patent Application No. PCT/US91/05805, US Patent No. 251,392 (US Registration No. 5,567,599) using appropriate selection regimes depending on the selectable marker gene cassette. Kanamycin selection for *nptII* selectable marker genes and chlorsulfuron selection for *SuRB* selectable marker genes Other methods may also be employed. For example, Aida et al, Bleeding Science, 54:51-58, 2004; Dolgov et al, Acta Hort 447, 329-334, 1997; Toguri et al, Plant Biotechnology, 20:121-127, 2003; Ledger et al, Plant Cell Reports, 10:195-199, 1991; da Silva, Biotechnology Advances 21, 715-766, 2003.

Chrysanthemum varieties were obtained from CleanGrow Australia or Seikoen, Japan.

### Transgenic Analysis

### Color- coding

The Royal Horticultural Society's Color Charts, Third and/or Fifth edition (London, UK), 1995 and/or 2007 were used to provide a description of observed color. They provide an alternative means by which to describe the color phenotypes observed. The designated numbers, however, should be taken only as a guide to the perceived colors and should not be regarded as limiting the possible colors which may be obtained.

Chrysanthemum inflorescences generally consist of many flowers called florets. There are generally two kinds of florets, ray florets and disk florets. On a daisy type of chrysanthemum such as Improved Reagan, the outer parts are ray or ligulate florets and the petals contribute to the majority of the color observed and the center is composed of disc (disk) or tubular florets (National Chrysanthemum Society, Inc., USA, 2001, Glimn-Lacy and Kaufman, Botany Illustrated, Introduction to Plants, Major Groups, Flowering Plant Families, 2nd ed, Springer, USA, 2006)

The petal colors reported in this specification are from the ray or ligulate florets.

### Chromatographic analysis

Thin Layer Chromatography (TLC) and High Performance Liquid Chromatography (HPLC) analysis was performed generally as described in Brugliera et al, (Plant J. 5:81-92, 1994). These techniques enable detection of delphinidin-based flavonoid molecules such as delphinidin.

### Extraction of anthocyanidins

Prior to HPLC analysis, the anthocyanin and flavonol molecules present in petal and stamen extracts were acid hydrolysed to remove glycosyl moieties from the anthocyanidin or flavonol core. Anthocyanidin and flavonol standards were used to help identify the compounds present in the floral extracts.

Anthocyanidins in the reaction mixture were analysed by HPLC *via* gradient elution using gradient conditions of 50%B to 60%B over 10 minutes, then 60% B for 10 minutes and finally 60% B to 100% B over 5 minutes where solvent A consisted of TFA: H₂O (5:995) and solvent B consisted of acetonitrile: TFA: H₂O (500:5:495). An Asahi Pac ODP-50 cartridge column (250 mm x 4.6 mm ID) was used for the reversed phase chromatographic separations. The flow rate was 1 mL/min and the temperature was 40°C. The detection of the anthocyanidin compounds was carried out using a Shimadzu SPD-M6A three dimensional detector at 400-650 nm.

The anthocyanidin peaks were identified by reference to know standards, *viz* delphinidin, petunidin, malvidin, cyanidin and peonidin

### Stages of flower development

Chrysanthemum inflorescences were harvested at developmental stages defined as follows:

| | |
|---|---|
| Stage 1: | Closed bud, petals not visible. |
| Stage 2: | Inflorescence buds opening: tips of petals visible. |
| Stage 3: | Tips of nearly all petals exposed. |
| Stage 4: | Outer petals at 45° angle to stem. |
| Stage 5: | Inflorescence fully open. |

For TLC or HPLC analysis, ray petals were collected from stage 4 inflorescences at the stage of maximum pigment accumulation.

For Northern blot analysis, ray petals were collected from stage 3 to 4 inflorescences at the stage of maximal expression of flavonoid pathway genes.

The *Petunia hybrida* cultivars used are presented in Table 2.

**TABLE 2**

| ***Genotypes of Petunia hybrida cultivars*** | | |
|---|---|---|
| **Plant variety** | **Propertied** | **SourcelRefer ence** |
| V23 | *An1, An2, An3, An4, An6, An8, An9, An10, ph1, Hf1, Hf2, ht1, Rt, po, Bl, Fl* | Wallroth et al. (Mol. Glen. Genet. 202:6-15, 1986) Doodeman et al. (Theor. Appl. Genet. 67:357-366, 1984) |
| Old Glory Blue (OGB) | F₁ Hybrid (commercial cultivar) | Ball Seed, USA |
| Th7 | *Art1, An2, An3, An4, An6, An9, An10, An11, Hf1, Hf2, Ht1, Ht2, Ph1, Ph2, Ph5, Rt, po, mf1, mf2, Gf, fl* | INRA |

| | | |
|---|---|---|
| INRA = Institut National de la Recherche Agronomique, Cedex, France | | |

### Northern/RNA blot analysis,

Transcription of a transferred gene was monitored by isolating RNA and estimating the quantity and size of the expected transcript. Northern blot analysis was used to monitor the steady-state level of particular transcripts in petals. A transcript was determined to be intact or full-length based on the estimated size expected from the gene used. In general when cDNAs were used as coding sequences the size of the transcript expected would be the size of the cDNA plus any 5' untranslated component of the fused promoter fragment plus any 3' untranslated sequence from the fused terminator fragment. In some cases where a cDNA region contained a putative polyadenylation site and the terminator region contained a putative polyadenylation site, 2 transcripts would be detected. One would be of a size consistent with polyadenylation occurring just downstream from the polyadenylation site within the cDNA sequence. The second transcript would be larger and consistent with the transcript being polyadenylated after the polyadenylation site within the terminator fragment.

Total RNA was isolated from petals or leaves using a Plant RNAeasy kit (QIAGEN) following procedures recommended by the manufacturer or using the method of Turpen and Griffith (*BioTeclaniques 4:*11-15, 1986).

RNA samples (5 µg) were electrophoresed through 2.2 M formaldehyde/1.2% w/v agarose gels using running buffer containing 40 mM morpholinopropanesulphonic acid (pH 7.0), 5 mM sodium acetate, 0.1 mM EDTA (pH 8.0). The RNA was stained with ethidium bromide and visualized under UV-light. The ribosomal RNA was generally used as a guide in confirming that the RNA had not been degraded by intra- or extra- cellular ribonucleases. The RNA was transferred to Hybond-N membrane filters (Amersham) and treated as described by the manufacturer.

RNA blots were probed with ³²P-labeled fragments. Prehybridization (1 hour at 42°C) and hybridization (16 hours at 42°C) of the membrane filters were carried out in 50% v/v formamide, 1 M NaCl, 1% w/v SDS, 10% w/v dextran sulphate. The membrane filters were generally washed in 2 x SSC, 1% w/v SDS at 65°C for between 1 to 2 hours and then 0.2 x SSC, 1% w/v SDS at 65°C for between 0.5 to 1 hour. Membrane filters were generally exposed to Kodak XAR film with an intensifying screen at -70°C for 16 to 72 hours.

Abbrievations used in DNA construct preparations are defined in Table 3.

**TABLE 3**

| ***Abbreviations used in construct preparations*** | |
|---|---|
| **ABBREVIATION** | **DESCRIPTION** |
| *35S 3'* | terminator fragment from *CaMV 35S* gene (Franck *et al,* 1980 *supra*) |
| *35S 5'* | promoter fragment from *CaMV 35S* gene (Franck et al, Cell 21:285-294, 1980) with an ~60bp 5' untranslated leader sequence from the petunia chlorophyll a/b binding protein gene (*Cab 22* gene) [Harpster et al, MGG, 212:182-190, 1988] |
| *AmCHS 5'* | 1.2 kb promoter fragment from the *Antirrhinum majus* chalcone synthase *(CHS)* gene (Sommer and Saedler, Mol Gen. Gent., 202:429-434, 1986) |
| *BPF3'5'H#18* | *F3'5'H* cDNA clone from *Viola sp* cv Black Pansy (see International Patent Application No. PCT/AU03/01111) [SEQ ID NO:1] |
| *BPF3'5'H#40* | *F3'4'H* cDNA clone from *Viola sp* cv Black Pansy (see International Patent Application No. PCT/AU03/01111) [SEQ ID NO:3] |
| *BPF3'5'H#40 5'* | ~2.0 kb fragment bearing the promoter region of the *Viola BPF3'5'H#40* gene (SEQ ID NO: 18) |
| *CaMV 35S* | ~0.2 kb incorporating *Bgl*II fragment containing the promoter region from the Cauliflower Mosaic Virus 35S (*CaMV 35S*) gene - (Franck *et al,* 1980 *supra,* Guilley et al, Cell, 30:763-773. 1982) |
| *carnANS 3'* | ~0.7 kb fragment containing the terminator region from the anthocyanidin synthase(*ANS*) gene of carnation (SEQ ID NO:11) |
| *carnANS 5'* | ~2.5 kb fragment containing the promoter region from the anthocyanidin synthase (*ANS*) gene of carnation (SEQ ID NO: 10) |
| *ChrysCHS 5'* | ~2.8 kb fragment containing the promoter region from a chalcone synthase *(CHS)* gene of chrysanthemum (International Patent Application No. PCT/AU03/01111) |
| *Cineraria F3'5'H 3'* | Terminator region from Cineraria *F3'5'H* gene (SEQ ID NO: 15) |
| *Cineraria F3'5'H 5'* | Promoter region from Cineraria *F3'5'H* gene (SEQ ID NO: 14) |
| *Cineraria gF3'5'H* | ~4.5kb *Cineraria F3'5'H* genomic clone with it's own promoter and terminator (SEQ ID NO: 12) |
| *ds chrysF3'H* | "double stranded (ds) chrysanthemum *F3'H"* fragment harboring a ~1.0 kb sense partial chrysanthemum *F3'H* cDNA fragment (see International PCT application PCT/AU97/00124): 180bp petunia *DFR-A* intron 1 fragment (Beld *et al, Plant Mol. Biol. 13:*491-502, 1989): ~1.1kb antisense partial chrysanthemum *F3'H* fragment (see International PCT application PCT/AU97/00124) with the aim of formation of double stranded (hairpin loop) RNA molecule to induce silencing of the endogenous chrysanthemum *F3'H* through RNAi |
| *dschrysF3'H^{*}* | "double stranded (ds) chrysanthemum *F3'H"* harboring a 1.1 kb sense partial chrysanthemum *F3'H* cDNA fragment (see International PCT application PCT/AU97/00124): 0.8kb antisense partial chrysanthemum *F3'H* fragment with the aim of formation of double stranded (hairpin loop) RNA molecule to induce silencing of the endogenous chrysanthemum *F3'H* through RNAi |
| *Mac* | Hybrid promoter consisting of the promoter from the mannopine synthase (*mas*) gene and a *CaMV 35S* enhancer region (Comai et al, Plant Mol. Biol. 15:373-381, 1990) |
| *mas 3'* | Terminator region from the mannopine synthase (*mas*) gene of *A. tumefaciens* (Janssen and Gardner, Plant Molecular Biology, 14:61-72, 1989) |
| *nos 3'* | Terminator region from the nopaline synthase (*nos*) gene of *A. tumefaciens* (Depicker et al., J Mol. and Appl. Genetics 1: 561-573, 1982) |
| *nptII* | Kanamycin-resistance gene (encodes neomycin phosphotransferase which deactivates aminoglycoside antibiotics such as kanamycin, neomycin and G418) |
| *ocs 3'* | ~1.6kb terminator fragment from octopine synthase gene of *A. tumefaciens* (described in Janssen and Gardner, 1989, *supra*) |
| *PetCHS 5'* | ~ 0.8kb promoter fragment from the chalcone synthase- A *(CHS)* gene of petunia (Koes, Genes involved in flavonoid biosynthesis in Petunia hybrida: The chalcone synthase multigene family. PhD thesis, Vrije Universiteit, Amsterdam, Netherlands, 1988) |
| *petD8 3'* | ~0.7kb terminator region from a phospholipid transfer protein gene (*D8*) of *Petunia hybrida* cv. OGB (Holton, Isolation and characterization of petal specific genes from Petunia hybrid. PhD thesis, University of Melbourne, Australia, 1992) |
| *petD8 5'* | ~3.2kb promoter region from a phospholipid transfer protein gene (*D8*) of *Petunia hybrida* (Holton, 1992 *supra*) |
| *petHf1* | Petunia *F3'5'HHf1* cDNA clone (Holton *et al,* 1993, *supra*) [SEQ ID NO:7] |
| *petRT 3'* | Terminator region of a *3RT* gene from *P. hybrida* (Brugliera, *1994, supra)* |
| *petRT 5'* | Promoter region of an anthocyanidin-3- glucoside rhamnosyltransferase (*3RT*) gene from *P. hybrida* (Brugliera, Characterization of floral specific genes isolated from Petunia hybrida. RMIT, Australia. PhD thesis, 1994) |
| *R. rugosa DFR 5'* | ~1.0 kb fragment bearing the promoter region of the *Rosa rugosa DFR* gene (SEQ ID NO: 16). |
| *R. rugosa F3H 5'* | ~1.2 kb fragment bearing the promoter region of the *Rosa rugosa F3H* gene (SEQ ID NO: 17). |
| *RoseCHS 5'* | ~2.8kb fragment containing the promoter region from a *CHS* gene of *Rosa hybrida* (see International Patent Application No. PCT/AU03/01111) [SEQ ID NO: 9] |
| *SalviaF3'5'H#47* | *F3'5'H* cDNA clone from *Salvia sp* (see International Patent Application No. PCT/AU03/01111) [SEQ ID NO:5] |
| *SuRB* | Chlorsulfuron-resistance gene [encodes Acetolactate Synthase (ALS)) with its own terminator from *Nicotiana tabacum* (Lee et al, EMBO J. 7:1241-1248, 1988) |
| *SuRB 3'* | terminator fragment from Chlorsulfuron-resistance gene *of Nicotiana tabacum* (Lee *et al,* 1988, *supra*) |
| *tml 3*' | ~2.2kb fragment containing the terminator region of tumour morphology large gene from *A. tumefaciens* (Garfinkel *et al,* 1981 *supra)* |

### EXAMPLE 1

### Introduction of chimeric F3'5'H genes into chrysanthemum

The pattern of hydroxylation of the B-ring of the anthocyanidin molecule plays a key role in determining petal color. The production of the dihydroflavonol, DHM, leads to the production of the purple/blue delphinidin-based pigments in plants such as petunia. The absence of F3'5'H activity has been correlated with the absence of blue flowers in many plant species such as *Rosa* (eg. rose), *Gerbera* (eg. gerbera), *Antirrhinum* (eg. snapdragon), *Dianthus* (eg. carnation) and *Dendranthema (eg.* chrysanthemum).

Based on success in producing delphinidin-based pigments in a mutant petunia line (Holton *et al,* 1993, *supra* and International Patent Application No. PCT/AU92/00334), in tobacco flowers (International Patent Application No. PCT/AU92/00334), in carnation flowers (International Patent Application No. PCT/AU96/00296) and in rose flowers (International Patent Application No. PCT/AU03/01111), similar chimeric *F3'5'H* genes were also introduced into chrysanthemum in order to produce selected or desired delphinidin-based pigments and modify flower color.

Chimeric *F3'5'H gene* expression cassettes are shown in Table 4.

**TABLE 4**

| ***Summary of chimeric F3'5'H gene expression cassettes contained in transformation vector constructs used in the transformation of chrysanthemums (see Table 3 for an explanation of abbreviations).*** | |
|---|---|
| **PLASMID** | **FLAVONOID PATHWAY GENES** |
| pCGP90 | *Mac 5': petHf1: mas 3'* |
| pCGP1452 | *AmCHS 5': petHf1: petD8 3'* |
| pCGP1455 | *genomic petHf1* |
| pCGP1457 | *petD8 5': petHf1: petD8 3'* |
| pCGP1476 | *PetCHS 5': petHf1: mas 3 '* |
| pCGP1616 | *petRT 5': petHf1: nos 3'* |
| pCGP1627 | *ChrysCHS 5':petHf1: nos 3'* |
| pCGP1633 | *ChrysCHS 5': petHf1: petRT3'* |
| pCGP1638 | *CaMV 35S: petHf1: ocs 3'* |
| pCGP1860 | *RoseCHS 5': petHf1: nos 3'* |
| pCGP2119 | *CaMV 35S: SalviaF3'5'H#47: ocs 3'* |
| pCGP2205 | *carnANS 5': BPF3'5'H#18: carnANS 3'* |
| pCGP2217 | *RoseCHS 5': BPF3'5'H#18: nos 3'* |
| pCGP2610 | *CaMV 35S: BPF3'5'H #18: ocs 3'; CaMV 35S: petDFR: 35S 3'* |
| pCGP2788 | *CaMV 35S: BPF3'5'H #40: 35S 3'* |
| pCGP3141 | *Cineraria gF3'5'H* |
| pCGP3149 | *35S 5': BPF3'H#40: SuRB 3'* |
| pCGP3408 | *BPF3'S'H#40 5': BPF3'5'H#40: nos 3'* |
| pCGP3409 | *R. rugosa DFR 5': BPF3'5'H#40: nos 3'* |
| pCGP3410 | *R. rugosa F3H 5': BPF3'5'H#40: nos 3'* |
| pCGP3424 | *RoseCHS 5': BPF3'5'H#18: nos 3'; 35S 5': dschrysF3'H: ocs 3'* |
| pCGP3429 | *RoseCHS 5': BBPF35H#18: nos 3'; RoseCHS 5': dschrysF3'H*: nos 3'* |
| pCGP3434 | *RoseCHS 5': BPF3'5'H#40: nos 3'* |

### Preparation of chimeric F3'5'H gene constructs

### The transformation vector pCGP90 (Mac: petHf1: mas 3')

The transformation vector pCGP90 contains a chimeric petunia *F3'5'H* (*petHf1*) gene under the control of a promoter fragment from the mannopine synthase (*mas*) gene of *Agrobacterium* and a *CaMV 35S* enhancer region with a terminator fragment from the *mas* gene of *Agrobacterium* (*mas 3'*)*.* The chimeric petunia *F3'5'H* cassette is in a tandem orientation with respect to the *35S 5*': *SuRB* selectable marker gene cassette of the plasmid pWTT2132 (DNAP) [see International Patent Application No. PCT/AU03/01111].)] Construction of the transformation vector pCGP90 is described in Holton *et al,* 1993 *sura.*

### The transformation vector pCGP1452 (AmCHS 5': petHf1: petD8 3')

The plasmid pCGP1452 contains a chimeric petunia *F3'5'H (petHf1)* gene under the control of a promoter fragment from the *Antirrhinum majus* chalcone synthase gene *(CHS)* [Sommer and Saedler, 1986, *supra*] with a terminator fragment from the petunia phospholipid transfer protein (PLTP) gene (*petD8 3'*) [Holton, 1992, *supra*]. The chimeric petunia *F3'5'H* cassette is in a tandem orientation with respect to the *35S 5*': *SuRB* gene of the plasmid pWTT2132 (DNA Plant Technologies, USA = DNAP)(see International Patent Application No. PCT/AU03/01111). Construction of pCGP1452 is described in International Patent Application No. PCT/AU03/01111.

### The transformation vectorpCGP1455 (genomic petHf1)

A chimeric genomic petunia *F3'5'H Hf1* (genomic *petHf1*) clone was constructed using various petunia *F3'5'H* genomic and cDNA clone fragments as described below.

### Construction of the intermediate plasmid pCGP1352

Initially genomic DNA isolated from the petunia cultivar V23 was completely digested with the restriction endonuclease *EcoRV.* The digested DNA was size-fractionated using a sucrose density gradient. DNA containing 1 to 3 lcb fractions was ligated with an EcoRI adaptor and then ligated with λZAPII *Eco*RI (Stratagene) and packaged to yield a petunia V23 sub-genomic library following methods as recommended by the manufacturer.

Pfus of the V23 sub-genomic library were screened with ³²P-labeled fragments of an *Eco*RI*-Eco*RV fragment from pCGP602 containing the 5' end of petunia *F3'5'H petHf1* cDNA clone representing the N-terminal portion of the protein and containing 5' noncoding sequences (Holton *et al,* 1993, *supra).* One of the hybridising clones was designated as pCGP1352 and contained around 2kb of promoter region of the petunia *F3'5'H* gene and 5' coding sequences representing the N-terminal portion of the protein.

### Construction of the intermediate plasmids, pCGP1354, pCGP1355 and 1356

The library was also screened with ³²P-labeled fragments of an *Eco*RI*-Kpn*I fragment from pCGP602 (see International Patent Application No. PCT/AU03/01111) containing the full petunia *F3*'*5*'*H* (*petHf1*) cDNA clone (Holton *et al,* 1993, *supra*) including the 3'-non coding region. One hybridising clone was designated as pCGP1354 and contained the coding region of petunia *F3'5'H Hf1* and two introns. Another 2 hybridising clones designated as pCGP1355 and pCGP1356 (both with 1.3 kb inserts) contained sequences representing the C-terminal portion of the protein along with the 3'-non coding region of the petunia *F3'5'H Hf1* gene.

### Construction of the intermediate plasmidpCGP1358

A ~2.4 kb *Bst*X1 fragment (containing the promoter region of the petunia *F3*'*5*'*H Hf1* gene and sequence representing the N-terminal portion of the F3'5'H protein) derived from pCGP1352 (described above) was ligated with *Bst*XI fragment (containing the coding region of petunia *F3'5'H Hf1* cDNA clone along with the 3'-non coding region) derived from pCGP176 (Holton *et al*, 1993, *supra*). The resulting plasmid was designated as pCGP1358.

### Construction of the intermediate plasmid pCGP1359

A whole genomic petunia *F3*'*5*'*H Hf1* clone was constructed by ligating the following fragments: an *Eco*RI (of which the recognition sequence is located in the first intron)-*Pvu*II fragment derived from pCGP1354 (described above), a *Pvu*II*-Hind*III fragment (containing most of the petunia *F3*'*5*'*H Hf1* coding region) derived from pCGP176 (Holton *et al,* 1993, *supra)* and a *Hind*III*-Eco*RI fragment (bearing sequences representing the C-terminal portion of the protein along with 3'-non coding sequences and the cloning vector pBluescript SK- (Stratagene)) of pCGP176 (Holton *et al,* 1993, *supra*)*.* The resultant plasmid was designated as pCGP1359.

### Construction of the intermediate plasmid pCGP649

A 2.5kb product harboring the genomic *Hf1* sequence was amplified using the following primers:
Hf1-5 5'TCC AAA TAT TTA CTT CGG CC 3' (SEQ ID NO:19)
Hf1-3 5' TTT CAT TCG ACA TCC TTT GG 3' (SEQ ID NO:20)
and genomic DNA isolated from *P. hybrida* cultivar Th7 as template. The amplified product was cloned into *Eco*RV ddT-filled site of pBluescript (STRATAGENE) to yield the plasmid pCGP649.

### Construction of the intermediate plasmid pCGP1360

A fragment (bearing the promoter region and sequences representing the N-terminal portion of the F3'5'H protein) was released upon digestion of the plasmid pCGP1352 (described above) with the restriction endonuclease *Bst*XI*.* The fragment was purified and ligated with the *Bst*XI ends of the plasmid pCGP649 (bearing the *Hf1* genomic sequence amplified using *P. hybrida* cultivar Th7 as template in a pBluescript SK- backbone vector)(described above) to yield the plasmid pCGP1360.

### Construction of the intermediate plasmidpCGP1361

An ~2.5 kb *Eco*RI fragment of pCGP1360 (bearing the *Hf1* promoter region, sequences encoding the N-terminal portion of the protein and a section of the first intron)(described above) was ligated with *Eco*RI ends of the plasmid pCGP1359 (described above) to yield the plasmid pCGP1361 (containing the full genomic *Hf1* sequence).

### Construction of the intermediate plasmid pCGP1363

A 4.5kb fragment was released from the plasmid pCGP1361 (described above) upon digestion with the REs *Sac*I and *Bst*BI*.* The fragment was purified and ligated with the *Sac*I and *Bst*BI ends of the plasmid pCGP1356 (described above) to yield pCGP1363.

### Construction of the transformation vector pCGP1455

A 6kb fragment bearing the petunia genomic *F3'5'H Hf1* clone was released from the plasmid pCGP1363 upon digestion with the REs *Apa*I and *Sac*I*.* The ends were repaired and the fragment was purified and ligated with *Sma*I ends of the plasmid pWTT2132. Correct insertion the the petunia gemonic *F3*'*5*'*H Hf1* clone in a tandem orientation to the selectable marker gene cassette was confirmed by restriction endonuclease analysis of plasmid DNA isolated from tetracycline-resistant transformants. The resulting plasmid was designated pCGP1455.

### The transformation vector pCGP1457 (petD8 5':petHf1:pet D8 3')

The plasmid pCGP1457 contains a chimeric petunia *F3*'*5*'*H* (*petHf1*) gene under the control of a promoter fragment from the petunia *PLTP* gene *(petD8 5'*) with a terminator fragment from the petunia *PLTP* gene (*petD8 3*'). The chimeric petunia *F3*'*5*'*H* cassette is in a tandem orientation with respect to the *35S 5*': *SuRB* gene of the plasmid pWTT2132 (DNAP) [see International Patent Application No. PCT/AU03/01111]. Construction of pCGP1457 is described in International Patent Application No. PCT/AU03/01111.

### The transformation vector pCGP1476 (PetCHS 5': petHf1: mas 3')

The transformation vector pCGP1476 contains a chimeric petunia *F3*'*5*'*H* (*petHf1*) gene under the control of a promoter fragment from the *P. hybrida CHS-A* gene (*PetCHS 5'*) [Koes, 1988 *supra]* with a terminator fragment from the mannopine synthase gene (*mas 3'*) of *Agrobacterium* (Janssen and Gardner, 1989, *supra).* The chimeric petunia *F3'5'H* cassette is in a tandem orientation with respect to the *35S 5*': *SuRB* gene of the plasmid pWTT2132 (DNAP) [see International Patent Application No. PCT/AU03/01111].

### Construction of intermediate plasmid pCGP669 (PetCHS 5' fragment in pBluescript II SK)

An ~800bp product bearing the *PetCHS* 5' promoter fragment was amplified using the following primers:
CHSA+34 5' ACGTGACAAGTGTAAGTATC 3' (SEQ ID NO:21)
CHSA-782 5' GTTTTCCAAATCTTGACGTG 3' (SEQ ID NO:22)
and genomic DNA isolated from *P. hybrida* cv. V30 as template.

The amplified product was ligated into ddT-tailed pBluescript II SK (STRATAGENE) and sequenced. The plasmid was designated pCGP669.

### Construction of intermediate plasmid pCGP672 (PetCHS 5' :mas 3')

The plasmid pCGP669 (described above) was firstly linearized by digestion with the REs *Eco*RI*.* After repairing the overhanging ends, the linearized plasmid was digested with the restriction endonuclease *Hin*dIII releasing an ~800bp fragment bearing the *PetCHS 5*' promoter fragment. The fragment was purified and ligated with the *Xba*I (repaired ends)/*Hind*III ends of the plasmid pCGP293 (a transformation vector containing *Mac 5': mas* 3' expression cassette) [described in Brugliera *et al,* 1994 *supra].* Correct replacement of the *Mac 5'* promoter fragment with the *PetCHS* 5' promoter fragment was established by restriction endonuclease analysis of plasmid DNA isolated from gentamycin-resistant transformants. The resulting plasmid containing *PetCHS 5': mas 3'* expression cassette was designated as pCGP672.

### Construction of pCGP483 (AmCHS 5': petHf1: petD8 3' in pBI221 backbone)

A chimeric petunia *F3*'*5*'*H* gene under the control *Antirrhinum majus CHS* (*AmCHS 5'*) promoter with a petunia PLTP terminator (*petD8 3'*) was constructed by cloning the 1.6kb *Bcl*I/*Fsp*I petunia *F3*'*5*'*H* (*petHf1*) fragment from pCGP601 (described in International Patent Application No. PCT/AU03/01111) between a 1.2 kb *Antirrhinum majus CHS* gene fragment 5' to the site of translation initiation (Sommer and Saedler, 1986, *supra)* and a 0.7 kb *Sma*I/*Xho*I PLTP fragment (*petD8 3'*) from pCGP13ΔBam (Holton, 1992, *supra),* 3' to the deduced stop codon. The resulting plasmid in a pBluescribe (STRATAGENE) backbone vector was designated pCGP483.

### Construction of intermediate plasmid pCGP1471 (PetCHS 5' : petHf1: mas 3')

A 1.6kb fragment harboring the *petHf1* cDNA clone was released from the plasmid pCGP483 (described above) upon digestion with the restriction endonucleases *Xba*I and *Bam*HI*.* The fragment was purified and ligated with the *Xba*I*lBam*HI ends of the plasmid pCGP672 (described above). Correct insertion of the *petHf1* cDNA clone between the *AmCHS 5'* promoter fragment and *petD8* 3' terminator fragment was established by restriction endonuclease analysis of plasmid DNA isolated from gentamycin-resistant transformants. The resulting plasmid was designated pCGP1471.

### Construction of the transformation vector pCGP1476 (PetCHS 5': petHf1: mas 3')

The plasmid pCGP1471 was firstly partially digested with the restriction endonuclease *Hind*III*.* The overhanging ends were repaired and the partially digested plasmid was further digested with the restriction endonuclease *Pst*I*.* The 3.2kb fragment harboring the *PetCHS 5' : petHf1: mas* 3' expression cassette was purified and ligated with *Sma*I/*Pst*I ends of the Ti plasmid pWTT2132 ((DNAP) [see International Patent Application No. PCT/AU03/01111]. Correct insertion of the expression cassette in a tandem orientation with respect to the *35S 5*': *SuRB* selectable marker cassette was established by restriction endonuclease analysis of plasmid DNA isolated from tetracycline-resistant transformants. The resulting transformation vector was designated as pCGP1476.

### The transformation vector pCGP1616 (petRT 5':petHf1: nos 3')

The transformation vector pCGP1616 contains a chimeric petunia *F3*'*5*'*H* (*petHf1*) gene under the control of a promoter fragment from the *P. hybrida 3RT* gene *(petRT 5'*) [Brugliera, 1994, *supra]* with a terminator fragment from the nopaline synthase gene (*nos* 3') of *Agrobacterium* (Depicker, *et al,* 1982, *supra*). The chimeric petunia *F3*'*5*'*H* cassette is in a tandem orientation with respect to the *35S 5*': *SuRB* gene of the plasmid pWTT2132 (DNAP) [see International Patent Application No. PCT/AU03/01111]. Construction of pCGP1616 is described in International Patent Application No. PCT/AU03/01111.

### The transformation vector pCGP1627 (ChrysCHS 5': petHf1: nos 3')

The transformation vector pCGP1627 contains a chimeric petunia *F3*'*5*'*H* (*petHf1*) gene under the control of a promoter fragment from the chalcone synthase (*CHS*) gene of chrysanthemum with a terminator fragment from the nopaline synthase (*nos*) gene of *Agrobacterium* (*nos 3'*)*.* The chimeric petunia *F3*'*5*'*H* cassette is in a tandem orientation with respect to the *35S 5*': *SuRB* selectable marker gene cassette of the plasmid pWTT2132 (DNAP).

### Isolation of chrysanthemum CHS promoter

Isolation of a chrysanthemum *CHS* genomic clone and preparation of the intermediate plasmid pCGP1622 containing the *chrysCHS 5*'*: GUS: nos 3'* expression cassette is described in International Patent Application No. PCT/AU03/01111).

### Construction of the intermediate plasmid pCGP1624 (chrysCHS 5': petHf1: nos 3')

The petunia *F3*'*5*'*H* (*petHf1*) cDNA clone was released from the plasmid pCGP1303 (described in International Patent Application No. PCT/AU03/01111) upon digestion with the restriction endonucleases *Bsp*HI and *Xma*I*.* The 1.6 kb fragment containing the petunia *F3'5'H (petHf1)* cDNA clone was purified and ligated with *Nco*I/*Xma*I ends of pCGP1622. Correct insertion of the fragment was established by restriction endonuclease analysis of plasmid DNA isolated from ampicillin-resistant transformants. The resulting plasmid containing *chrysCHS 5*': *petHf1: nos 3'* expression cassette was designated as pCGP1624.

### Construction of the transformation vector pCGP1627

A 4.5 kb fragment containing the *chrysCHS 5': petHf1: nos 3'* gene was released from the plasmid pCGP1624 upon digestion with the restriction endonucleases *Pst*I and *Bgl*II*.* The purified fragment was ligated with *Pst*I/*Bam*HI ends of pWTT2132 (DNAP) [see International Patent Application No. PCT/AU03/01111]. Correct insertion of the *chrysCHS 5*': *petHf1: nos 3'* gene in a tandem orientation with respect to the *35S 5*'*: SuRB* selectable marker gene cassette was established by restriction endonuclease analysis of plasmid DNA isolated from tetracycline-resistant transformants. The resulting transformation vector was designated pCGP1627.

### The transformation vector pCGP1633 (ChrysCHS 5': petHf1: petRT 3')

The plasmid pCGP1633 contains a chimeric petunia *F3*'*5*'*H* (*petHf1*) gene under the control of a promoter fragment from the chalcone synthase (*CHS*) gene of chrysanthemum with a terminator fragment from the *3RT* gene (Brugliera, 1994, *supra*) of *Petunia hybrida* (*petRT 3'*). The chimeric petunia *F3*'*5*'*H* cassette is in a tandem orientation with respect to the *35S 5*': *SuRB* selectable marker gene cassette of the plasmid pWTT2132 (DNAP).

### Construction of the intermediate plasmid pCGP1630 (chrysCHS 5': petHf1: petRT 3')

The *petRT 3'* terminator fragment was released from the plasmid pCGP1610 (Brugliera, 1994, *supra*) upon digestion with the restriction endonucleases *Sac*I and *Bam*HI*.* The 2.5 kb fragment was purified and ligated with *Sac*I*lBgl*III ends of pCGP1624 (described above). Correct insertion of the fragment was established by restriction endonuclease analysis of plasmid DNA isolated from ampicillin-resistant transformants. The resulting plasmid containing *chrysCHS 5': petHf1: petRT 3'* expression cassette was designated as pCGP1630.

### Construction of the transformation vector pCGP1633 (chrysCHS 5': petHf1: petRT 3')

A 6.6 kb fragment containing the *chrysCHS 5': petHf1: petRT 3'* gene was released from the plasmid pCGP1630 upon digestion with the restriction endonuclease *Pst*I*.* The purified fragment was ligated with *Pst*I ends of pWTT2132 (DNAP) [see International Patent Application No. PCT/AU03/01111]. Correct insertion of the *chrysCHS 5*': *petHf1: petRT 3'* gene in a tandem orientation with respect to the *35S 5*': *SuRB* selectable marker gene cassette was established by restriction endonuclease analysis of plasmid DNA isolated from tetracycline-resistant transformants. The plasmid was designated as pCGP1633.

### The transformation vector pCGP1638 (CaMV 35S: petHf1: ocs 3')

The transformation vector pCGP1638 contains a chimeric petunia *F3*'*5*'*H* (*petHf1*) gene under the control of a *CaMV 35S* promoter (*35S 5'*) with an octopine synthase terminator (*ocs 3'*). A ~60 bp 5' untranslated leader sequence from the petunia chlorophyll a/b binding protein gene (*Cab* 22 gene) [Harpster *et al,* 1988, *supra*] is included between the *CaMV 35S* promoter fragment and the petunia *F3*'*5*'*H petHf1* cDNA clone. The chimeric petunia *F3*'*5*'*H* cassette is in a tandem orientation with respect to the *35S 5*': *SuRB* selectable marker gene cassette of the Ti plasmid vector pWTT2132 (DNAP) [see International Patent Application No. PCT/AU03/01111]. Construction of the transformation vector pCGP1638 is described in International Patent Application No. PCT/AU03/01111.

### The transformation vector pCGP1860 (RoseCHS 5':petHf1: nos 3')

The transformation vector pCGP1860 contains a chimeric petunia *F3*'*5*'*H* (*petHf1*) gene under the control of a promoter fragment from the chalcone synthase gene of *Rosa hybrida (RoseCHS 5'*) with a terminator fragment from the nopaline synthase gene of *Agrobacterium* (*nos 3'*). The chimeric petunia *F3*'*5*'*H* cassette is in a tandem orientation with respect to the *35S 5*': *SuRB* selectable marker gene cassette of the plasmid pWTT2132 (DNAP) [see International Patent Application No. PCT/AU03/01111]. Construction of the transformation vector pCGP1860 is described in International Patent Application No. PCT/AU03/01111.

### The transformation vector pCGP2119 (CaMV 35S: salvia F3'5'H: ocs 3')

The transformation vector pCGP2119 contains a chimeric *CaMV 35S: salvia F3'5*'*H: ocs 3'* gene cassette in a tandem orientation with respect to the *35S 5': SuRB* selectable marker gene cassette of the plasmid pCGP1988 (see International Patent Application No. PCT/AU03/01111). Construction of pCGP2119 is described in International Patent Application No. PCT/AU03/01 111.

### The transformation vector pCGP2205 (carnANS 5': BPF3'5'H#18: carnANS 3')

The transformation vector pCGP2205 (Figure 2) contains a chimeric *carnANS 5': BPF3'5'H#18: carnANS* 3' gene cassette in a tandem orientation with respect to the *35S 5*': *SuRB* selectable marker gene cassette of the plasmid pCGP1988 (see International Patent Application No. PCT/AU03/01111).

### Isolation of carnation ANS genomic clone (pCGP787)

A genomic DNA library was constructed from *Dianthus caryophyllus* cv. Laguna DNA in EMBL 3 lambda vector (STRATAGENE) using a *Sau*3A partial digest. The digested genomic DNA was size fractionated on a glycerol density gradient and fractions containing DNA fragments ranging from 15 to 20kb were pooled and ligated with *Bam*HI ends of EMBL3 λ vector. The ligation mixture was packaged using Gigapack XL Gold (PROMEGA). The total size of the library was 1 x 10⁶ recombinants.

A total of 300, 000 pfu of the Laguna genomic library were screened in duplicate with ³²P-labeled fragments of carnation *ANS* cDNA clone from pCGP786 (see International PCT application PCT/AU96/00296). Hybridization was carried using high stringency conditions (42°C, 50% formamide, 0.1%PVP, 0.1%BSA, 0.1%ficoll, 1%SDS, 100*µ*g/mL denatured herring sperm DNA. Washes were 2 x SSC/1%SDS and 0.2 x SSC/1%SDS at 65°C. A genomic clone (4.3) was subsequently purified and further characterised. An 8 kb *Hin*dIII fragment of the genomic clone was subcloned into pBluescript KS for further analysis. The resulting plasmid was designated pCGP787.

### Construction of the intermediate plasmid pCGP1274 (carnANS 5')

A ~2.5 kb fragment containing a promoter region from the carnation *ANS* gene was released from pCGP787 (described above) upon digestion with the restriction endonucleases *Eco*RI and *Bam*HI*.* The fragment was purified and ligated with *Eco*RI*lBam*HI ends of pBluescript KS to give the plasmid pCGP793. This 2.5kb fragment also contained 200bp of 5' coding sequence of the *ANS* gene. The 3' end of the promoter region was then amplified by PCR using primers that introduced an *Xba*I site 5' upstream from the ATG codon. The amplified 700 bp PCR fragment was then digested with *Nde*I*lXba*I and ligated with *Nde*I*lXba*I ends of pCGP793 to produce a 2.3kb fragment bearing the *ANS* promoter without the 200bp of coding sequence. This new plasmid was designated as pCGP1274.

### Construction of the intermediate plasmid pCGP1275 (carnANS 5': carnANS 3')

The plasmid pCGP795 (described above) was digested with the restriction endonucleases *Ecl136*II/*Xba*I to release a 0.7 kb *ANS* terminator bearing fragment. The fragment was purified and ligated with *Apa*I (blunted)/*Xba*I ends of pCGP1274 to produce the plasmid pCGP1275 (containing promoter and terminator fragments of the carnation *ANS* gene with multicloning sites interrupting the 2 fragments for ease of cloning) [*carnANS 5*': *carnANS 3'*]*.*

### Construction of the intermediate plasmid pCGP2150 (carnANS 5': BPF3'5'H#18: carnANS 3')

The plasmid pCGP1959 (containing *BPF3'5'H#18* cDNA clone) [described in International Patent Application No. PCT/AU03/01111] was digested with the restriction endonucleases *Bam*HI and *Asp*718. The ends were repaired and the purified *BPF3'5'H#18* fragment was ligated with the repaired *Pst*I/*Xba*I ends of pCGP1275 (containing *carnANS 5*': *carnANS 3'*) to produce the plasmid pCGP2150 (containing the *carnANS 5': BPF3'5'H#18: carnANS 3'* expression cassette).

### Construction of the transformation vector pCGP2205

The chimeric *carnANS 5*': *BPF3'5'H#18: carnANS 3'* gene was released from pCGP2150 (described above) upon digestion with the restriction endonuclease *Cla*I*.* The *Cla*I ends of the ~4.8kb fragment were repaired and the fragment was isolated, purified and ligated with repaired *Asp*718 I ends of the plasmid pCGP1988 (see International Patent Application No. PCT/AU03/01111). Correct insertion of *carnANS 5': BPF3'5'H#18: carnANS 3'* gene in a tandem orientation with respect to the *35S 5*': *SuRB* selectable marker gene cassette was established by restriction endonuclease analysis of plasmid DNA isolated from tetracycline-resistant transformants. The transformation vector was designated as pCGP2205 (Figure 2).

### The transformation vector pCGP2217 (Rose CHS: BPF3'5'H#18: nos 3')

The transformation vector pCGP2217 contains the *RoseCHS: Viola F3 '5'H#18: nos 3'* expression cassette in a tandem orientation with respect to the *35S 5': SuRB* selectable marker cassette of the Ti plasmid pCGP1988 (see International Patent Application No. PCT/AU03/01 111).

### Isolation of Rose CHS promoter

Isolation of a rose *CHS* genomic clone and preparation of the plasmids pCGP1116 containing a 2.7-3.0 kb promoter fragment of the *CHS* gene from rose and pCGP197 containing *RoseCHS 5*': *GUS : nos 3'* expression cassette in pUC18 backbone is described in International Patent Application No. PCT/AU03/01111.

### Construction of intermediate plasmid pCGP197 (RoseCHS 5': GUS : nos 3' in pUC18 backbone)

An ~3.0 kb fragment bearing the rose chalcone synthase (*CHS*) promoter (*RoseCHS 5'*) was released from the plasmid pCGP1116 upon digestion with the restriction endonucleases *Hind*III and *Asp*718*.* The fragment was purified and ligated with a *Hin*dII/*Asp*718 fragment from pJB1 ((Bodeau, Molecular and genetic regulation of Bronze-2 and other maize anthocyanin genes. Dissertation, Stanford University, USA, 1994) containing the vector backbone, β-glucoronidase (*GUS*) and *nos 3'* fragments. Correct insertion of the Rose *CHS* promoter fragment upstream of the *GUS* coding sequence was established by restriction endonuclease analysis of plasmid DNA isolated from ampicillin-resistant transformants. The resulting plasmid was designated as pCGP197.

### Construction of intermediate plasmid pCGP2201 (Rose CHS 5': nos 3' in pBluescript backbone)

The plasmid pCGP197 (containing *RoseCHS 5': GUS : nos 3'* in pUC18 backbone) was firstly linearized upon digestion with the restriction endonuclease *Hind*III*.* The overhanging *Hin*dIII ends were repaired and the Rose *CHS* promoter fragment was then released upon digestion with the restriction endonuclease *Sal*I*.* The ~2.8kb fragment was purified and ligated with *Xho*I (repaired ends)/Sa*l*I ends of the plasmid pCGP1986 (containing the *nos* terminator fragment in a pBluescript backbone). Correct insertion of the Rose *CHS* promoter fragment in front of the *nos* terminator fragment was established by restriction endonuclease analysis of plasmid DNA isolated from ampicillin-resistant transformants. The resulting plasmid was designated as pCGP2201.

### Construction of intermediate plasmid pCGP2203 (Rose CHS 5': BPF3'5'H#18: nos 3' in pBluescript backbone)

The *BPF3'5'H#18* cDNA clone was released from the plasmid pCGP1959 (containing *BPF3'5'H#18* in a pBluescript backbone) [described in International Patent Application No. PCT/AU03/01111] upon digestion with the restriction endonucleases *Bam*HI and *Asp*718. After repairing the overhanging ends, a 1.6kb fragment containing the *BPF3'5'H#18* was purified and ligated with the *Sal*I (repaired ends) of pCGP2201 (described above). Correct insertion of the *BPF3*'*5*'*H*#*18* cDNA clone between the *Rose CHS 5*' and *nos 3'* fragments was established by restriction endonuclease analysis of plasmid DNA isolated from ampicillin-resistant transformants. The resulting plasmid was designated as pCGP2203.

### Construction of transformation vector pCGP2217 (Rose CHS 5': BPF3'5'H#18: nos 3' in pCGP1988 backbone)

The plasmid pCGP2203 was firstly linearized upon digestion with the restriction endonuclease *Spe*I*.* The overhanging ends were repaired and the fragment containing the *Rose CHS 5*': *BPF3'5'H#18: nos 3'* expression cassette was released upon digestion with the restriction endonuclease *Bgl*II*.* The ~5kb fragment was purified and ligated with the *Sal*I (repaired ends)/BamHI ends of the plasmid pCGP1988 (see International Patent Application No. PCT/AU03/01111). Correct insertion of *Rose CHS 5': BPF3'5'H#18: nos 3'* gene in a tandem orientation with respect to the *35S 5*': *SuRB* selectable marker gene cassette was established by restriction endonuclease analysis of plasmid DNA isolated from tetracycline-resistant transformants. The transformation vector was designated as pCGP2217 (Figure 3).

### The transformation vectoz· pCGP2788 (CaMV 35S: BPF3'5'H #40: 35S)

The transformation vector pCGP2788 contains the *CaMV 35S: BPF3'5'H#40: 35S 3'* expression cassette in a tandem orientation with respect to the *35S 5': SuRB* selectable marker cassette of the Ti plasmid pCGP1988 (see International Patent Application No. PCT/AU03/01111). Construction of pCGP2788 is described in International Patent Application No. PCT/AU03/00079.

### The transformation vector pCGP3141 (Cineraria gF3'5'H)

The transformation vector pCGP3141 contains a *Cineraria F3*'*5*'*H* gene (see Japanese Patent Application Number 2008-276029) in a tandem orientation with respect to the *35S 5*': *SuRB* selectable marker cassette of the Ti plasmid pCGP1988 (see International Patent Application No. PCT/AU03/01111).

### Isolation of the cineraria F3'5'H genomic clone

A genomic clone of *Cineraria F3'5'H* was isolated as described in Japanese Patent Application Number 2008-276029. An ~4.6kb fragment harboring the *Cineraria gF3'5'H* clone (SEQ ID NO: 12) was released from the plasmid gCi01 [*Cineraria F3'5'H* gene in pBluestar vector backbone (NOVAGEN)] upon digestion with the restriction endonucleases *Pv*uII and *Eco*RV. The fragment was purified and ligated with the *Sma*I ends of the plasmid pCGP1988. Correct insertion of the *Cineraria gF3'5'H* gene in a convergent orientation relative to the *35S 5': SuRB* selectable marker cassette was confirmed by restriction endonuclease analysis of plasmid DNA isolated from tetracycline-resistant transformants. The plasmid was designated as pCGP3141 (Figure 5).

### The transformation vector pCGP3149 (35S 5': BPF3'5'H#40: SuRB 3')

The transformation vector pCGP3149 contains a *35S 5*': *BPF3'5'H#40: SuRB 3'* expression cassette in a tandem orientation with respect to the *35S 5': SuRB* selectable marker cassette of the Ti plasmid pCGP1988 (see International Patent Application No. PCT/AU03/11111).

Fragments used in the construction of the transformation vector pCGP3149 include, a *Pst*I*lStu*I fragment containing the *SuRB 3'* terminator sequence from pCGP1988 (see International Patent Application No. PCT/AU03/01111), a *Pst*I*lNco*I fragment bearing the *35S 5'* promoter sequence along with the backbone vector released from the plasmid pCGP1273 (described in International Patent Application No. PCT/AU03/01111) and a BPF3'5'H#40 fragment that originated from the plasmid pCGP1961 (see International Patent Application No. PCT/AU03/01111). The resulting plasmid pCGP3148 contained *35S 5*': *BPF3'5'H#40: SuRB* 3' expression cassette in a pBluescript backbone vector.

The *35S 5': BPF3'5'H #40: SuRB 3'* expression cassette was released from the plasmid pCGP3148 upon digestion with the restriction endonuclease *Bam*HI*.* The fragment was purified and ligated with th *Bam*HI ends of the plasmid pCGP1988. Correct insertion of the *35S 5': BPF3'5'H#40: SuRB 3'* chimeric gene in a tandem orientation with respect to the *35S 5': SuRB* selectable marker cassette was confirmed by restriction endonuclease analysis of plasmid DNA isolated from tetracycline-resistant transformants. The plasmid was designated as pCGP3149.

### The transformation vector pCGP3408 (BPF3'5'H#40 5': BPF3'5'H#40: nos 3')

The transformation vector pCGP3409 contains a *BPF3'5'H#40 5*'*: BPF3'5'H#40: nos* expression cassette in a tandem orientation with respect to the *35S 5': SuRB* selectable marker cassette of the Ti plasmid pCGP1988 (see International Patent Application No. PCT/AU03/01111).

### Isolation of the promoter fragment from the BPF3'5'H#40 gene.

Genomic DNA was isolated from the *Viola* cv. black pansy leaves using DNA easy plant kit (QIAGEN) following procedures recommended by the manufacturer. Three µg of genomic DNA was digested with the restriction endonuclease *Hind*III*.* The digested DNA was ligated with *Hin*dIII ends of TaKaRa LA PCR (Trade Mark) [contained in the *in vitro* Cloning Kit TAKARA] using Ligation High (TAKARA) for 40 min at 16°C using the manufacturer's protocol.

The promoter fragment of *BPF3'5H#40* gene was amplified using nested PCR. The 1^{st} PCR was carried out as follows: 4 µL of the ligated genomic DNA (gDNA) [described above] was diluted with 10 µL of water and denatured at 94 °C for 10 min and then transferred to ice. 5 pmol of the following primers:
Primer C1 (5'-GTACATATTGTCGTTAGAACGCGTAATACGACTCA-3', SEQ ID NO:23) [designed to the cassette and contained in the kit]
Primer BP40-i5 (5'-AGGTGCATGATCGGACCATACTTC-3', SEQ ID NO:24) designed to the coding region of BPF3'5'H#40 (reverse orientation)
were added to 14 µL ligated gDNA. PCR was carried out in 25 µL following the protocol recommended by the manufacturer (30 cycles of incubation at 98°C for 20 sec, then 68 °C for 15 min.).

The reaction products of the 1^{st} PCR were then diluted 10-fold with water. 0.5 µL of the diluted 1^{st} PCR products, and 5 pmol of the following primers:
Primer C2 (5'-CGTTAGAACGCGTAATACGACTCACTATAGGGAGA-3', SEQ ID NO:25) [designed to the cassette and contained in the kit]
Primer BP-i7, (5'-GACCATACTTCTTAGCGAGTTTGGC-3', SEQ ID NO:26) [designed to the coding region of BPF3'5'H#40 (reverse orientation)]
were subjected to PCR in a total reaction volume of 25µL. The PCR conditions included a denaturation step at 98 °C for 5 min, followed by 30 cycles of incubation at 98°C for 20 sec, then 68°C for 15 min.

The products of the 2^{nd} PCR were cloned into pCR2.1 (INVITROGEN). The inserts were sequenced to confirm that the correct *F3'5'H* inserts were obtained.

The promoter region of the *BPF3'5'H#40* gene along with 200 bp of coding sequence was amplified by PCR using 200 ng of the pansy genomic DNA as template along with 50pmol of the following primers:
Primer BP40-i7 (described above) and
Primer BP40 pro-F (5'-ACTCAAACAAGCATCTCGCCATAGG-3', SEQ ID NO:27)

### [designed to the promoter region of BPF3'5'H#40 gene]

in a total reaction volume of 25µL using Ex-Taq DNA Polymerase (TOYOBO). Conditions used were an initial denaturation step at 98°C for 5 min followed by 30 cycles of incubation at 98°C for 20 sec and 68°C for 15 min.

The PCR products were cloned into the cloning vector pCR2.1 (INVITROGEN). The inserts were sequenced and one plasmid containing 200bp of coding region along with ~2.1kb of 5' non coding region was designated as pSFL614.

### Construction of the intermediate plasmid pSFL620 (BPF3'5'H#40 5':BPF3'5'H#40: nos 3')

About 1.1 kb of the 5'-upstream region of the genomic sequence in pSFL614 (described above) was used to control expression of *BPF3 '5'H#40* coding sequence as described below. A *Bam*HI site in the region was converted to *Nhe*I for convenience of construction.

A 470bp DNA fragment bearing the 5' region of the promoter of the *BPF3'5'H#40* gene was amplified using 1 ng of the pSFL614 plasmid as template along with 50pmol of the following primers:
Primer BP40 pro-HindIII-F (5'AAG CTT GTG ATC GAC ATC TCT CTC C-3', SEQ ID NO:28) and Primer BP40 pro-NheI-R (5'- CGA GGC TAG CTA AAC ACT TAT -3', SEQ ID NO:29)

in a total reaction volume of 25*µ*L using Ex-Taq DNA Polymerase (TOYOBO).

Conditions used were an initial denaturation step at 98°C for 5 min followed by 25 cycles of incubation at 98°C for 20 sec and 68°C for 15 min. The amplified DNA was cloned into pCR2.1 (INVITROGEN). The sequence was confirmed to be free from PCR error. The 470 bp DNA fragment was recovered by digesting with the restriction endonucleases *Hin*dIII and *Nhe*I (designated as Fragment A).

A 630 bp DNA fragment bearing the 3' region of the promoter of the *BPF3'5'H#40* gene was then amplified by PCR using 1 ng of the plasmid pSFL614 (described above) as template along with 50pmol of the following primers:
Primer BP40 pro-NheI-F (5'-TTT AGC TAG CCT CGA AGT TG-3', SEQ ID NO:30) and
Primer BP40 pro-BamHI-R (5'- GGA TCC CTA TGT TGA GAA AAA GGG ACT -3', SEQ ID NO:31)
in a total reaction volume of 25 *µ*L using Ex-Taq DNA Polymerase (TOYOBO).

Conditions used were an initial denaturation step at 98°C for 5 min followed by 25 cycles of incubation at 98°C for 20 sec and 68°C for 15 min. The amplified DNA was then cloned into pCR2.1 (INVITROGEN). The sequence was confirmed to be free from PCR error. The 630 bp DNA fragment was released upon digestion with the restriction endonucleases *Nhe*I and *Bam*HI (designated as Fragment B).

The plasmid pSPB567 (described in Patent Publication Number WO2005/017147 and containing *enhanced CaMV35S: BPF3'5'H#40: nos 3'* gene cassette in a pUC backbone) was digested with the restriction endonucleases *Hin*dIII (partial digestion) and *Bam*HI with the aim of removing the *CaMV35S* promoter bearing fragment. The 430bp *Hin*dIII/*Nh*eI Fragment A (described above and bearing the 5' region of the promoter of the BPF3'5'H#40 gene) and *Nhe*I/*Bam*HI Fragment B (described above and bearing the 3' region of the promoter of the *BPF3'5'H#40* gene) were then ligated with *Hin*dIII*lBam*HI fragment bearing the *BPF3'5'H#40: nos* 3' cassette on a pUC backbone from pSPB567 to yield pSFL620 (*BPF3'5'H#40 5': BPF3'5'H#40: nos 3'* in a pUC backbone). The nucleotide sequecne of the *BPF3'5'H#40 5'* promoter fragment is shown in SEQ ID No: 18.

### Construction of the intermediate plasmid pSFL622 (BPF3'5'H#40 5':BPF3'5'H#40: nos 3')

The 3.1 kb *BPF3'5'H#40 5': BPF3'5'H#40: nos 3'* expression cassette was excised from the intermediate plasmid pSFL620 (described above) using the restriction endonuclease *Pac*I, and ligated with the *Pac*I ends of pBINPLUS (van Engelen et al, Transgenic Research 4, 288-290, 1995). Correct insertion of the *BPF3'5'H#40 5*': *BPF3'5'H#40: nos 3'* chimeric gene in a tandem orientation with respect to the *nos 5': nptII: nos 3'* selectable marker gene cassette was established by restriction endonuclease analysis of plasmid DNA isolated from kanamycin-resistant transformants. The resulting plasmid was designated pSFL622.

### Construction of the transformation vector pCGP3408 (BPF3'5'H#40 5':BPF3'5'H#40: nos 3')

The *BPF3'5'H#40 5': BPF3'5'H#40: nos 3'* expression cassette was released from pSFL622 (described above) upon digestion with the restriction endonuclease *Pac*I*.* The 3.1kb fragment was purified and ligated with *Pac*I ends of the plasmid pCGP1988 (see International Patent Application No. PCT/AU03/01111). Correct insertion of the *BPF3'5'H#40 5': BPF3'5'H#40: nos 3'* chimeric gene in a tandem orientation with respect to the *35S 5*': *SuRB* selectable marker gene cassette was established by restriction endonuclease analysis of plasmid DNA isolated from tetracycline-resistant transformants. The resulting transformation vector was designated pCGP3408.

### The transformation vector pCGP3409 (R. rugosa DFR 5': BPF3'5'H#40: nos 3')

The transformation vector pCGP3409 contains a *R. rugosa DFR 5*'*: BPF3'5'H#40: nos* expression cassette in a tandem orientation with respect to the *35S 5*': *SuRB* selectable marker cassette of the Ti plasmid pCGP1988 (see International Patent Application No. PCT/AU03/01111).

### Isolation of a DFR genomic clone from Rosa rugosa

A *Rosa rugosa DFR* genomic clone was isolated from a *R. rugosa* genomic library prepared using λBlueSTAR™ *Xho* I Half-Site Arms Kit (Novagen, http://www.merckbiosciences.com/product/69242).

Genomic DNA was isolated from young leaves of *R. rugosa* using Nucleon Phytopure [Registered] (TEPNEL Life Sciences). About 100 µg of genomic DNA was partially digested with the restriction endonuclease *Sau*3AI. The digested ends were partially filled-in with DNA polymerase I Klenow fragment (TOYOBO) in the presence of dGTP and dATP. The genomic DNA (gDNA) was then subjected to size fractionation by sucrose density gradient.

The fractions containing gDNA fragments of around 13 kb were combined and subjected to ethanol precipitation. About 180 ng of the gDNA was ligated with 1 µL of λBlueSTAR™ *Xho* I Half-Site Arms Kit at 4°C overnight and then packaged. The genomic library obtained was subjected to screening with the rose *DFR* cDNA (Tanaka et al, Plant and Cell Physiology, 36, 1023-1031, 1995). The DNA labeling and detection were carried out with DIG (Roche Applied Science) following the protocol provided by the manufacturer.

Positive plaques were purified and subjected to *in vivo* excision following the procedure for λBlueSTAR™ (Novagen). One of the clones (pSFL710) containing an insert of around 10kb was further characterised. Sequence determination of the DNA fragment in pSFL710 revealed that the fragment contained a sequence homologous to *R. hybrida DFR* cDNA Tanaka *et al,* 1995, *supra*).

In order to isolate the 5'-non coding region of *R. rugosa DFR* gene, an *Eco*RI recognition sequence was mutated and *Hin*dIII and *Bam*HI recognition sequences were added using PCR. One µL of each of the following primers (both at 25µM):
DFRproHindIIIF (5'-TAATAAGCTTACAGTGTAATTATC-3', SEQ ID NO:32) and
DFRproNheIR (5'-TTATGCTAGCGTGTCAAGACCAC-3', SEQ ID NO:33)
were added to a mix containing the template pSFL710 (described above) and ExTaq DNA Polymerase (TOYOBO) [final volume 50µL]. PCR conditions included a denaturation step at 94°C for 5 min followed by 30 cycles of incubation at 94°C for 30 sec, 50°C for 30 sec and 72°C for 30 sec, followed by a final incubation at 72°C for 7 minutes. A 350bp DNA fragment was amplified and was designated as Fragment C.

A second PCR was carried out using 1 µL each of the primers (both at 25µM):
DFRproNheIF (5'-ACACGCTAGCATAAGTCTGTTG -3', SEQ ID NO:34) and
DFRproBamHI-R (5'-GCTTGGGGATCCATCTTAGG -3', SEQ ID NO:35)
along with the template pSFL710 (described above) and ExTaq DNA Polymerase (TOYOBO). PCR conditions included a denaturation step at 94°C for 5 min, followed by 30 cycles of incubation at 94°C for 30 sec, 50°C for 30 sec, 72°C for 30 see with a final incubation at 72°C for 7 minutes. A 600bp DNA fragment was amplified and designated Fragment D.

Fragment C (described above) was digested with the restriction endonuclease *Hin*dIII and *Nhe*I and Fragment D (described above) was digested with the restriction endonucleases *Nhe*I and *Bam*HI. The plasmid pSPB567 (containing *enhanced CaMV35S: BPF3'5'H#40: nos 3'* gene cassette in a pUC backbone (described in Patent Publication Number WO2005/017147) was digested with *Hind*III (partial) and *Bam*HI with the aim of removing the *CaMV35S* promoter bearing fragment. Therefore *Hin*dIII/*Nhe*I Fragment C (bearing the 5' portion of the *R. rugosa DFR* promoter sequence) along with *Nhe*I/*Bam*HI Fragment D (bearing the 3' portion of the *R. rugosa DFR* promoter sequence) and *Hin*dIII/*Bam*HI pSPB567 (bearing the *BPF3'5'H#40*: *nos 3'* cassette in a pUC backbone) were ligated together to give the plasmid pSFL721 (*R. rugosa DFR 5': BPF3'5'H#40: not* 3' in a pUC based vector). (The sequence of the *R. rugosa DFR* promoter with an *EcoRI* recognition site mutated to a *Nhe*I site and *Hin*dIII and *Bam*HI recognition sites added is shown in SED ID NO:16).

### Construction of the transformation vector pCGP3409

A 3.11 kb fragment bearing *R. rugosa DFR 5': BPF3'5'H#40: nos* 3' expression cassette was excised from the intermediate plasmid pSFL721 (described above) using the restriction endonuclease *Pac*I, and ligated with *Pac*I ends of the plasmid pCGP1988 (see International Patent Application No. PCT/AU03/01111). Correct insertion of the *R. rugosa DFR 5': BPF3'5'H#40: not* 3' chimeric gene in a tandem orientation with respect to the *35S 5'*: *SuRB* selectable marker gene cassette was established by restriction endonuclease analysis of plasmid DNA isolated from tetracycline-resistant transformants. The resulting transformation vector was designated pCGP3409.

### The transformation vector pCGP3410 (R. rugosa F3H 5': BPF3'5'H#40: nos 3')

The *transformation* vector pCGP3410 contains a *R. rugosa F3H 5': BPF3'5'H#40*: *nos 3'* expression cassette in a tandem orientation with respect to the *35S 5': SuRB* selectable marker cassette of the Ti plasmid pCGP1988 (see International Patent Application No. PCT/AU03/01111).

### Isolation of a F3H genomic clone from Rosa rugosa

The genomic library of *Rosa rugosa* (described above) was screened with DIG-labeled fragments of the torenia flavanone 3-hydroxylase (F3H) cDNA (NCBI No. AB211958). A selection of hybridising plaques were purified and the plasmids were excised (using methods recommended by the manufacturer). One of the clones, #5, was selected for further study. The plasmid was digested with the restriction endonuclease *Spe*I and one of the resultant fragment of around 2.6 kb in size was isolated and purified. The fragment was subcloned into *Spe*I ends of pBluescript SKII- (STRATAGENE) and the resultant plasmid was designated as. Sequence analysis of the fragment contained in pSPB804 revealed that the plasmid contained F3H-like sequence.

In order to amplify a 5' non coding region of *Rosa rugosa F3H* gene, PCR was carried out using the following primers:
RrF3H-F (5'- AAGCTTCTAGTTAGACAAAAAGCTA-3', SEQ ID NO:36) and
RrF3H (5'- GGATCCTCTCTTGATATTTCCGTTC-3', SEQ ID NO:37),
along with 1ng of pSPB804 as template and the Ex-Taq DNA Polymerase (TOYOBA) in a final reaction volume of 50 µL. PCR conditions included an incubation at 94°C for 5 min, followed by 30 cycles of incubation at 94 °C for 30 sec, then 50°C for 30 sec and 72 °C for 30 sec with a final incubation at 72°C for 7 min. The amplified fragment was subcloned into pCR-TOPO vector (INVITROGEN) to yield the plasmid pSPB811.

The fragment bearing the *R. rugosa F3H* promoter region (SEQ ID NO:17) was released from pSPB811 upon digestion with the restriction endonucleases *Hin*dIII and *Bam*HI. The plasmid pSPB567 (described in Patent Publication Number WO2005/017147 and containing *enhanced CaMV35S: BPF3'5'H#40: nos 3'* gene cassette in a pUC backbone) was digested with the restriction endonucleases *Hin*dIII (partial digestion) and *Bam*HI with the aim of removing the *CaMV35S* promoter bearing fragment. The 1.2kb *Hin*dIII/*Bam*HI fragment bearing the *R. rugosa F3H* promoter region from pSPB811 was then ligated with *Hin*dIII/*Bam*HI fragment bearing the *BPF3'5'H#40*: *nos 3'* cassette on a pUC backbone from pSPB567 to yield pSFL814 (*R. rugosa F3H 5*'*: BPF3'5'H#40*: *nos 3'* in a pUC backbone).

### Construction of the transformation vector pCGP3410

An ~3.2 kb fragment bearing the *R. rugosa F3H 5'*: *BPF3'5'H#40: nos 3'* expression cassette was excised from the intermediate plasmid pSFL814 (described above) using the restriction endonucleases *Pac*I and *Asc*I, and ligated with *Pac*I/*Asc*I ends of the plasmid pCGP1988 (see International Patent Application No. PCT/AU03/01111). Correct insertion of the *R. rugosa F3H 5'*: *BPF3'5'H#40: nos 3'* chimeric gene in a tandem orientation with respect to the *35S 5'*: *SuRB* selectable marker gene cassette was established by restriction endonuclease analysis of plasmid DNA isolated from tetracycline-resistant transformants. The resulting transformation vector was designated pCGP3410.

### The transformation vector pCGP3424 (RoseCHS 5': BPF3'5'H#18: nos 3': 35S 5': ds chrysF3 'H: ocs 3')

The transformation vector pCGP3424 contains the *RoseCHS 5'*: ***BPF3'5'H#18:** nos* 3' expression cassette in a tandem orientation with respectto *35S 5': SuRB* selectable marker cassette of the transformation vector pCGP2217 (described above) along with a *35S 5': ds chysF3'H: ocs* 3' cassette (from pCGP3134, described below) directed at down-regulating expression of the endogenous chrysanthemum *F3'H.*

### Construction of intermediate plasmid pCGP1472 (petunia DFR-A genomic clone)

A genomic library was made from *Petunia hybrida* cv. Old Glory Blue DNA in the vector λ2001 (Holton, 1992 *supra).* Approximately 200, 000 pfu were plated out on NZY plates, lifts were taken onto NEN filters and the filters were hybridized with 400, 000 cpm/mL of ³²P-labeled petunia *DFR-A* cDNA fragment (described in Brugliera et al, The Plant Journal 5(1): 81-92, 1994). Hybridising clones were purified, DNA was isolated from each and mapped by restriction endonuclease digestion. A 13 kb *Sac*I fragment of one of these clones was isolated and ligated with *Sac*I ends of pBluescriptII to create the plasmid pCGP1472. Finer mapping indicated that an ~5.3 kb *Bgl*II fragment contained the entire petunia *DFR-A* gene (Beld et al, Plant Mol. Biol. 13: 491-502, 1989).

### Construction of intermediate plasmid pCGP3127 (CaMV 35S: petunia DFR-A intron 1: ocs 3').

A fragment bearing 180 bp of the petunia *DFR-A* intron 1 was amplified by PCR using the plasmid pCGP1472 (described above) as template and the following primers:
DFRintF 5' GCAT CCATGG AGATCT TCG TGA TCC TGG TAT GTT TG 3' (SEQ ID NO:38)
DFRintR 5' GCAT TCTAGA CTT CTT GTT CTC TAC AAA ATC 3' (SEQ ID NO:39)

The forward primer (DFRint F) was designed to incorporate the restriction endonuclease recognition sites *Nco*I and *Bgl*II at the 5'-end. The reverse primer (DFRint R) was designed to incorporate an *Xba*I restriction endonuclease recognition site at the 3'-end of the 180 bp product that was amplified. The resulting 180 bp PCR product was then digested with the restriction endonucleases *Nco*I and *Xba*I and ligated with *Nco*I/*Xba*I ends of the plasmid pCGP1634 (described in International Patent Application No. PCT/AU03/01111). Correct insertion of the petunia *DFR-A* intron 1 fragment between the *35S 5'* and *ocs* 3' fragments of pCGP1634 was confirmed by restriction endonuclease analysis of plasmid DNA isolated from ampicillin-resistant transformants. The resulting plasmid was designated pCGP3127.

### Construction of intermediate plasmid pCGP3369 (partial Chrysanthemum F3'H clone).

A 1.2 kb fragment containing a partial chrysanthemum *F3'H* sequence (The sequence of a full-length chrysanthemum ***F3'H*** cDNA clone is shown in SEQ ID NO:44, see International PCT application PCT/AU97/00124) was amplified by PCR using a chrysanthemum petal cDNA library (Klondike) as template. A Klondike petal cDNA library was prepared from RNA isolated from floral stages 1 to 3 according to methods described in International PCT application PCT/AU97/00124.
The following primers were used in the PCR:
Chrys Red F 5' CCTCATCCTCGGGTCAGTAC 3' (SEQ ID NO:40)
Chrys Red R 5' GCAACTGGACCATTCTCAAGC 3' (SEQ ID NO:41)

The resulting 1.2kb PCR product was ligated with the T/A-vector pCR2.1 (INVITROGEN). Correct insertion of the PCR product was confirmed by restriction endonuclease analysis of plasmid DNA isolated from ampicillin-resistant transformants and by sequence analysis. The resulting plasmid was designated as pCGP3369.

### Construction of intermediate plasmid pCGP3133 (chrysanthemum F3'H).

The plasmid pCGP3133 contains a partial chrysanthemum ***F3'H*** clone generated by PCR to incorporate the *Xba*I restriction endonuclease recognition site at either ends. An ~1.1 kb fragment of chrysanthemum ***F3'H*** cDNA clone was amplified using the following primers:
3369Xba.F 5'GCATCTCTAGAACACCATGGCCTATAGTCG3' (SEQ ID NO:42)
3369Xba.R 5' GCATCTCTAGATTCAAGAGGATCCGCCCAC 3' (SEQ ED NO:43)
and the plasmid pCGP3369 (described above) as template. The resulting ~1.1 kb PCR product was ligated with the T/A-vector pCR2.1 (INVITROGEN). Correct insertion of the PCR fragment into the pCR2.1 vector was confirmed by restriction endonuclease and sequence analysis of ampicillin-resistant transformants. The resulting plasmid was designated pCGP3133.

### Construction of intermediate plasmid pCGP3129 (35S 5': chrysanthemum F3'H sense: Petunia DFR-A intron 1: ocs 3).

An ~1 kb fragment containing the partial chrysanthemum ***F3'H*** cDNA was excised from the intermediate plasmid pCGP3369 (described above) by digestion with the restriction endonucleases *Nco*I and *Bam*HI. The resulting fragment was purified and ligated with *Nco*I/*Bgl*II ends of the plasmid pCGP3127 (described above). Correct insertion of the chrysanthemum ***F3'H*** fragment in a sense direction between the *35S 5'* promoter fragment and the petunia genomic *DFR-A* intron 1 fragment was confirmed by restriction endonuclease analysis of plasmid DNA isolated from ampicillin-resistant transformants. Further confirmation was obtained by sequence analysis of the correctly mapped transformant. The resulting intermediate plasmid was designated pCGP3129.

### Construction of the intermediate plasmid pCGP3134 (35S 5': ds chrysF3'H: ocs 3').

The intermediate plasmid pCGP3134 contains *35S 5': ds chrysF3'H: ocs 3'* cassette in a pCR2.1 (INVITROGEN) backbone. The chrysanthemum ***F3'H*** partial cDNA fragment was released from the plasmid pCGP3133 (described above) upon digestion with the restriction endonuclease, *Xba*I. The resulting ~1.1kb fragment was purified and ligated with *XbaI* ends of pCGP3129 (described above). Correct insertion of the chrysanthemum *F3 'H* fragment in an antisense direction between the petunia *DFR-A* intron 1 and the *ocs 3'* terminator fragment was confirmed by restriction endonuclease analysis of plasmid DNA isolated from ampicillin resistant transformants. The resulting plasmid was designated pCGP3134.

### Construction of the transformation vector pCGP3424 (Rose CHS 5': BPF3'5'H#18: nos 3': 35S 5': ds chrysF3'H.- ocs 3')

The expression cassette *35S 5': ds **chrysF3 'H:** ocs* 3' was released from the plasmid pCGP3134 (described above) upon digestion with the restriction endonucleases *Sma*I and *Pvu*II. The resulting 3.5kb fragment was purified and ligated with *Pme*I SAP treated ends of the transformation vector pCGP2217 (described above). Correct insertion of the *35S 5': ds **chrysF3 'H:** ocs 3'* cassette in a tandem orientation with respect to the *RoseCHS 5'*: *BPF3'5'H#18: nos 3'* and *35S 5'*: *SuRB* selectable marker cassette was confirmed by restriction endonuclease analysis of plasmid DNA isolated from tetracycline-resistant transformants. The resulting transformation vector was designated as pCGP3424 (Figure 4).

### The transformation vector pCGP3429 (RoseCHS 5': BPF3'5'H#18: nos 3': RoseCHS 5': ds chrysF3'H*: nos 3')

The transformation vector pCGP3429 contains a *RoseCHS 5'*: *BPF3'5'H#18: nos 3'* expression cassette in a tandem orientation with respect to *35S* 5': *SuRB* selectable marker cassette of along with a *RoseCHS 5': ds chrysF3'H*: ocs 3'* cassette directed at down-regulating expression of the endogenous chrysanthemum *F3'H.*

### Construction of the intermediate plasmid pSPB3349 (RoseCHS 5' in pBINPlus)

A fragment bearing the *RoseCHS 5'* promoter region released from pCGP2203 (described above) upon digestion with the restriction endonucleases *Hin*dIII and *Sma*I digestion. The ~2.7kb fragment was purified and ligated with *Hind*III/*Sma*I ends of pBINPLus (van Engelen *et al.,* 1995, *supra*). Correct insertion of the fragment was established by restriction endonuclease analysis of plasmid DNA isolated from kanamycin-resistant transformants. The resulting plasmid was designated pSPB3349.

### Construction of the intermediate plasmid pSPB3350 (partial antisense chrys F3 'H: nos 3')

A fragment bearing a partial region of the chrysanthemum *F3'H* cDNA clone was released from the plasmid pRm6i (International Patent Application No. PCT/AU97/00124) upon digestion with the restriction endonucleases *Sal*I and *Hind*III. The 0.83 kb fragment containing 5'-portion of the chrysanthemum ***F3'H*** cDNA was purified and ligated with *Hind*III/*Sal*I ends of the plasmid pSPB176 (pSPB176 contains an *e35S 5': GUS: nos 3'* in pBINPlus and is described in International Patent Application No. PCT/AU03/00079). Correct insertion of the fragment in an antisense direction to the *nos 3'* terminator fragment was established by restriction endonuclease analysis of plasmid DNA isolated from kanamycin-resistant transformants. The resulting plasmid was designated pSPB3350.

### Construction of the intermediate plasmid pSPB3351 (RoseCHS 5': ds chrys F3'H* nos 3')

A fragment bearing the *RoseCHS 5'* promoter region was released from the plasmid pSPB3349 (described above) upon digestion with the restriction endonucleases *Asc*I and *Kpn*I. The fragment was purified and designated Fragment A (*Asc*I/*Kpn*I *RoseCHS 5'* fragment).

The plasmid pSPB3348 (described above) was firstly linearised with the restriction endonuclease *Bam*HI. The overhanging ends were repaired and then a 1.1kb fragment bearing the 5' portion of the chrysanthemum ***F3'H*** cDNA clone was released upon digestion with the restriction endonuclease *Kpn*I. The fragment was purified and designated as Fragment B *(Bam*HI(blunted)/*Kpn*I *chrysF3'H*).

The plasmid pSPB3350 (described above) was firstly linearised with the restriction endonuclease *Hin*dIII. The overhanging ends were repaired and then the lineraised plasmid was digested with the restriction endonuclease *Asc*I. The linearised plasmid was designated as Fragment C (*Hin*dIII (blunted)/*Asc*I antisense *chrysF3'H: nos* 3' in pBINPlus). Fragments A, B and C were ligated. Correct insertion of the fragments to yield *RoseCHS 5': sense chrysF3'H: antisense chrysF3*'*H: nos 3'* was established by restriction endonuclease analysis of plasmid DNA isolated from kanamycin-resistant transformants. The resulting plasmid was designated pSPB3351 *(RoseCHS 5':ds chrysF3'H***: nos 3'* in a pBINPlus backbone).

### Construction of the intermediate plasmid pSPB3352 (RoseCHS 5': ds chrys F3'H* :nos 3'; RoseCHS 5': BPF3'5'H#18 : nos 3'; nos 5' : nptII: nos 3')

A fragment bearing the *RoseCHS 5': **BPF3'5'H#18:** nos* 3' expression cassette was released from the plasmid pSPB459 (described in International Patent Application No. WO2005-017147)(contains *RoseCHS 5'*: *BPF3'5'H#18: nos* 3' in a pUC based backbone) upon digestion with the restriction endonuclease *Pac*I. The fragment was purified and ligated with *Pac*I ends of the plasmid pSPB3351 (described above). Correct insertion of the fragment bearing the *RoseCHS 5'*: *BPF3'5'H#18: nos* 3' chimeric gene in a tandem orientation with respect to the *RoseCHS 5': ds chrysF3'H** *: nos* 3' expression cassette was established by restriction endonuclease analysis of plasmid DNA isolated from kanamycin-resistant transformants. The resulting plasmid was designated pSPB3352.

### Construction of the intermediate plasmid pCGP3426 (RoseCHS 5': ds chrys F3'H* :nos 3'; 35S 5': SuRB)

The fragment bearing the *RoseCHS 5': ds chrys F3'H* : nos 3'* chimeric gene was released from the plasmid pSPB3352 (described above) upon digestion with the restriction endonucleases *AscI* and *PacI.* The fragment was purified and ligated with *Asc*I*lPac*I ends of the Ti plasmid pCGP1988 ((see International Patent Application No. PCT/AU03/01111)). Correct insertion of the fragment bearing the *RoseCHS 5': ds chrysF3*'*H** *: nos 3'* chimeric gene in a tandem orientation with respect to the *35S 5'*: *SuRB* selectable marker cassette was established by restriction endonuclease analysis of plasmid DNA isolated from teracylcine-resistant transformants. The resulting plasmid was designated pCGP3426.

### Construction of the transformation vector pCGP3429 (RoseCHS 5': ds chrys F3'H* :nos 3'; RoseCHS 5': BPF3'5'H#18 : nos 3'; 35S 5': SuRB)

The fragment bearing the *RoseCHS 5': BPF3'5'H#18 : nos* 3' chimeric gene was released from the plasmid pSPB3352 (described above) upon digestion with the restriction endonuclease *Pac*I. The fragment was purified and ligated with *Pac*I ends of the the plasmid pCGP3426 (described above). Correct insertion of the fragment bearing the *RoseCHS 5': BPF3'5'H#18 : nos* 3' in a in a tandem orientation with respect to the *RoseCHS 5'*: *ds chrysF3'H* : nos 3'* chimeric gene and the *35S 5'*: *SuRB* selectable marker cassette was established by restriction endonuclease analysis of plasmid DNA isolated from teracylcine-resistant transformants. The resulting plasmid was designated pCGP3429 (Figure 6).

### The transformation vector pCGP3434 (RoseCHS: BPF3'5'H#40: nos)

The transformation vector pCGP3434 contains the *RoseCHS 5'*: *BPF3'5'H#40*: *nos* 3' expression cassette and the *35S 5': SuRB* selectable marker cassette of the Ti plasmid pCGP1988 (see International Patent Application No. PCT/AU03/01111).

### Construction of the intermediate plasmid pCGP3425 (RoseCHS 5': BPF3'5'H#40: nos 3')

A 1.6 kb fragment bearing the *BPF3'5'H#40* cDNA clone was excised from the plasmid pCGP1961 (see International Patent Application No. PCT/AU03/01111) using the restriction endonucleases *Bam*HI and *Asp*718. The ends of the fragment were repaired and then ligated with the repaired ends of *Sal*I of the plasmid pCGP2201 (described above). Correct insertion of the *BPF3'5'H#40* cDNA clone between the *RoseCHS 5'* and *nos 3'* fragments was confirmed by restriction endonuclease analysis of plasmid DNA isolated from ampicillin-resistant transformants. The resulting plasmid was designated as pCGP3425.

### Construction of intermediate plasmid pCGP3438 (RoseCHS 5': BPF3'5'H#40: nos 3')

A 4.9kb fragment bearing the *RoseCHS 5'*: *BPF3'5'H#40: nos 3'* expression cassette was released from the plasmid pCGP3425 (described above) upon digestion with the restriction endonucleases *Spe*I and *Bgl*II. The fragment was purified and ligated with *Xba*I/*Bam*HI ends of pUCAP/AscI cloning vector. (The plasmid pUCAP/AscI is a pUC19 based cloning vector with extra cloning sites specifically an *Asc*I recognition site at either ends of the multicloning site.) Correct insertion of the *RoseCHS 5'*: *BPF3'5'H#40*: *nos 3'* bearing fragment was confirmed by restriction endonuclease analysis of plasmid DNA isolated from ampicillin-resistant transformants. The resulting plasmid was designated as pCGP3438.

### Construction of the transformation vector pCGP3434 (RoseCHS 5': BPF3'5'H#40: nos 3')

A 4.9 kb fragment bearing the *RoseCHS 5'*: *BPF3'5'H#40: nos* 3' expression cassette was released from the plasmid pCGP3438 (described above) upon digestion with the restriction endonuclease *Asc*I. The fragment was purified and ligated with *Asc*I ends of the plasmid pCGP1988 (see International Patent Application No. PCT/AU03/01111). Correct insertion of the *RoseCHS 5': BPF3'5'H#40: nos* 3' cassette in a convergent orientation relative to the *35S 5': SuRB* selectable marker cassette was confirmed by restriction endonuclease analysis of plasmid DNA isolated from tetracycline-resistant transformants. The resulting transformation vector was designated as pCGP3434.

The expression cassettes contained on the T-DNAs from the transformation vectors (Table 4) were introduced into various chrysanthemum cultivars including BlueRidge (BR), Madeline, Moneymaker, Mundial, Improved Reagan, Dark Splendid Reagan. A summary of the results is shown in Table 5.

**TABLE 5**

| ***Results of analysis of transgenic chrysanthemum petals containing various F3***'***5***'***H chimeric genes***. | | | | | | | |
|---|---|---|---|---|---|---|---|
| **Cultivar** | **Construct** | **Cassettes** | **#tg** | **#CC** | **TLC** | **HPLC** | **Northern** |
| BR | pCGP1452 | *AmCHS 5': petHf1:petD83': 35S 5': SuRB* | 70 | 0 | 0/22 | nd | 4/19 Hf1 17/18 SuRB |
| Money maker | pCGP1452 | *AmCHS 5':petHf1:petD83' 35S 5': SuRB* | 217 | 0 | 0/22 | nd | nd |
| Mundial | pCGP1452 | *AmCHS 5':petHf1:petD83' 35S 5': SuRB* | 10 | 0 | 0/2 | nd | nd |
| BR | pCGP1476 | *PetCHS 5': petHf1: mas 3' 35S 5': SuRB* | 147 | 0 | 0/20 | nd | 1/18 Hf1, 15/15 SuRB |
| Money maker | pCGP1476 | *PetCHS 5': petHf1: mas 3'; 35S 5': SuRB* | 19 | 0 | 0/10 | nd | nd |
| BR | pCGP1457 | *petD85':petHf1:petD83'; 35S 5': SuRB* | 150 | 0 | 0/20 | nd | 1/7 Hf1, 6/7 *SuRB* |
| BR | pCGP1616 | *petRT 5': petHf1: nos 3'; 35S 5': SuRB* | 102 | 0 | 0/20 | nd | 0/8 Hf1 |
| Money maker | pCGP1616 | *petRT 5': petHf1: nos 3'; 35S 5': SuRB* | 51 | 0 | 0/18 | nd | nd |
| BR | pCGP90 | *Mac: petHf1: mas 3'; CaMV 35S: nptII: tml3*' | 32 | 0 | 0/16 | nd | nd |
| Madeline | pCGP90 | *Mac: petHf1: mas 3'; CaMV 355: nptII: tml3'* | 48 | 0 | 0/33 | nd | 15/15 Hfl 17/17 nptII |
| BR | pCGP1455 | *genomic petHf1; 35S 5': SuRB* | 62 | 0 | nd | nd | nd |
| BR | pCGP1633 | *ChrysCHS 5':petHf1:petRT3' 35S 5': SuRB* | 21 | 0 | nd | nd | nd |
| BR | pCGP1627 | *CluysCHS 5': petHf1: nos 3'; 35S 5': SuRB* | 23 | 0 | nd | nd | nd |
| Mundial | pCGP1627 | *ChrysCHS 5*'*: petHf1: nos 3'; 35S 5': SuRB* | 8 | 0 | nd | nd | nd |
| BR | pCGP1638 | *35S 5': petHf1: ocs 3'; 35S 5': SuRB* | 19 | 0 | nd | nd | nd |
| BR | pCGP1860 | *RoseCHS 5': petHf1: nos 3'; 35S 5': SuRB* | 40 | 0 | nd | nd | nd |
| IR | pCGP2119 | *CaMV 35S: SalviaF3'5'H#47: ocs 3' 35S 5': SuRB* | 9 | 0 | 0/8 | nd | 5/5 Salvia F3'5'H 5/5 SuRB |
| IR | pCGP2205 | *carnANS 5': BPF3'5'H#18: carnANS 3'; 35S 5': SuRB* | 12 | 1 | 9/12* | 3-18% (8 of 10) | 4/11 BPF3'5'#18 11/11 SuRB |
| DSR | pCGP2205 | *carnANS 5': BPF3'5'H#18: carnANS 3*'*; 35S 5': SuRB* | 19 | 4 | 2 of 2 | 2-22% of 4) | nd |
| IR | pCGP2610 | *CaMV 35S: BPF3'5'H#18: ocs 3'; CaMV 35S: petDFR: 35S 35S 5': SuRB* | 9 | 0 | 0/6* | 0% (0 of 2) | 3/8 BPF3'5'#18 8/8 SuRB 5/8 petDFR |
| IR | pCGP2788 | *CaMV 35S: BPF3'5'H #40: 35S 3*'*; 35S 5': SuRB* | 14 | 0 | 1/14* | 0.5-3% (2 of 3) | 3/3 BPF3'5'#4C 3/3 SuRB |
| DSR | pCGP2788 | *CaMV 35S: BPF3'5'H #40: 35S* 3'; *35S 5': SuRB* | 11 | 0 | 1 of 4 | 3% (1 of 1) | nd |
| IR | pCGP2217 | *Rose CHS 5': BPF3'5'H#18: nos 3'; 35S 5': SuRB* | 37 | 5 | 25/27* | 0.1-37% (26 of 27) | 11/11 BPF3'5'#18 11/11 SuRB |
| DSR | pCGP2217 | *RoseCHS 5': BPF3'5'H#18: nos 3'; 35S 5': SuRB* | 25 | 2 | 11 of 16 | 12% (1 of 2) | nd |
| IR | pCGP3141 | *Cineraria gF3'5'H; 35S 5': SuRB* | 50 | 2 | 8 of 24 | 3-20% (11 of 12) | 10/10 cinF3'5'f 10/10 SuRB |
| DSR | pCGP3141 | *Cineraria gF3'5'H; 35S 5': SuRB* | 12 | 1 | 1 of 1 | 7-11% (3 of 3) | nd |
| IR | pCGP3149 | *35S 5': BP3'5'H#40: SuRB 3' 35S 5'*: *SuRB* | 80 | 0 | 5 of 18 | 0.4-9% (4 of 5) | 10/10 *BPF3'5'H#40* 10/10 SuRB |
| DSR | pCGP3149 | *35S 5': BP3'5'H#40: SuRB 3' 35S 5': SuRB* | 12 | 0 | 2 of 11 | nd | nd |
| IR | pCGP3424 | *RoseCHS 5': BPF3'5'H#18: nos 3'; 35S 5': dschrysF3'H: ocs 35S 5': SuRB* | 50 | 5 | 4 of 10 | 3-41% (6 of 6) | nd |
| DSR | pCGP3424 | *Rose CHS 5': BPF3'5'H#18: nos 3*'*; 35S 5': dschrysF3'H: ocs 3' 35S 5': SuRB* | 30 | 6 | nd | 7-23% (4 of 4) | nd |
| IR | pCGP3429 | *Rose CHS 5': BPF3'5'H#18: nos 3'; Rose CHS 5': dschrysF3'H***: nos 3' 35S 5': SuRB* | 2 | 1 | nd | nd | nd |
| IR | pCGP3434 | *RoseCHS 5': BPF3'5'H40: nos 3'; 35S 5': SuRB* | 29 | 0 | nd | nd | nd |

| | | | | | | | |
|---|---|---|---|---|---|---|---|
| Cultivar = The chrysanthemum cultivar that was transformed Construct = The transformation vector used in the transformation experiment Cassettes = The gene expression cassettes contained in the transformation vector #tg = Total number of transgenics produced and flowered to date #CC = Number of individual transgenic events that produced inflorescences with a significant shift in color compared to that of a non-transformed plant TLC = Number of inflorescences from individual events in which delphinidin was detected in petals (as determined by TLC) over the total number of individual events analyzed * = Extracts used for TLC were concentrated (x20) in an effort to detect very low levels of delphinidin HPLC = Ranges in levels of delphinidin detected by HPLC shown as a percentage of the total anthocyanidins detected in petal extracts. The number of events with delphinidin detected from the total number analysed is represented in brackets Northern = Number of individual events in which the specific intact *F3'5'H*, ALS (*SuRB*) or *nptII* transcripts were detected by Northern blot analysis in total RNA isolated from petals over the total number of events analyzed *und* = Not done Hf1 = Petunia *F3'5'H* Hf1 cDNA clone cinF3'5'H = 576bp *Bgl*II/*Hin*dIII cineraria *F3'5'H* fragment (from SEQ ID No: 12) bearing the 3' end of the coding region used as a template for labeling | | | | | | | |

Surprisingly, of the 23 transformation vector combinations tested to date only five, pCGP2217 (containing *Rose CHS 5': BPF3'5'H#18: nos 3'; 35S 5': SuRB*), pCGP3424 (containing *Rose CHS 5'*: *BPF3'5'H#18: not 3'; 35S 5'*: *ds chrysF3'H: ocs 3'; 35S 5*'*: SuRB*), pCGP3429 (containing *Rose CHS 5': BPF3'5'H#18: nos* 3'; *RoseCHS 5': ds chrysF3'H*: nos 3'; 35S 5': SuRB*), pCGP2205 (containing *carnANS 5': BPF3'5'H#18: carnANS 3'; 35S 5': SuRB)* and pCGP3141 (containing *Cineraria gF3'5'H; 35S 5': SuRB)* resulted in a significant change in petal color (Tables 5 and 6). Expression of selected chimeric genes resulted in the accumulation of novel delphinidin-based flavonoid molecules in chrysanthemum petals (Table 5). However only the introduction of the T-DNAs from pCGP2217, pCGP3424, pCGP3429, pCGP2205, pCGP3141 transformation vectors led to high enough levels of delphinidin-based flavonoids to significantly shift the color to the purple and violet ranges.

**TABLE 6:**

| ***Color codes of the transgenic chrysanthemum petals with significant color changes compared to controls*** | | | | |
|---|---|---|---|---|
| **Cultivar** | **Construct** | **Flavonoid gene cassettes** | **RHS Color Code** | **RHS Color Group** |
| IR | Control | - | 75A | PURPLE |
| IR | pCGP2205 | *carnANS 5':BPF3'5'H#18: carnANS 3'* | 84C | VIOLET |
| IR | pCGP2217 | *RoseCHS 5': BPF3'5'H#18: nos 3'* | 84C | VIOLET |
| IR | pCGP3424 | *RoseCHS 5': BPF3'5'H#18: nos 3'; 35S 5': dschrysF3'H: ocs 3'* | 84B | VIOLET |
| IR | pCGP3141 | *Cineraria gF3'5'H* | 77D | PURPLE |
| IR | pCGP3429 | *RoseCHS 5': BPF3'5'H#18: nos 3'; RoseCHS 5': dschrysF3'H: nos 3'* | N82D | PURPLE-VIOLET |
| DSR | Control | - | 70B | RED-PURPLE |
| DSR | pCGP2205 | *carnANS 5':BPF3'5'H#18: carnANS 3'* | N78B, 77B, | PURPLE |
| DSR | pCGP2217 | *RoseCHS 5': BPF3'5'H#18: nos 3'* | N78B/C | PURPLE |
| DSR | pCGP3424 | *Rose CHS 5': BPF3'5'H#18: nos 3'; 35S 5': dschtysF3'H: ocs 3'* | N80C | PURPLE-VIOLET |
| DSR | pCGP3141 | *Cineraria gF3'5'H* | 77B | PURPLE |

| | | | | |
|---|---|---|---|---|
| Cultivar = The chrysanthemum cultivar that was transformed Construct = The transformation vector used in the transformation experiment Flavonoid Pathway cassettes = The flavonoid gene expression cassettes contained in the transformation vector construct RHS Color Code = The color code of the petals from the inflorescences of transgenic chrysanthemums with the most significant color changes as compared to the control petals. RHS Color Group = The Color group associated with the petal color according to the Royal Horticultural Society, London | | | | |

The result of the expression of the *F3'5'H* chimeric genes in the chrysanthemum lines listed in Table 6 shows that there was a shift to purple or purple-violet in petal color as compared to the control color. The petal color of non-transgenic control Improved Reagan (RHSCC 75A) is grouped in the purple color group, however expression of *RoseCHS 5': BPF3'5'H#18: nos 3'* chimeric gene with or without a *ds chrysF3'H* chimeric gene or *carnANS 5': BPF3'5'H#18: carnANS* 3' gene led to a shift in petal color to the violet range of the RHS Color Charts (see Table 6). The petal color of non-transgenic control Dark Splendid Reagan is in the red-purple color range, however expression of *RoseCHS 5': BPF3'5'H#18: nos 3'* chimeric gene with or without a *ds chrysF3'H* chimeric gene or *carnANS 5*'*: BPF3'5'H#18:carnANS* 3' or *Cineraria gF3'5'H* genes led to a shift in petal color to the purple and violet range of the RHS Color Charts.

These results indicate that only a small combination of promoter, terminator and *F3 '5'H* sequences leads to the production of significant delphinidin-based pigments and thus a shift in the petal color to a violet/purple /blue range in chrysanthemum petals. The combination of a *F3'5'H* sequence from *Viola* or *Cineraria* controlled by promoters from flavonoid specific genes such as a *CHS* promoter from rose or an *ANS* promoter from carnation or an *F3'5'H* promoter from *Cineraria* has led to a significant color change in chrysanthemum petals.

Increased delphinidin production is achieved by down-regulating an endogenous *F3'H* and/or the endogenous *DFR* in chrysanthemum using silencing strategies such as co-suppression, antisense or ddRNAi. Where the endogenous *DFR* is down-regulated a DFR capable of utilizing dihydromyricetin preferentially over dihydroquercetin is included. Such a DFR can be, but is not limited to, be isolated from petunia or iris.

The *Rose CHS 5': BPF3'5'H#18: nos* 3' gene contained in pCGP2217 and pCGP3424 was also introduced into a number of chrysanthemum varieties ("Sei" varieties) including Sei Figaro, Sei Florea, Sei Spire, Sei Titan, Sei Titan406, Sei Norma, Sei Amelie (Table 7).

**TABLE 7:**

| ***Summary of the transgenic chrysanthemum petals of the "Sei "varieties*** | | | | | |
|---|---|---|---|---|---|
| **Cultivar** | **Construct** | **#tg** | **#CC** | **RHSCC** | **RHSCG** |
| Sei Figaro | pCGP2217 | 25 | 3 | 76C | PURPLE |
| Sei Figaro | pCGP3424 | 50 | 3 | 76C | PURPLE |
| Sei Figaro | control | | | 65D, 75D, 69B | RED-PURPLE |
| Sei Florea | pCGP2217 | 7 | 3 | N80C | PURPLE-VIOLET |
| Sei Florea | control | | | 75A, B | PURPLE |
| Set Spire | pCGP2217 | 42 | 3 | N80D, 76A | PURPLE-VIOLET |
| Sei Spire | control | | | 75C, D | PURPLE |
| Sei Titan | pCGP2217 | 50 | 7 | 77C, N80D | PURPLE/PURPLE-VIOLET |
| Sei Titan | pCGP3424 | 48 | 4 | N80D, N81B | PURPLE-VIOLET |
| Sei Titan | control | | | 78D-, 75B | PURPLE |
| Sei Titan 406 | pCGP2217 | 25 | 4 | 84C- | VIOLET |
| Sei Titan 406 | pCGP3424 | 34 | 3 | 76B | PURPLE |
| Sei Titan 406 | control | | | 75C, D | PURPLE |

| | | | | | |
|---|---|---|---|---|---|
| Cultivar = The chrysanthemum cultivar that was transformed Construct = The transformation vector used in the transformation experiment #tg = Total number of transgenics produced and flowered to date #CC = Number of individual transgenic events that produced inflorescences with an altered flower color compared to that of a non-transformed plant RHS Color Code = The color code of the petals from the inflorescences of chrysanthemum with the most significant color changes as compared to the control petals. RHS Color Group = The Color group associated with the petal color according to the Royal Horticultural Society, London | | | | | |

Expression of the *F3'5'H* chimeric genes in the chrysanthemum lines listed in Table 7 shows that there was a shift to purple or purple-violet in petal color as compared to the control color. The petal color of non-transgenic control Sei Figaro is in the red-purple color range, however expression of *RoseCHS 5': BPF3'5'H#18: nos* 3' chimeric gene with or without a *chrys dsF3'H* chimeric gene led to a shift in petal color to the purple range of the RHS Color Charts. The petal color of non-transgenic control Sei Florea is in the purple color range, however expression of *RoseCHS 5': BPF3'5'H#18: nos* 3' chimeric gene in the transgenic lines led to a shift in petal color to the purple-violet range of the RHS Color Charts. The petal color of non-transgenic control Sei Titan is in the purple color range, however expression of *RoseCHS: BPF3'5'H#18: nos 3'*chimeric gene with or without a *chrys dsF3'H* chimeric gene led to a shift in petal color to the purple-violet range of the RHS Color Charts. The petal color of non-transgenic control Sei Titan406 is in the purple color range, however expression of *RoseCHS 5': BPF3'5'H#18: nos 3'* chimeric gene led to a shift in petal color to the violet range of the RHS Color Charts.

The invention also includes all of the steps, features, compositions and compounds referred to or indicated in this specification, individually or collectively, and any and all combinations of any two or more of said steps or features.

### BIBLIOGRAPHY

Aida et al, Breeding Science, 54: 51-58, 2004
Altschul et al, J. Mol. Biol. 215(3):403-410, 1990
Barker et al, Plant Mol. Biol. 2:235-350, 1983
Beld et al, .Plant Mol. Biol. 13:491-502, 1989
Bodeau, Molecular and genetic regulation of Bronze-2 and other maize anthocyanin genes. Dissertation, Stanford University, USA, 1994
Bonner and Laskey, Eur. J. Biochem. 46:83, 1974
Brugliera, Characterization of floral specific genes isolated from Petunia hybrida. RMIT, Australia. PhD thesis, 1994
Brugliera et al, Plant J. 5:81-92, 1994
Bullock et al, Biotechniques 5:376, 1987
Comai et al, Plant Mol. Biol. 15:373-381, 1990
Depicker et al., J Mol. and Appl. Genetics 1:561-573, 1982
da Silva, Biotechnology Advances 21:715-766, 2003.
Dolgov et al, Acta Hort 447:329-334, 1997
Doodeman et al, Theor. Appl. Genet. 67:357-366, 1984
Franck et al, Cell 21:285-294, 1980
Garfinkel et al, Cell 27:143-153, 1981
Glimn-Lacy and Kaufman, Botany Illustrated, Introduction to Plants, Major Groups, Flowering Plant Families, 2nd ed, Springer, USA, 2006
Greve, J. Mol. Appl. Genet. 1:499-511, 1983
Guilley et al, Cell, 30:763-773, 1982
Hanahan, J. Mol. Biol. 166: 557, 1983
Harpster et al, MGG, 212:182-190, 1988
Holton, Isolation and characterization of petal specific genes from Petunia hybrida. PhD thesis, University of Melbourne, Australia, 1992
Holton et al, Nature, 366:276-279, 1993
Holton and Cornish, Plant Cell 7:1071-1083, 1995
Huang and Miller, Adv. Appl. Math. 12.-373-381, 1991
Inoue et al, Gene 96:23-28, 1990
Janssen and Gardner, Plant Molecular Biology, 14:61-72, 1989
Koes, Genes involved in flavonoid biosynthesis in Petunia hybrida: The chalcone synthase multigene family. PhD thesis, Vrije Universiteit, Amsterdam, Netherlands, 1988
Lazo et al Bio/technology 9:963-967, 1991
Ledger et al, Plant Cell Reports, 10:195-199, 1991
Lee et al, EMBO J. 7:1241-1248, 1988
Marmur and Doty, J. Mol. Biol. 5:109, 1962
Mol et al, Trends Plant Sci. 3:212-217, 1998
Pearson and Lipman, Proc. Natl. Acad. Sci. USA 85(8):2444-2448, 1988
Plant Molecular Biology Labfax, Croy (ed), Bios scientific Publishers, Oxford, UK, 1993
Plant Molecular Biology Manual (2nd edition), Gelvin and Schilperoot (eds), Kluwer Academic Publisher, The Netherlands, 1994
Salomon et al, EMBO, J. 3:141- 146, 1984
Sambrook et al, Molecular Cloning: A Laboratory Manual, Cold Spring Harbor Laboratories, Cold Spring Harbor, NY, USA, 1989
Sambrook and Russell, Molecular Cloning: A Laboratory Manual 3rd edition, Cold Spring Harbor Laboratories, Cold Spring Harbor, NY, USA, 2001
Schwinn et al, Phytochemistry, 35:145-150, 1993
Seitz and Hinderer, Anthocyanins. In: Cell Culture and Somatic Cell Genetics of Plants. Constabel and Vasil (eds.), Academic Press, New York, USA, 5:49-76, 1988
Sommer and Saedler, Mol Gem. Gent., 202:429-434, 1986
Strack and Wray, In: The Flavonoids - Advances in Research since 1986. Harborne, J.B. (ed), Chapman and Hall, London, UK, 1-22,1993
Tanaka et al, Plant and Cell Physiology, 36, 1023-1031, 1995
Tanaka and Mason, In Plant Generic Engineering, Singh and Jaiwal (eds) SciTech Publishing Llc., USA, 1:361-385, 2003
Tanaka et al, Plant Cell, Tissue and Organ Culture 80:1-24, 2005
Tanaka and Brugliera, In Flowering and Its Manipulation, Annual Plant Reviews Ainsworth (ed), Blackwell Publishing, UK, 20:201-239, 2006
Thompson et al, Nucleic Acids Research 22:4673-4680, 1994
Toguri et al, Plant Biotechnology, 20:121-127, 2003;
Turpen and Griffith, BioTechniques 4:11-15, 1986
van Engelen et al, Transgenic Research 4:288-290, 1995
Wallroth et al, (Mol. Gen. Genet. 202:6-15, 1986
Winkel-Shirley, Plant Physiol. 126:485-493, 2001a
Winkel-Shirley, Plant Playsiol. 127:1399-1404, 2001b

## Claims

1. A genetically modified chrysanthemum plant exhibiting altered inflorescence, said chrysanthemum comprising expressed genetic material encoding a flavonoid 3',5' hydroxylase (F3'5'H) enzyme operably linked to a selected promoter and terminator, wherein:
(a) the F3'5'H enzyme is encoded by SEQ ID NO: 1, or a nucleotide sequence capable of hybridizing to a complementary form of SEQ ID NO: 1 under high stringency conditions, and the promoter is from an anthocyanidin synthase (ANS) gene; or
(b) the F3'5'H enzyme is encoded by SEQ ID NO: 12, or a nucleotide sequence capable of hybridizing to a complementary form of SEQ ID NO: 12 under high stringency conditions, and the promoter is from a F3'5'H gene derived from Cineraria.

2. A genetically modified plant of Claim 1 further comprising expressed genetic material which down-regulates an indigenous flavonoid 3' hydroxylase (F3'H).

3. The genetically modified plant of Claim 1 or 2 further comprising expressed genetic material which down-regulates an indigenous DFR.

4. The genetically modified plant of Claim 1 or 2 or 3 further comprising expressed genetic material which encodes a heterologous dihydroflavonol-4-reductase (DFR).

5. The genetically modified plant of any one of Claims 1 to 4 wherein the altered inflorescence is a color in the range red/purple to blue color.

6. The genetically modified plant of Claim 5 wherein the color is a violet/purple color.

7. The genetically modified plant of Claim 5 wherein the color is selected from the list consisting of 75C, 75D, 76A, 76B, 76C, 77C, 78D, N80D, N81B and 84C of the Royal Horticultural Society color chart.

8. The genetically modified plant of Claim 5 or 6 or 7 wherein the altered inflorescence is in a flower, petal, anther or style of said chrysanthemum plant.

9. The genetically modified plant of any one of Claims 1 to 8 wherein the chrysanthemum accumulates delphinidin-based flavonoid molecules as determined by TLC or HPLC which is absent in non-genetically modified chrysanthemum plants.

10. The genetically modified plant of any one of Claims 1 to 9 wherein the chrysanthemum has a genetic background of a chrysanthemum cultivar selected from BlueRidge, Madeline, Moneymaker, Mundial, Improved Reagan, Dark Splendid Reagan, Sei Figaro, Sei Florea, Sei Titan, Sei Titan406, Sei Norma and Sei Amelie.

11. The genetically modified plant of Claim 1 wherein the terminator is CarnANS 3' terminator.

12. The genetically modified plant of Claim 1 wherein the promoter is from a Cineraria gF3'5'H clone.

13. The genetically modified plant of Claim 1 comprising a CarnANS 5' promoter and carnANS 3' terminator.

14. The genetically modified plant of Claim 1 comprising carnANS 5': BPF3'5'H#18:carnANS 3'.

15. The genetically modified plant of Claim 1 wherein the promoter and terminator sequences are from the Cineraria gF3'5'H clone.

16. The genetically modified plant of Claim 1 comprising expressed genetic material according to SEQ ID NO.: 12.

17. The genetically modified plant of Claim 1 further comprising expressed genetic material which encodes two or more F3'5'H enzymes and/or DFR enzymes.

18. Progeny having the genetic modification of a genetically modified chrysanthemum plant according to any one of claims 1 to 17, cut flowers, tissue culturable cells and regenerable cells from the genetically modified plant of any one of Claims 1 to 17.

19. A method for producing a chrysanthemum plant exhibiting altered inflorescence, said method comprising introducing into regenerable cells of a chrysanthemum plant genetic material encoding a flavonoid 3',5' hydroxylase (F3'5'H) enzyme operably linked to a selected promoter and terminator, wherein:
(a) the F3'5'H enzyme is encoded by SEQ ID NO: 1, or a nucleotide sequence capable of hybridizing to a complementary form of SEQ ID NO: 1 under high stringency conditions, and the promoter is from an anthocyanidin synthase (ANS) gene; or
(b) the F3'5'H enzyme is encoded by SEQ ID NO: 12, or a nucleotide sequence capable of hybridizing to a complementary form of SEQ ID NO: 12 under high stringency conditions, and the promoter is from a F3'5'H gene derived from Cineraria;
said method further comprising regenerating a planttherefrom.

20. The method of Claim 19 further comprising introducing the genetic sequence down-regulating an indigenous F3'H.

21. The method of Claim 19 or 20 further comprising introducing the genetic sequence encoding a heterologous DFR

22. The method of any one of Claim 19 to 21 further comprising introducing the genetic sequence down-regulating an indigenous DFR.

## Patentansprüche

1. Genetisch modifizierte Chrysanthemenpflanze, die veränderte Infloreszenz aufweist, wobei die Chrysantheme exprimiertes genetisches Material umfasst, das ein Flavonoid-3'-5'-Hydroxylase-(F3'5'H)-Enzym kodiert, das in operativer Weise mit einem ausgewählten Promotor und Terminator verbunden ist, wobei:
(a) das F3'5'H-Enzym durch SEQ ID NO: 1 oder eine Nukleotidsequenz, die imstande ist, mit einer komplementären Form von SEQ ID NO: 1 unter Bedingungen hoher Stringenz zu hybridisieren, kodiert wird und der Promotor aus einem Anthocyanidinsynthase(ANS)-Gen ist; oder
(b) das F3'5'H-Enzym durch SEQ ID NO: 12 oder eine Nukleotidsequenz, die imstande ist, mit einer komplementären Form von SEQ ID NO: 12 unter Bedingungen hoher Stringenz zu hybridisieren, kodiert wird und der Promotor aus einem von Cineraria abgeleiteten F3'5'H-Gen ist.

2. Genetisch modifizierte Pflanze gemäß Anspruch 1, ferner umfassend exprimiertes genetisches Material, welches eine indigene Flavonoid-3'-Hydroxylase (F3'H) herunterregelt.

3. Genetisch modifizierte Pflanze gemäß Anspruch 1 oder 2, ferner umfassend exprimiertes genetisches Material, welches eine indigene DFR herunterregelt.

4. Genetisch modifizierte Pflanze gemäß Anspruch 1 oder 2 oder 3, ferner umfassend exprimiertes genetisches Material, welches eine heterologe Dihydroflavonol-4-Reduktase (DFR) kodiert.

5. Genetisch modifizierte Pflanze gemäß einem der Ansprüche 1 bis 4, wobei die veränderte Infloreszenz eine Farbe im Bereich von roter/purpurner bis blauer Farbe ist.

6. Genetisch modifizierte Pflanze gemäß Anspruch 5, wobei die Farbe eine violette/purpurne Farbe ist.

7. Genetisch modifizierte Pflanze gemäß Anspruch 5, wobei die Farbe ausgewählt wird aus der Liste, bestehend aus 75C, 75D, 76A, 76B, 76C, 77C, 78D, N80D, N81B und 84C der Farbkarte der Royal Horticultural Society.

8. Genetisch modifizierte Pflanze gemäß Anspruch 5 oder 6 oder 7, wobei die veränderte Infloreszenz in einer Blüte, einem Blütenblatt, einem Staubbeutel oder einem Griffel der Chrysanthemenpflanze vorliegt.

9. Genetisch modifizierte Pflanze gemäß einem der Ansprüche 1 bis 8, wobei die Chrysantheme Delphinidin-basierte Flavonoid-Moleküle akkumuliert, bestimmt durch DC oder HPLC, was bei nicht-genetisch modifizierten Chrysanthemenpflanzen fehlt.

10. Genetisch modifizierte Pflanze gemäß einem der Ansprüche 1 bis 9, wobei die Chrysantheme einen genetischen Hintergrund einer Chrysanthemensorte, ausgewählt aus BlueRidge, Madeline, Moneymaker, Mundial, Imroved Reagan, Dark Splendid Reagan, Sei Figaro, Sei Florea, Sei Titan, Sei Titan406, Sei Norma und Sei Amelie, hat.

11. Genetisch modifizierte Pflanze gemäß Anspruch 1, wobei der Terminator ein CarnANS-3'-Terminator ist.

12. Genetisch modifizierte Pflanze gemäß Anspruch 1, wobei der Promotor aus einem Cineraria gF3'5'H-Klon ist.

13. Genetisch modifizierte Pflanze gemäß Anspruch 1, umfassend einen CarnANS-5'-Promotor und einen carnANS-3'-Terminator.

14. Genetisch modifizierte Pflanze gemäß Anspruch 1, umfassend carnANS 5':BPF3'5'H#18:carnANS 3'.

15. Genetisch modifizierte Pflanze gemäß Anspruch 1, worin die Promotor- und Terminator-Sequenzen aus dem Cineraria gF3'5'H-Klon sind.

16. Genetisch modifizierte Pflanze gemäß Anspruch 1, umfassend exprimiertes genetisches Material gemäß SEQ ID NO: 12.

17. Genetisch modifizierte Pflanze gemäß Anspruch 1, ferner umfassend exprimiertes genetisches Material, welches zwei oder mehrere F3'5'H-Enzyme und/oder DFR-Enzyme kodiert.

18. Nachkommen, aufweisend die genetische Modifikation einer genetisch modifizierten Chrysanthemenpflanze gemäß einem der Ansprüche 1 bis 17, Schnittblumen, Gewebekultivierbare Zellen und regenerierbare Zellen aus der genetisch modifizierten Pflanze gemäß einem der Ansprüche 1 bis 17.

19. Verfahren zur Herstellung einer Chrysanthemenpflanze, die veränderte Infloreszenz aufweist, wobei das Verfahren Einbringen von genetischem Material einer Chrysanthemenpflanze, das ein Flavonoid-3',5'-Hydroxylase-(F3'5'H)-Enzym kodiert, das in operativer Weise mit einem ausgewählten Promotor und Terminator verbunden ist, in regenerierbare Zellen einer Chrysanthemenpflanze, wobei:
(a) das F3'5'H-Enzym durch SEQ ID NO: 1 oder eine Nukleotidsequenz, die imstande ist, mit einer komplementären Form von SEQ ID NO: 1 unter Bedingungen hoher Stringenz zu hybridisieren, kodiert wird und der Promotor aus einem Anthocyanidinsynthase(ANS)-Gen ist; oder
(b) das F3'5'H-Enzym durch SEQ ID NO: 12 oder eine Nukleotidsequenz, die imstande ist, mit einer komplementären Form von SEQ ID NO: 12 unter Bedingungen hoher Stringenz zu hybridisieren, kodiert wird und der Promotor aus einem von Cineraria abgeleiteten F3'5'H-Gen ist;
wobei das Verfahren ferner Regenerieren einer Pflanze daraus umfasst.

20. Verfahren gemäß Anspruch 19, ferner umfassend Einbringen einer genetischen Sequenz, die eine indigene F3'H herunterregelt.

21. Verfahren gemäß Anspruch 19 oder 20, ferner umfassend Einbringen einer genetischen Sequenz, die eine heterologe DFR kodiert.

22. Verfahren gemäß einem der Ansprüche 19 bis 21, ferner umfassend Einführen der genetischen Sequenz, die eine indigene DFR herunterregelt.

## Revendications

1. Plant de chrysanthème génétiquement modifié présentant une inflorescence modifiée, ledit chrysanthème comprenant un matériau génétique exprimé codant pour une enzyme flavonoïde 3',5' hydroxylase (F3'5'H) fonctionnellement liée à un promoteur et un terminateur sélectionnés, dans lequel :
(a) l'enzyme F3'5'H est codée par la SEQ ID NO : 1, ou une séquence nucléotidique capable de s'hybrider à une forme complémentaire de la SEQ ID NO : 1 dans des conditions de stringence élevée et le promoteur provient d'un gène de l'anthocyanidine synthase (ANS) ; ou
(b) l'enzyme F3'5'H est codée par la SEQ ID NO : 12, ou une séquence nucléotidique capable de s'hybrider à une forme complémentaire de la SEQ ID NO : 12 dans des conditions de stringence élevée et le promoteur provient d'un gène de la F3'5'H dérivé de Cineraria.

2. Plant génétiquement modifié selon la revendication 1, comprenant en outre un matériau génétique exprimé qui régule à la baisse une flavonoïde 3' hydroxylase (F3'H) indigène.

3. Plant génétiquement modifié selon la revendication 1 ou 2, comprenant en outre un matériau génétique exprimé qui régule à la baisse une DFR indigène.

4. Plant génétiquement modifié selon la revendication 1 ou 2 ou 3, comprenant en outre un matériau génétique exprimé qui code pour une dihydroflavonol-4-réductase (DFR) hétérologue.

5. Plant génétiquement modifié selon l'une quelconque des revendications 1 à 4, dans lequel l'inflorescence modifiée est une couleur dans la plage de couleurs rouge/pourpre à bleu.

6. Plant génétiquement modifié selon la revendication 5, dans lequel la couleur est une couleur violet/pourpre.

7. Plant génétiquement modifié selon la revendication 5, dans lequel la couleur est sélectionnée dans la liste constituée de 75C, 75D, 76A, 76B, 76C, 77C, 78D, N80D, N81B et 84C de la gamme des couleurs de la Royal Horticultural Society.

8. Plant génétiquement modifié selon la revendication 5 ou 6 ou 7, dans lequel l'inflorescence modifiée se trouve dans une fleur, un pétale, une anthère ou un style dudit plant de chrysanthème.

9. Plant génétiquement modifié selon l'une quelconque des revendications 1 à 8, dans lequel le chrysanthème accumule des molécules de flavonoïde à base de delphinidine comme déterminé par CCM ou CLHP qui sont absentes des plants de chrysanthème non génétiquement modifiés.

10. Plant génétiquement modifié selon l'une quelconque des revendications 1 à 9, dans lequel le chrysanthème a un bagage génétique d'un cultivar de chrysanthème sélectionné parmi BlueRidge, Madeline, Moneymaker, Mundial, Improved Reagan, Dark Splendid Reagan, Sei Figaro, Sei Florea, Sei Titan, Sei Titan406, Sei Norma et Sei Amelie.

11. Plant génétiquement modifié selon la revendication 1, dans lequel le terminateur est le terminateur CarnANS 3'.

12. Plant génétiquement modifié selon la revendication 1, dans lequel le promoteur provient d'un clone gF3'5'H de Cineraria.

13. Plant génétiquement modifié selon la revendication 1, comprenant un promoteur CarnANS 5' et un terminateur carnANS 3'.

14. Plant génétiquement modifié selon la revendication 1, comprenant carnANS 5':BPF3'5'H#18:carnANS 3'.

15. Plant génétiquement modifié selon la revendication 1, dans lequel les séquences du promoteur et du terminateur proviennent du clone gF3'5'H de Cineraria.

16. Plant génétiquement modifié selon la revendication 1, comprenant le matériau génétique exprimé selon la SEQ ID NO : 12.

17. Plant génétiquement modifié selon la revendication 1, comprenant en outre le matériau génétique exprimé qui code pour au moins deux enzymes F3'5'H et/ou DFR.

18. Progéniture ayant la modification génétique d'un plant de chrysanthème génétiquement modifié selon l'une quelconque des revendications 1 à 17, des fleurs coupées, des cellules cultivables de tissu et des cellules régénérables du plant génétiquement modifié selon l'une quelconque des revendications 1 à 17.

19. Procédé de production d'un plant de chrysanthème présentant une inflorescence modifiée, ledit procédé comprenant l'introduction dans des cellules régénérables d'un matériau génétique de plant de chrysanthème codant pour une enzyme flavonoïde 3',5' hydroxylase (F3'5'H) fonctionnellement liée à un promoteur et un terminateur sélectionnés, dans lequel :
(a) l'enzyme F3'5'H est codée par la SEQ ID NO : 1, ou une séquence nucléotidique capable de s'hybrider à une forme complémentaire de la SEQ ID NO : 1 dans des conditions de stringence élevée, et le promoteur provient d'un gène de l'anthocyanidine synthase (ANS) ;
ou
(b) l'enzyme F3'5'H est codée par la SEQ ID NO : 12, ou une séquence nucléotidique capable de s'hybrider à une forme complémentaire de la SEQ ID NO : 12 dans des conditions de stringence élevée, et le promoteur provient d'un gène de la F3'5'H dérivé de Cineraria ;
ledit procédé comprenant en outre la régénération d'un plant à partir de celui-ci.

20. Procédé selon la revendication 19, comprenant en outre l'introduction de la séquence génétique régulant à la baisse une F3'H indigène.

21. Procédé selon la revendication 19 ou 20, comprenant en outre l'introduction de la séquence génétique codant pour une DFR hétérologue.

22. Procédé selon l'une quelconque des revendications 19 à 21, comprenant en outre l'introduction de la séquence génétique régulant à la baisse une DFR indigène.
